(19) Europäisches Patentamt
European Patent Office
Office européen des brevets

(11) EP 2 843 060 B1

(12) # EUROPEAN PATENT SPECIFICATION

(45) Date of publication and mention of the grant of the patent:
**26.10.2016 Bulletin 2016/43**

(51) Int Cl.:
***C12Q 1/68*** *(2006.01)*

(21) Application number: **14182820.2**

(22) Date of filing: **29.08.2014**

(54) **Method and program for determining sensitivity to breast cancer neoadjuvant chemotherapy**

Verfahren und ein Programm zur Ermittlung der Sensitivität auf eine gegen Brustkrebs gerichtete neoadjuvante Chemotherapie

Un procédé et un programme de détermination de la sensibilité à une chimiothérapie néo-adjuvante de cancer du sein

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.08.2013 JP 2013179877**

(43) Date of publication of application:
**04.03.2015 Bulletin 2015/10**

(73) Proprietors:
- **SYSMEX CORPORATION**
  **Kobe-shi,**
  **Hyogo 651-0073 (JP)**
- **OSAKA UNIVERSITY**
  **Suita-shi, Osaka 565-0871 (JP)**

(72) Inventors:
- **Noguchi, Shinzaburou**
  **Suita-shi**
  **Osaka 565-0871 (JP)**
- **Naoi, Yasuto**
  **Suita-shi**
  **Osaka 565-0871 (JP)**
- **Sota, Yoshiaki**
  **Suita-shi**
  **Osaka 565-0871 (JP)**
- **Kishi, Kazuki**
  **Kobe-shi**
  **Hyogo 651-0073 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**WO-A1-2010/076322**
**WO-A1-2013/014296**
**WO-A2-2008/006517**
**US-A1- 2007 172 833**
**US-A2- 2007 239 415**

- **C. DENKERT ET AL: "Tumor-Associated Lymphocytes As an Independent Predictor of Response to Neoadjuvant Chemotherapy in Breast Cancer", JOURNAL OF CLINICAL ONCOLOGY, vol. 28, no. 1, 16 November 2009 (2009-11-16), pages 105-113, XP055160521, ISSN: 0732-183X, DOI: 10.1200/JCO.2009.23.7370**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 843 060 B1

SEQUENCE LISTING

<110> OSAKA UNIVERSITY SYSMEX CORPORATION

<120> Method, apparatus and program for determining sensitivity to breast cancer neoadjuvant chemotherapyr

<130> SYSM-083-EP

<150> JP2013-179877
<151> 2013-08-30

<160> 276

<170> PatentIn version 3.5

<210> 1
<211> 473
<212> DNA
<213> Homo sapiens

<400> 1
agctctcaga caaaggctgc cttgccggcg gggggagccc gaaacagccc tttgcagctc 60

tgcaccagga gcaggttttg cggaaccccc atgacgcagg cctgagcagc ggggagccgc 120

ccgagaagga gcggcggcgc ctcaaagaga gttttgagaa ctaccgcagg aagcgcgccc 180

tcaggaagat gcagaaagga tggcggcagg gggaggagga ccgggagaac accacgggca 240

gcgacaacac cgacactgag ggctcctagc cgcagcagac ttccccgagc cgtcgctgac 300

ttggcctgga acgaggaatc tggtgccctg aaaggcccag ccggactgcc gggcattggg 360

gccgtttgtt aagcggcact cattttgcgg aggccatgcg ggtgctcacc acccccatgc 420

acacgccatc tgtgtaactt caggatctgt tctgtttcac catgtaacac aca 473

<210> 2
<211> 398
<212> DNA
<213> Homo sapiens

<400> 2

accccagct atcagacggt ctggtgtatg aagggttctg ggaagaccca aaggagtttg 60
caggggcag tgcaggccaa agcagcgtct ttcagtgctt tgacgtcctg ctgggcatcc 120
agcagactgc tggtggagga catgctgctc agttcctcca ggacatgaga agatatatgc 180
caccagctca caggaacttc ctgtgctcat tagagtcaaa tccctcagtc cgtgagtttg 240
tcctttcaaa aggtgatgct ggcctgcggg aagcttatga cgcctgtgtg aaagctctgg 300
tctccctgag gagctaccat ctgcaaatcg tgactaagta catcctgatt cctgcaagcc 360
agcagccaaa ggagaataag acctctgaag acccttca 398

<210> 3
<211> 367
<212> DNA
<213> Homo sapiens

<400> 3

gattatcaat taccacacca tctcccatga agaaagggaa cggtgaagta ctaagcgcta 60
gaggaagcag ccaagtcggt tagtggaagc atgattggtg cccagttagc ctctgcagga 120
tgtggaaacc tccttccagg ggaggttcag tgaattgtgt aggagaggtt gtctgtggcc 180
agaatttaaa cctatactca ctttcccaaa ttgaatcact gctcacactg ctgatgattt 240
agagtgctgt ccggtggaga tcccacccga acgtcttatc taatcatgaa actccctagt 300
tccttcatgt aacttccctg aaaaatctaa gtgtttcata aatttgagag tctgtgaccc 360
acttacc 367

<210> 4
<211> 548
<212> DNA
<213> Homo sapiens

<400> 4

gcttctcact catcactaac aaggtcatca tggattatga aagcctggag aaggccaacc 60

atgaagaagt cttagcagct ggcaaacaag ctgcacagaa attggaacag tttgtctcca 120

ttcttatggc cagcattcca ctccctgaca aagccagttg acctgccttg gagtcgtctg 180

gcatctccca cacaagaccc aagtagctgc taccttcttt ggccccttgc tggagtcatg 240

tgcctctgtc cttaggttgt agcagaaagg aaaagattcc tgtccttcac ctttcccact 300

ttcttctacc agacccttct ggtgccagat cctcttctca aagctgggat tacaggtgtg 360

agcatagtga gaccttggcg ctacaaaata aagctgttct cattcctgtt ctttcttaca 420

caagagctgg agcccgtgcc ctaccacaca tctgtggaga tgcccaggat ttgactcggg 480

ccttagaact ttgcatagca gctgctacta gctctttgag ataatacatt ccgaggggct 540

cagttctg 548

<210> 5
<211> 416
<212> DNA
<213> Homo sapiens

<400> 5

gagacttttc tatggttttg tgactttcaa cttttgtaca gttatgtgaa ggatgaaagg 60

tgggtgaaag gaccaaaaac agaaatacag tcttcctgaa tgaatgacaa tcagaattcc 120

actgcccaaa ggagtccaac aattaaatgg atttctagga aaagctacct taagaaaggc 180

tggttaccat cggagtttac aaagtgcttt cacgttctta cttgttgtat tatacattca 240

tgcatttcta ggctagagaa ccttctagat ttgatgctta caactattct gttgtgacta 300

tgagaacatt tctgtctcta gaagttatct gtctgtattg atctttatgc tatattacta 360

tctgtggtta cagtggagac attgacatta ttactggagt caagccctta taagtc 416

<210> 6
<211> 369
<212> DNA
<213> Homo sapiens

<400> 6

cgccggcaaa actttggcac tggggcactt ggcagcgcgg ggagcccgtc ggtaatttta 60

atattttatt atatatatat atctatattt ttgtccaaac caaccgcaca tgcagatggg 120

gctcccgccc gtggtgttat ttaaagaaga aacgtctatg tgtacagatg aatgataaac 180

tctctgcttc tccctctgcc cctctccagg cgccggcggg cgggccggtt tcgaagttga 240

tgcaatcggt ttaaacatgg ctgaacgcgt gtgtacacgg gactgacgca acccacgtgt 300

aactgtcagc cgggccctga gtaatcgctt aaagatgttc ctacgggctt gttgctgttg 360

atgttttgt 369

<210> 7
<211> 547
<212> DNA
<213> Homo sapiens

<400> 7

tccatttctc atgttttcca ttgtttgaaa caaagaaggt taccaagaag cctttcctgt 60

agccttctgt aggaattctt ttggggaagt gaggaagcca ggtccacggt ctgttcttga 120

agcagtagcc taacacactc caagatatgg acacacggga gccgctggca gaagggactt 180

cacgaagtgt tgcatggatg ttttagccat tgttggcttt cccttatcaa acttgggccc 240

ttcccttctt ggtttccaaa ggcatttatt gctgagttat atgttcactg tccccctaat 300

attagggagt aaaacggata ccaagttgat ttagtgtttt tacctctgtc ttggctttca 360

tgttattaaa cgtatgcatg tgaagaaggg tgtttttctg ttttatattc aactcataag 420

actttgggat aggaaaaatg agtaatggtt actaggctta atacctgggt gattacataa 480

tctgtacaac gaacccccat gatgtaagtt tacctatgta acaaacctgc acttataccc 540

atgaact 547

<210> 8
<211> 534
<212> DNA
<213> Homo sapiens

<400> 8

aacacttcga gcactgtcca gtattctacc gtggtacaca gtggctacag acaccaagtt 60

ccgtcagtcc aagtcttctc aagatccgag tctacccagc ccttgttaga ttcagaggag 120

cggccagaag atctacaatt agtagatcat gtagatggcg gtgatggtat tttgcccagg 180

caacagtact tcaaacagaa ctgcagtcag catgaatcca gtccagatat ttcacatttt 240
gaaaggtcaa agcaagtttc atcagtcaat gaggaagatt ttgttagact taaacagcag 300
atttcagatc atatttcaca atcctgtgga tctgggcaaa tgaaaatgtt tcaggaagtt 360
tctgcagcag atgcttttgg tccaggtact gagggacaag tagaaagatt tgaaacagtt 420
ggcatggagg ctgcgactga tgaaggcatg cctaaaagtt acttaccaca gactgtacgg 480
caaggcggct acatgcctca gtgaaggact agtagttcct gctacaactt cagc 534

<210> 9
<211> 503
<212> DNA
<213> Homo sapiens

<400> 9

agcccctgcc catctgggaa aataccccat cattcatgct actgccaacc tggggagcca 60
gggctatggg agcagctttt ttttcccccc tagaaacgtt tggaacaatg taaaacttta 120
aagctcgaaa acaattgtaa taatgctaaa gaaaaagtca tccaatctaa ccacatcaat 180
attgtcattc ctgtattcac ccgtccagac cttgttcaca ctctcacatg tttagagttg 240
caatcgtaat gtacagatgg ttttataatc tgatttgttt tcctcttaac gttagaccac 300
aaatagtgct cgctttctat gtagtttggt aattatcatt ttagaagact ctaccagact 360
gtgtattcat tgaagtcaga tgtggtaact gttaaattgc tgtgtatctg atagctcttt 420
ggcagtctat atgtttgtat aatgaatgag agaataagtc atgttccttc aagatcatgt 480
accccaattt acttgccatt act 503

<210> 10
<211> 462
<212> DNA
<213> Homo sapiens

<400> 10

gtcatgaggc agctttcatc acaccctttt aacatttatc taaaagaatt taaattcttt 60
ttcaaaaatt acactacaag tttataagcc caaatggctc tgtgaaatca gaagtgcaaa 120
ggtgtgcaaa cttgtatctg aagacctacc agggacaagc aggtaagagc tgatgtgagt 180
gtgtgtgatg ggatctgtaa ggaactggaa cacacatgtc ctatccaaag gaatcagctg 240
cagctgcttg ttgtcaagta taaagtcagg acctggcttg gctttaaccg tttttcaaga 300
aaactggaaa tctggatttt cagcgaacat gcctgatttt aaaaggttga ctcaagtttt 360
tacaaaatac tatgtgggac acctcaaata catacctact gactgatgac aaacccagga 420
gtttgtgtgt cttttataaa aagtttgccc tggatgtcat at 462

<210> 11
<211> 334
<212> DNA
<213> Homo sapiens

<400> 11

gactcagcct ctgggatgga ttactggatt gttaaaaaca gctggggcac cggctggggt 60
gagaatggct acttccggat ccgcagagga actgatgagt gtgcaattga gagcatagca 120
gtggcagcca caccaattcc taaattgtag ggtatgcctt ccagtatttc ataatgatct 180
gcatcagttg taaaggggaa ttggtatatt cacagactgt agactttcag cagcaatctc 240
agaagcttac aaatagattt ccatgaagat atttgtcttc agaattaaaa ctgcccttaa 300
ttttaatata cctttcaatc ggccactggc catt 334

<210> 12
<211> 544
<212> DNA
<213> Homo sapiens

<400> 12

taactctacc ctggcactat aatgtaagct ctactgaggt gctatgttct tagtggatgt 60
tctgaccctg cttcaaatat ttccctcacc tttcccatct tccaagggta ctaaggaatc 120
tttctgcttt ggggtttatc agaattctca gaatctcaaa taactaaaag gtatgcaatc 180
aaatctgctt tttaaagaat gctctttact tcatggactt ccactgccat cctcccaagg 240
ggcccaaatt ctttcagtgg ctacctacat acaattccaa acacatacag gaaggtagaa 300
atatctgaaa atgtatgtgt aagtattctt atttaatgaa agactgtaca aagtataagt 360
cttagatgta tatatttcct atattgtttt cagtgtacat ggaataacat gtaattaagt 420
actatgtatc aatgagtaac aggaaaattt taaaaataca gatagatata tgctctgcat 480
gttacataag ataaatgtgc tgaatggttt tcaaataaaa atgaggtact ctcctggaaa 540
tatt 544

<210> 13
<211> 212
<212> DNA
<213> Homo sapiens

<400> 13

gcttctggag gcaccttcag aaattatagt ctcaactgcg tgcgacaggc ccctggacaa 60
gggcttgagt ggatgggagg gatcgtccct atctttggta catcaaacta cacacaaaag 120
ttccagggga gagtcacgat taccgcggac ttatccacca gcacagccta catggagctg 180
aacagcctga gatctgagga cacggccatg ca 212

<210> 14
<211> 404
<212> DNA
<213> Homo sapiens

<400> 14

aagcaccctg tatcacagac tggcaacaag aagatggtac cgtgcatcgc acctatttaa 60
gagggaactt agcagagagc aaatgctatt tgataacagt tactccagta tatgctgatg 120
gaccaggaag ccctgaatcc ataaaggcat accttaaaca agctccacct tccaaaggac 180
ctactgttcg gacaaaaaaa gtagggaaaa acgaagctgt cttagagtgg gaccaacttc 240
ctgttgatgt tcagaatgga tttatcagaa attatactat attttataga accatcattg 300
gaaatgaaac tgctgtgaat gtggattctt cccacacaga atatacattg tcctctttga 360
ctagtgacac attgtacatg gtacgaatgg cagcatacac agat 404

<210> 15
<211> 539
<212> DNA
<213> Homo sapiens

<400> 15

aggactcaag acatctcagt gttcttcacc gtctctttca gcatcaccag gcagtcccac 60
acgaccacag atacggcaaa agatagagaa taaacccctt caagaacaac tttctgttaa 120
ccaaattaaa actgaacctg tggattatga attcaaaccc atagtggttg cttcaggaat 180
caactgttca accccttac aaaatggggt tttcactggt ggtggcccat tacaggcaac 240
cagttctcct cagggcatgg tgcaagctgt tgttctgcca acagttggtt tggtgtctcc 300
cataagtatc aatttaagtg atattcagaa tgtacttaaa gtggcggtag atggtaatgt 360
aataaggcaa gtgttggaga ataatcaagc caatcttgca tccaaagaac aagaaacaat 420
caatgcttca cccatacaac aaggtggcca ttctgttatt tcagccatca gtcttccttt 480
ggttgatcaa gatggaacaa ccaaaattat catcaactac agtcttgagc agcctagcc 539

<210> 16
<211> 511
<212> DNA
<213> Homo sapiens

<900> 16

atgagatgta tcttttgctc tctcttgctc tcttatttgt accggttttt gtatataaaa 60
ttcatgtttc caatctctct ctccctgatc ggtgacagtc actagcttat cttgaacaga 120
tatttaattt tgctaacact cagctctgcc ctccccgatc ccctggctcc ccagcacaca 180
ttcctttgaa agagggtttc aatatacatc tacatactat atatatattg ggcaacttgt 240
atttgtgtgt atatatatat atatatgttt atgtatatat gtgatcctga aaaaataaac 300
atcgctattc tgttttttat atgttcaaac caaacaagaa aaaatagaga attctacata 360
ctaaatctct ctcctttttt aattttaata tttgttatca tttatttatt ggtgctactg 420

tttatccgta ataattgtgg ggaaaagata ttaacatcac gtctttgtct ctagtgcagt 480
ttttcgagat attccgtagt acatatttat t 511

<210> 17
<211> 552
<212> DNA
<213> Homo sapiens

<400> 17

aaagacactc ggcagcaaca tcagcaggga gatgaatcac agaaaatgag aggggactat 60
ggcaagttaa aggccctcat caatagtcgg aaaagtagaa acaggaggaa tcagttgcca 120
gagtcataat attttcttat gtgggtctta tgcttccatt aacaaatgct ctgtcttcaa 180
tgatcaaatt ttgagcaaag aaacttgtgc tttaccaagg ggaattactg aaaaaggtga 240
ttactcctga agtgagtttt acacgaactg aaatgagcat gcattttctt gtatgatagt 300
gactagcact agacatgtca tggtcctcat ggtgcatata aatatattta acttaaccca 360
gattttattt atatctttat tcaccttttc ttcaaaatcg atatggtggc tgcaaaacta 420
gaattgttgc atccctcaat tgaatgaggg ccatatccct gtggtattcc tttcctgctt 480
tggggcttta gaattctaat tgtcagtgat tttgtatatg aaaacaagtt ccaaatccac 540
agcttttacg ta 552

<210> 18
<211> 505
<212> DNA
<213> Homo sapiens

<400> 18

agcccagttt cactgccata tactcttcaa ggactttctg aagcctcact tatgagatgc 60

ctgaagccag gccatggcta taaacaatta catggctcta aaaagttttg cccttttaa 120

ggaaggcact aaaaagagct gtcctggtat ctagacccat cttcttttg aaatcagcat 180

actcaatgtt actatctgct tttggttata atgtgttttt aattatctaa agtatgaagc 240

attttctggg gttatgatgg ccttaccttt attaggaagt atggttttat tttgatagta 300

gcttcctcct ctggtggtgt taatcatttc atttttaccc ttactgtttg agtttctctc 360

acattactgt atatactttg cctttccata atcactcagt gattgcaatt tgcacaagtt 420

tttttaaatt atgggaatca agatttaatc ctagagattt ggtgtacaat tcaggctttg 480

gatgtttctt tagcagtttt gtgat 505

<210> 19
<211> 285
<212> DNA
<213> Homo sapiens

<400> 19

aagccatagt cgtgcctgtt tgcttagcat tcctattgac aactcttctg ggagtgctgt 60

tctgctttaa taagcgagac ctaattaaaa aacacatctg gcctaatgtt ccagatcctt 120

caaagagtca tattgcccag tggtcacctc acactcctcc aaggcacaat tttaattcaa 180

aagatcaaat gtattcagat ggcaatttca ctgatgtaag tgttgtggaa atagaagcaa 240

atgacaaaaa gccttttcca gaagatctga aatcattgga cctgt 285

<210> 20
<211> 519
<212> DNA
<213> Homo sapiens

<400> 20

ggcatctcct gagcctaggc aatacctgta gggtgacttc tggagccatc cccgtttccc 60

cgcccccaa aagaagcgga gatttaacgg ggacgtgcgg ccagagctgg ggaaatgggc 120

ccgcgagcca ggccggcgct tctcctcctg atgcttttgc agaccgcggt cctgcagggg 180

cgcttgctgc gttcacactc tctgcactac ctcttcatgg gtgcctcaga gcaggacctt 240

ggtctttcct tgtttgaagc tttgggctac gtggatgacc agctgttcgt gttctatgat 300

catgagagtc accgtgtgga gccccgaact ccatgggttt ccagtagaat ttcaagccag 360

atgtggctgc agctgagtca gagtctgccc agccgtcaaa agagtcttcc tatatatatc 420

cagatggcat gtgtttactt tatgttacta catgcacttg gctgcataaa tgtggtacaa 480

gcattctgtc ttgaagggca ggtgcttcag gataccata 519

<210> 21
<211> 491
<212> DNA
<213> Homo sapiens

<400> 21

```
tccaggctgg aagtacttgg ccccctcag gagcctggcc aggcagggag agagtagctg      60
cagccttcat cagaactctt cctcctccca aggcattctc ccagctctag cctctggact     120
ggaaagcaca agactggccc agtgccagca agtccttagg ctactgtaat gctgcctcag     180
gacccatccc tgcctggagg ctcctctagg ccctgtgagc acaaagaaga aagctgattt     240
ttgtctttta atccatttca ggactctctc caggagggct cggggtgtgt catttctata     300
ttcctccagc tgggattggg gggtgggctt tgttgtgaga atggcctgga gcaggcccaa     360
tgctgctttt ggggggtcagc atccagtgtg agatactgtg tatataaact atatataatg    420
tatataaact gggatgtaag tttgtgtaaa ttaatgtttt attctttgca aataaaacgc     480
tttccccgtc a                                                          491
```

<210> 22
<211> 267
<212> DNA
<213> Homo sapiens

<400> 22

```
gcctgaacct cagacctagt aatttttcat gcagttttca aagttaggaa caagtttgta      60
acatgcagca gattagaaaa ccttaatgac tcagagagca acaatacaag aggttaaagg     120
aagctgatta attagatatg catctggcat tgttttatct tatcagtatt atcactctta     180
tgttggttta ttcttaagct gtacaattgg gagaaatttt ataatttttt attggtaaac     240
atatgctaaa tccgcttcag tatttta                                         267
```

<210> 23
<211> 399
<212> DNA
<213> Homo sapiens

<400> 23

```
ttctttcccc aaatatcatg tagcacatca atatgtaggg aaacattctt atgcatcatt    60
tggtttgttt tataaccaat tcattaaatg taattcataa aatgtactat gaaaaaaatt   120
atacgctatg ggatactggc aacagtgcac atatttcata accaaattag cagcaccggt   180
cttaatttga tgtttttcaa cttttattca ttgagatgtt ttgaagcaat taggatatgt   240
gtgtttactg tactttttgt tttgatccgt ttgtataaat gatagcaata tcttggacac   300
atttgaaata caaaatgttt ttgtctacca aagaaaaatg ttgaaaaata agcaaatgta   360
tacctagcaa tcacttttac tttttgtaat tctgtctct                          399
```

<210> 24
<211> 25
<212> DNA
<213> Artificial

<220>
<223> A sequence of a probe contained in a probe set

<400> 24
agctctcaga caaaggctgc cttgc 25

<210> 25
<211> 25
<212> DNA
<213> Artificial

<220>
<223> A sequence of a probe contained in a probe set

<400> 25
gggagccgcc cgagaaggag cggcg 25

<210> 26
<211> 25
<212> DNA
<213> Artificial

<220>
<223> A sequence of a probe contained in a probe set

<400> 26
aagagagttt tgagaactac cgcag 25

<210> 27
<211> 25
<212> DNA
<213> Artificial

<220>
<223> A sequence of a probe contained in a probe set

<400> 27
gcagaaagga tggcggcagg gggag 25

<210> 28
<211> 25
<212> DNA
<213> Artificial

<220>
<223> A sequence of a probe contained in a probe set

<400> 28
tcgctgactt ggcctggaac gagga 25

<210> 29
<211> 25
<212> DNA
<213> Artificial

<220>
<223> A sequence of a probe contained in a probe set

<400> 29
gggcattggg gccgtttgtt aagcg 25

<210> 30
<211> 25
<212> DNA
<213> Artificial

<220>
<223> A sequence of a probe contained in a probe set

<400> 30
ggggccgttt gttaagcggc actca 25

<210> 31
<211> 25
<212> DNA
<213> Artificial

<220>
<223> A sequence of a probe contained in a probe set

<400> 31
taagcggcac tcattttgcg gaggc 25

<210> 32
<211> 25
<212> DNA
<213> Artificial

<220>
<223> A sequence of a probe contained in a probe set

<400> 32
acgccatctg tgtaacttca ggatc 25.

<210> 33
<211> 25
<212> DNA

<213> Artificial

<220>
<223> A sequence of a probe contained in a probe set

<400> 33
cttcaggatc tgttctgttt cacca 25

<210> 34
<211> 25
<212> DNA
<213> Artificial

<220>
<223> A sequence of a probe contained in a probe set

<400> 34
gttctgtttc accatgtaac acaca 25

<210> 35
<211> 25
<212> DNA
<213> Artificial

<220>
<223> A sequence of a probe contained in a probe set

<400> 35
acccccagct atcagacggt ctggt 25

<210> 36
<211> 25
<212> DNA
<213> Artificial

<220>
<223> A sequence of a probe contained in a probe set

<400> 36
aggccaaagcagcgtctttc agtgc 25

<210> 37
<211> 25
<212> DNA
<213> Artificial

<220>
<223> A sequence of a probe contained in a probe set

<400> 37
gctggtggag gacatgctgc tcagt 25

<210> 38
<211> 25
<212> DNA
<213> Artificial

<220>

<223> A sequence of a probe contained in a probe set

<400> 38
tgctcagttc ctccaggaca tgaga 25

<210> 39
<211> 25
<212> DNA
<213> Artificial

<220>
<223> A sequence of a probe contained in a probe set

<400> 39
ggaacttcct gtgctcatta gagtc 25

<210> 40
<211> 25
<212> DNA
<213> Artificial

<220>
<223> A sequence of a probe contained in a probe set

<400> 40
attagagtca aatccctcag tccgt 25

<210> 41
<211> 25
<212> DNA
<213> Artificial

<220>
<223> A sequence of a probe contained in a probe set

<400> 41
atgacgcctg tgtgaaagct ctggt 25

<210> 42
<211> 25
<212> DNA
<213> Artificial

<220>
<223> A sequence of a probe contained in a probe set

<400> 42
tggtctccct gaggagctac catct 25

<210> 43
<211> 25
<212> DNA
<213> Artificial

<220>
<223> A sequence of a probe contained in a probe set

<400> 43

gagctaccat ctgcaaatcg tgact 25

<210> 44
<211> 25
<212> DNA
<213> Artificial

<220>
<223> A sequence of a probe contained in a probe set

<400> 44
catcctgatt cctgcaagcc agcag 25

<210> 45
<211> 25
<212> DNA
<213> Artificial

<220>
<223> A sequence of a probe contained in a probe set

<400> 45
gaataagacc tctgaagacc cttca 25

<210> 46
<211> 25
<212> DNA
<213> Artificial

<220>
<223> A sequence of a probe contained in a probe set

<400> 46
gattatcaat taccacacca tctcc 25

<210> 47
<211> 25
<212> DNA
<213> Artificial

<220>
<223> A sequence of a probe contained in a probe set

<400> 47
gaagcatgat tggtgcccag ttagc 25

<210> 48
<211> 25
<212> DNA
<213> Artificial

<220>
<223> A sequence of a probe contained in a probe set

<400> 48
cccagttagc ctctgcagga tgtgg 25

<210> 49

<211> 25
<212> DNA
<213> Artificial

<220>
<223> A sequence of a probe contained in a probe set

<400> 49
gtaggagagg ttgtctgtgg ccaga 25

<210> 50
<211> 25
<212> DNA
<213> Artificial

<220>
<223> A sequence of a probe contained in a probe set

<400> 50
acctatactc actttcccaa attga 25

<210> 51
<211> 25
<212> DNA
<213> Artificial

<220>
<223> A sequence of a probe contained in a probe set

<400> 51
ttgaatcact gctcacactg ctgat 25

<210> 52
<211> 25
<212> DNA
<213> Artificial

<220>
<223> A sequence of a probe contained in a probe set

<400> 52
tagagtgctg tccggtggag atccc 25

<210> 53
<211> 25
<212> DNA
<213> Artificial

<220>
<223> A sequence of a probe contained in a probe set

<400> 53
ccacccgaac gtcttatcta atcat 25

<210> 54
<211> 25
<212> DNA
<213> Artificial

<220>
<223> A sequence of a probe contained in a probe set

<400> 54
atcatgaaac tccctagttc cttca 25

<210> 55
<211> 25
<212> DNA
<213> Artificial

<220>
<223> A sequence of a probe contained in a probe set

<400> 55
gttccttcat gtaacttccc tgaaa 25

<210> 56
<211> 25
<212> DNA
<213> Artificial

<220>
<223> A sequence of a probe contained in a probe set

<400> 56
tttgagagtc tgtgacccac ttacc 25

<210> 57
<211> 25
<212> DNA
<213> Artificial

<220>
<223> A sequence of a probe contained in a probe set

<400> 57
gcttctcact catcactaac aaggt 25

<210> 58
<211> 25
<212> DNA
<213> Artificial

<220>
<223> A sequence of a probe contained in a probe set

<400> 58
gaacagtttg tctccattct tatgg 25

<210> 59
<211> 25
<212> DNA
<213> Artificial

<220>
<223> A sequence of a probe contained in a probe set

<400> 59
tccattctta tggccagcat tccac 25

<210> 60
<211> 25
<212> DNA
<213> Artificial

<220>
<223> A sequence of a probe contained in a probe set

<400> 60
actccctgac aaagccagtt gacct 25

<210> 61
<211> 25
<212> DNA
<213> Artificial

<220>
<223> A sequence of a probe contained in a probe set

<400> 61
tcccacacaa gacccaagta gctgc 25

<210> 62
<211> 25
<212> DNA
<213> Artificial

<220>
<223> A sequence of a probe contained in a probe set

<400> 62
ccaagtagct gctaccttct ttggc 25

<210> 63
<211> 25
<212> DNA
<213> Artificial

<220>
<223> A sequence of a probe contained in a probe set

<400> 63
tctaccagac ccttctggtg ccaga 25

<210> 64
<211> 25
<212> DNA
<213> Artificial

<220>
<223> A sequence of a probe contained in a probe set

<400> 64
tcattcctgt tctttcttac acaag 25

<210> 65
<211> 25
<212> DNA
<213> Artificial

<220>
<223> A sequence of a probe contained in a probe set

<400> 65
gactcgggcc ttagaacttt gcata 25

<210> 66
<211> 25
<212> DNA
<213> Artificial

<220>
<223> A sequence of a probe contained in a probe set

<400> 66
atagcagctg ctactagctc tttga 25

<210> 67
<211> 25
<212> DNA
<213> Artificial

<220>
<223> A sequence of a probe contained in a probe set

<400> 67
atacattccg aggggctcag ttctg 25

<210> 68
<211> 25
<212> DNA
<213> Artificial

<220>
<223> A sequence of a probe contained in a probe set

<400> 68
gagacttttc tatggttttg tgact 25

<210> 69
<211> 25
<212> DNA
<213> Artificial

<220>
<223> A sequence of a probe contained in a probe set

<400> 69
gtgactttca acttttgtac agtta 25

<210> 70
<211> 25
<212> DNA

<213> Artificial

<220>
<223> A sequence of a probe contained in a probe set

<400> 70
gacaatcaga attccactgc ccaaa 25

<210> 71
<211> 25
<212> DNA
<213> Artificial

<220>
<223> A sequence of a probe contained in a probe set

<400> 71
ggctggttac catcggagtt tacaa 25

<210> 72
<211> 25
<212> DNA
<213> Artificial

<220>
<223> A sequence of a probe contained in a probe set

<400> 72
tgctttcacg ttcttacttg ttgta 25

<210> 73
<211> 25
<212> DNA
<213> Artificial

<220>
<223> A sequence of a probe contained in a probe set

<400> 73
tacattcatg catttctagg ctaga 25

<210> 74
<211> 25
<212> DNA
<213> Artificial

<220>
<223> A sequence of a probe contained in a probe set

<400> 74
agagaacctt ctagatttga tgctt 25

<210> 75
<211> 25
<212> DNA
<213> Artificial

<220>

<223> A sequence of a probe contained in a probe set

<400> 75
gtctctagaa gttatctgtc tgtat 25

<210> 76
<211> 25
<212> DNA
<213> Artificial

<220>
<223> A sequence of a probe contained in a probe set

<400> 76
ctgtctgtat tgatctttat gctat 25

<210> 77
<211> 25
<212> DNA
<213> Artificial

<220>
<223> A sequence of a probe contained in a probe set

<400> 77
gctatattac tatctgtggt tacag 25

<210> 78
<211> 25
<212> DNA
<213> Artificial

<220>
<223> A sequence of a probe contained in a probe set

<400> 78
tactggagtc aagcccttat aagtc 25

<210> 79
<211> 25
<212> DNA
<213> Artificial

<220>
<223> A sequence of a probe contained in a probe set

<400> 79
cgccggcaaa actttggcac tgggg 25

<210> 80
<211> 25
<212> DNA
<213> Artificial

<220>
<223> A sequence of a probe contained in a probe set

<400> 80

caaaccaacc gcacatgcag atggg 25

<210> 81
<211> 25
<212> DNA
<213> Artificial

<220>
<223> A sequence of a probe contained in a probe set

<400> 81
gaagaaacgt ctatgtgtac agatg 25

<210> 82
<211> 25
<212> DNA
<213> Artificial

<220>
<223> A sequence of a probe contained in a probe set

<400> 82
gcgggccggt ttcgaagttg atgca 25

<210> 83
<211> 25
<212> DNA
<213> Artificial

<220>
<223> A sequence of a probe contained in a probe set

<400> 83
gatgcaatcg gtttaaacat ggctg 25

<210> 84
<211> 25
<212> DNA
<213> Artificial

<220>
<223> A sequence of a probe contained in a probe set

<900> 84
aacatggctg aacgcgtgtg tacac 25

<210> 85
<211> 25
<212> DNA
<213> Artificial

<220>
<223> A sequence of a probe contained in a probe set

<400> 85
gtgtgtacac gggactgacg caacc 25

<210> 86

<211> 25
<212> DNA
<213> Artificial

<220>
<223> A sequence of a probe contained in a probe set

<400> 86
tgacgcaacc cacgtgtaac tgtca 25

<210> 87
<211> 25
<212> DNA
<213> Artificial

<220>
<223> A sequence of a probe contained in a probe set

<400> 87
gggccctgag taatcgctta aagat 25

<210> 88
<211> 25
<212> DNA
<213> Artificial

<220>
<223> A sequence of a probe contained in a probe set

<400> 88
aaagatgttc ctacgggctt gttgc 25

<210> 89
<211> 25
<212> DNA
<213> Artificial

<220>
<223> A sequence of a probe contained in a probe set

<400> 89
gggcttgttg ctgttgatgt tttgt 25

<210> 90
<211> 25
<212> DNA
<213> Artificial

<220>
<223> A sequence of a probe contained in a probe set

<400> 90
tccatttctc atgttttcca ttgtt 25

<210> 91
<211> 25
<212> DNA
<213> Artificial

<220>
<223> A sequence of a probe contained in a probe set

<400> 91
caagaagcct ttcctgtagc cttct 25

<210> 92
<211> 25
<212> DNA
<213> Artificial

<220>
<223> A sequence of a probe contained in a probe set

<400> 92
gtccacggtc tgttcttgaa gcagt 25

<210> 93
<211> 25
<212> DNA
<213> Artificial

<220>
<223> A sequence of a probe contained in a probe set

<400> 93
tgaagcagta gcctaacaca ctcca 25

<210> 94
<211> 25
<212> DNA
<213> Artificial

<220>
<223> A sequence of a probe contained in a probe set

<400> 94
aagatatgga cacacgggag ccgct 25

<210> 95
<211> 25
<212> DNA
<213> Artificial

<220>
<223> A sequence of a probe contained in a probe set

<400> 95
gttttagcca ttgttggctt tccct 25

<210> 96
<211> 25
<212> DNA
<213> Artificial

<220>
<223> A sequence of a probe contained in a probe set

<400> 96
tggctttccc ttatcaaact tgggc 25

<210> 97
<211> 25
<212> DNA
<213> Artificial

<220>
<223> A sequence of a probe contained in a probe set

<400> 97
ctgagttata tgttcactgt ccccc 25

<210> 98
<211> 25
<212> DNA
<213> Artificial

<220>
<223> A sequence of a probe contained in a probe set

<400> 98
gttcactgtc cccctaatat taggg 25

<210> 99
<211> 25
<212> DNA
<213> Artificial

<220>
<223> A sequence of a probe contained in a probe set

<400> 99
gaacecccat gatgtaagtt tacct 25

<210> 100
<211> 25
<212> DNA
<213> Artificial

<220>
<223> A sequence of a probe contained in a probe set

<400> 100
aaacctgcac ttatacccat gaact 25

<210> 101
<211> 25
<212> DNA
<213> Artificial

<220>
<223> A sequence of a probe contained in a probe set

<400> 101
aacacttcga gcactgtcca gtatt 25

<210> 102
<211> 25
<212> DNA
<213> Artificial

<220>
<223> A sequence of a probe contained in a probe set

<400> 102
tgtccagtat tctaccgtgg tacac 25

<210> 103
<211> 25
<212> DNA
<213> Artificial

<220>
<223> A sequence of a probe contained in a probe set

<400> 103
tacagacacc aagttccgtc agtcc 25

<210> 104
<211> 25
<212> DNA
<213> Artificial

<220>
<223> A sequence of a probe contained in a probe set

<400> 104
gtcttctcaa gatccgagtc taccc 25

<210> 105
<211> 25
<212> DNA
<213> Artificial

<220>
<223> A sequence of a probe contained in a probe set

<400> 105
ctacccagcc cttgttagat tcaga 25

<210> 106
<211> 25
<212> DNA
<213> Artificial

<220>
<223> A sequence of a probe contained in a probe set

<400> 106
gcggtgatgg tattttgccc aggca 25

<210> 107
<211> 25
<212> DNA

<213> Artificial

<220>
<223> A sequence of a probe contained in a probe set

<400> 107
gcagatgctt ttggtccagg tactg 25

<210> 108
<211> 25
<212> DNA
<213> Artificial

<220>
<223> A sequence of a probe contained in a probe set

<400> 108
ggctgcgact gatgaaggca tgcct 25

<210> 109
<211> 25
<212> DNA
<213> Artificial

<220>
<223> A sequence of a probe contained in a probe set

<400> 109
accacagact gtacggcaag gcggc 25

<210> 110
<211> 25
<212> DNA
<213> Artificial

<220>
<223> A sequence of a probe contained in a probe set

<400> 110
ggcggctaca tgcctcagtg aagga 25

<210> 111
<211> 25
<212> DNA
<213> Artificial

<220>
<223> A sequence of a probe contained in a probe set

<400> 111
tagtagttcc tgctacaact tcagc 25

<210> 112
<211> 25
<212> DNA
<213> Artificial

<220>

<223> A sequence of a probe contained in a probe set

<400> 112
agcccctgcc catctgggaa aatac 25

<210> 113
<211> 25
<212> DNA
<213> Artificial

<220>
<223> A sequence of a probe contained in a probe set

<400> 113
gaaaataccc catcattcat gctac 25

<210> 114
<211> 25
<212> DNA
<213> Artificial

<220>
<223> A sequence of a probe contained in a probe set

<400> 114
ggctatggga gcagcttttt tttcc 25

<210> 115
<211> 25
<212> DNA
<213> Artificial

<220>
<223> A sequence of a probe contained in a probe set

<400> 115
gtcatccaat ctaaccacat caata 25

<210> 116
<211> 25
<212> DNA
<213> Artificial

<220>
<223> A sequence of a probe contained in a probe set

<400> 116
cttgttcaca ctctcacatg tttag 25

<210> 117
<211> 25
<212> DNA
<213> Artificial

<220>
<223> A sequence of a probe contained in a probe set

<400> 117

ttataatctg atttgttttc ctctt 25

<210> 118
<211> 25
<212> DNA
<213> Artificial

<220>
<223> A sequence of a probe contained in a probe set

<400> 118
gaccacaaat agtgctcgct ttcta 25

<210> 119
<211> 25
<212> DNA
<213> Artificial

<220>
<223> A sequence of a probe contained in a probe set

<400> 119
gtgctcgctt tctatgtagt ttggt 25

<210> 120
<211> 25
<212> DNA
<213> Artificial

<220>
<223> A sequence of a probe contained in a probe set

<400> 120
gaagactcta ccagactgtg tattc 25

<210> 121
<211> 25
<212> DNA
<213> Artificial

<220>
<223> A sequence of a probe contained in a probe set

<400> 121
tgttccttca agatcatgta cccca 25

<210> 122
<211> 25
<212> DNA
<213> Artificial

<220>
<223> A sequence of a probe contained in a probe set

<400> 122
gtaccccaat ttacttgcca ttact 25

<210> 123

<211> 25
<212> DNA
<213> Artificial

<220>
<223> A sequence of a probe contained in a probe set

<400> 123
gtcatgaggc agctttcatc acacc 25

<210> 124
<211> 25
<212> DNA
<213> Artificial

<220>
<223> A sequence of a probe contained in a probe set

<400> 124
tcatcacacc cttttaacat ttatc 25

<210> 125
<211> 25
<212> DNA
<213> Artificial

<220>
<223> A sequence of a probe contained in a probe set

<400> 125
aaaggtgtgc aaacttgtat ctgaa 25

<210> 126
<211> 25
<212> DNA
<213> Artificial

<220>
<223> A sequence of a probe contained in a probe set

<400> 126
gtatctgaag acetaccagg gacaa 25

<210> 127
<211> 25
<212> DNA
<213> Artificial

<220>
<223> A sequence of a probe contained in a probe set

<400> 127
ggaacacaca tgtcctatcc aaagg 25

<210> 128
<211> 25
<212> DNA
<213> Artificial

<220>
<223> A sequence of a probe contained in a probe set

<400> 128
ctatccaaag gaatcagctg cagct 25

<210> 129
<211> 25
<212> DNA
<213> Artificial

<220>
<223> A sequence of a probe contained in a probe set

<400> 129
taaagtcagg acctggcttg gcttt 25

<210> 130
<211> 25
<212> DNA
<213> Artificial

<220>
<223> A sequence of a probe contained in a probe set

<400> 130
tctggatttt cagcgaacat gcctg 25

<210> 131
<211> 25
<212> DNA
<213> Artificial

<220>
<223> A sequence of a probe contained in a probe set

<400> 131
ggacacctca aatacatacc tactg 25

<210> 132
<211> 25
<212> DNA
<213> Artificial

<220>
<223> A sequence of a probe contained in a probe set

<400> 132
actgactgat gacaaaccca ggagt 25

<210> 133
<211> 25
<212> DNA
<213> Artificial

<220>
<223> A sequence of a probe contained in a probe set

<400> 133
aaaaagtttg ccctggatgt catat 25

<210> 134
<211> 25
<212> DNA
<213> Artificial

<220>
<223> A sequence of a probe contained in a probe set

<400> 134
gactcagcct ctgggatgga ttact 25

<210> 135
<211> 25
<212> DNA
<213> Artificial

<220>
<223> A sequence of a probe contained in a probe set

<400> 135
ggtgagaatg gctacttccg gatcc 25

<210> 136
<211> 25
<212> DNA
<213> Artificial

<220>
<223> A sequence of a probe contained in a probe set

<400> 136
acttccggat ccgcagagga actga 25

<210> 137
<211> 25
<212> DNA
<213> Artificial

<220>
<223> A sequence of a probe contained in a probe set

<400> 137
gagagcatag cagtggcagc cacac 25

<210> 138
<211> 25
<212> DNA
<213> Artificial

<220>
<223> A sequence of a probe contained in a probe set

<400> 138
gcagccacac caattcctaa attgt 25

<210> 139
<211> 25
<212> DNA
<213> Artificial

<220>
<223> A sequence of a probe contained in a probe set

<400> 139
gtagggtatg ccttccagta tttca 25

<210> 140
<211> 25
<212> DNA
<213> Artificial

<220>
<223> A sequence of a probe contained in a probe set

<400> 140
gccttccagt atttcataat gatct 25

<210> 141
<211> 25
<212> DNA
<213> Artificial

<220>
<223> A sequence of a probe contained in a probe set

<400> 141
gatctgcatc agttgtaaag gggaa 25

<210> 142
<211> 25
<212> DNA
<213> Artificial

<220>
<223> A sequence of a probe contained in a probe set

<400> 142
aattggtata ttcacagact gtaga 25

<210> 143
<211> 25
<212> DNA
<213> Artificial

<220>
<223> A sequence of a probe contained in a probe set

<400> 143
gactgtagac tttcagcagc aatct 25

<210> 144
<211> 25
<212> DNA

<213> Artificial

<220>
<223> A sequence of a probe contained in a probe set

<400> 144
acctttcaat cggccactgg ccatt 25

<210> 145
<211> 25
<212> DNA
<213> Artificial

<220>
<223> A sequence of a probe contained in a probe set

<400> 145
taactctacc ctggcactat aatgt 25

<210> 146
<211> 25
<212> DNA
<213> Artificial

<220>
<223> A sequence of a probe contained in a probe set

<400> 146
gctctactga ggtgctatgt tctta 25

<210> 147
<211> 25
<212> DNA
<213> Artificial

<220>
<223> A sequence of a probe contained in a probe set

<400> 147
tcttagtgga tgttctgacc ctgct 25

<210> 148
<211> 25
<212> DNA
<213> Artificial

<220>
<223> A sequence of a probe contained in a probe set

<400> 148
tctgaccctg cttcaaatat ttccc 25

<210> 149
<211> 25
<212> DNA
<213> Artificial

<220>

<223> A sequence of a probe contained in a probe set

<400> .149
ggtactaagg aatctttctg ctttg 25

<210> 150
<211> 25
<212> DNA
<213> Artificial

<220>
<223> A sequence of a probe contained in a probe set

<400> 150
gctttggggt ttatcagaat tctca 25

<210> 151
<211> 25
<212> DNA
<213> Artificial

<220>
<223> A sequence of a probe contained in a probe set

<400> 151
aatgctcttt acttcatgga cttcc 25

<210> 152
<211> 25
<212> DNA
<213> Artificial

<220>
<223> A sequence of a probe contained in a probe set

<400> 152
ttacttcatg gacttccact gccat 25

<210> 153
<211> 25
<212> DNA
<213> Artificial

<220>
<223> A sequence of a probe contained in a probe set

<400> 153
attctttcag tggctaccta catac 25

<210> 154
<211> 25
<212> DNA
<213> Artificial

<220>
<223> A sequence of a probe contained in a probe set

<400> 154

gctacctaca tacaattcca aacac 25

<210> 155
<211> 25
<212> DNA
<213> Artificial

<220>
<223> A sequence of a probe contained in a probe set

<400> 155
aatgaggtac tctcctggaa atatt 25

<210> 156
<211> 25
<212> DNA
<213> Artificial

<220>
<223> A sequence of a probe contained in a probe set

<400> 156
gcttctggag gcaccttcag aaatt 25

<210> 157
<211> 25
<212> DNA
<213> Artificial

<220>
<223> A sequence of a probe contained in a probe set

<400> 157
atagtctcaa ctgcgtgcga caggc 25

<210> 158
<211> 25
<212> DNA
<213> Artificial

<220>
<223> A sequence of a probe contained in a probe set

<400> 158
gacaggcccc tggacaaggg cttga 25

<210> 159
<211> 25
<212> DNA
<213> Artificial

<220>
<223> A sequence of a probe contained in a probe set

<400> 159
gagtggatgg gagggatcgt cccta 25

<210> 160

<211> 25
<212> DNA
<213> Artificial

<220>
<223> A sequence of a probe contained in a probe set

<400> 160
atcgtcccta tctttggtac atcaa 25

<210> 161
<211> 25
<212> DNA
<213> Artificial

<220>
<223> A sequence of a probe contained in a probe set

<400> 161
ttggtacatc aaactacaca caaaa 25

<210> 162
<211> 25
<212> DNA
<213> Artificial

<220>
<223> A sequence of a probe contained in a probe set

<400> 162
tacacacaaa agttccaggg gagag 25

<210> 163
<211> 25
<212> DNA
<213> Artificial

<220>
<223> A sequence of a probe contained in a probe set

<400> 163
ggagagtcac gattaccgcg gactt 25

<210> 164
<211> 25
<212> DNA
<213> Artificial

<220>
<223> A sequence of a probe contained in a probe set

<400> 164
caccagcaca gcctacatgg agctg 25

<210> 165
<211> 25
<212> DNA
<213> Artificial

<220>
<223> A sequence of a probe contained in a probe set

<400> 165
ggagctgaac agcctgagat ctgag 25

<210> 166
<211> 25
<212> DNA
<213> Artificial

<220>
<223> A sequence of a probe contained in a probe set.

<400> 166
tgagatctga ggacacggcc atgca 25

<210> 167
<211> 25
<212> DNA
<213> Artificial

<220>
<223> A sequence of a probe contained in a probe set

<400> 167
aagcaccctg tatcacagac tggca 25

<210> 168
<211> 25
<212> DMA
<213> Artificial

<220>
<223> A sequence of a probe contained in a probe set

<400> 168
aacagttact ccagtatatg ctgat 25

<210> 169
<211> 25
<212> DNA
<213> Artificial

<220>
<223> A sequence of a probe contained in a probe set

<400> 169
gatggaccag gaagccctga atcca 25

<210> 170
<211> 25
<212> DNA
<213> Artificial

<220>
<223> A sequence of a probe contained in a probe set

<400> 170
ggcatacctt aaacaagctc cacct 25

<210> 171
<211> 25
<212> DNA
<213> Artificial

<220>
<223> A sequence of a probe contained in a probe set

<400> 171
taaacaagct ccaccttcca aagga 25

<210> 172
<211> 25
<212> DNA
<213> Artificial

<220>
<223> A sequence of a probe contained in a probe set

<400> 172
ttccaaagga cctactgttc ggaca 25

<210> 173
<211> 25
<212> DNA
<213> Artificial

<220>
<223> A sequence of a probe contained in a probe set

<400> 173
acgaagctgt cttagagtgg gacca 25

<210> 174
<211> 25
<212> DNA
<213> Artificial

<220>
<223> A sequence of a probe contained in a probe set

<400> 174
gtgggaccaa cttcctgttg atgtt 25

<210> 175
<211> 25
<212> DNA
<213> Artificial

<220>
<223> A sequence of a probe contained in a probe set

<400> 175
gtgaatgtgg attcttccca cacag 25

<210> 176
<211> 25
<212> DNA
<213> Artificial

<220>
<223> A sequence of a probe contained in a probe set

<400> 176
tacattgtcc tctttgacta gtgac 25

<210> 177
<211> 25
<212> DNA
<213> Artificial

<220>
<223> A sequence of a probe contained in a probe set

<400> 177
ggtacgaatg gcagcataca cagat 25

<210> 178
<211> 25
<212> DNA
<213> Artificial

<220>
<223> A sequence of a probe contained in a probe set

<400> 178
aggactcaag acatctcagt gttct 25

<210> 179
<211> 25
<212> DNA
<213> Artificial

<220>
<223> A sequence of a probe contained in a probe set

<400> 179
cccacacgac cacagatacg gcaaa 25

<210> 180
<211> 25
<212> DNA
<213> Artificial

<220>
<223> A sequence of a probe contained in a probe set

<400> 180
acccatagtg gttgcttcag gaatc 25

<210> 181
<211> 25
<212> DNA

<213> Artificial

<220>
<223> A sequence of a probe contained in a probe set

<400> 181
ttttcactgg tggtggccca ttaca 25

<210> 182
<211> 25
<212> DNA
<213> Artificial

<220>
<223> A sequence of a probe contained in a probe set

<400> 182
ggcccattac aggcaaccag ttctc 25

<210> 183
<211> 25
<212> DNA
<213> Artificial

<220>
<223> A sequence of a probe contained in a probe set

<400> 183
gttctcctca gggcatggtg caagc 25

<210> 1.84
<211> 25
<212> DNA
<213> Artificial

<220>
<223> A sequence of a probe contained in a probe set

<400> 184
ggtgcaagct gttgttctgc caaca 25

<210> 185
<211> 25
<212> DNA
<213> Artificial

<220>
<223> A sequence of a probe contained in a probe set

<400> 185
aacagttggt ttggtgtctc ccata 25

<210> 186
<211> 25
<212> DNA
<213> Artificial

<220>

<223> A sequence of a probe contained in a probe set

<400> 186
gaaacaatca atgcttcacc catac 25

<210> 187
<211> 25
<212> DNA
<213> Artificial

<220>
<223> A sequence of a probe contained in a probe set

<400> 187
caaggtggcc attctgttat ttcag 25

<210> 188
<211> 25
<212> DNA
<213> Artificial

<220>
<223> A sequence of a probe contained in a probe set

<400> 188
aactacagtc ttgagcagcc tagcc 25

<210> 189
<211> 25
<212> DNA
<213> Artificial

<220>
<223> A sequence of a probe contained in a probe set

<400> 189
atgagatgta tcttttgctc tctct 25

<210> 190
<211> 25
<212> DNA
<213> Artificial

<220>
<223> A sequence of a probe contained in a probe set

<900> 190
ccctgatcgg tgacagtcac tagct 25

<210> 191
<211> 25
<212> DNA
<213> Artificial

<220>
<223> A sequence of a probe contained in a probe set

<400> 191

tgacagtcac tagcttatct tgaac 25

<210> 192
<211> 25
<212> DNA
<213> Artificial

<220>
<223> A sequence of a probe contained in a probe set

<400> 192
acagatattt aattttgcta acact 25

<210> 193
<211> 25
<212> DNA
<213> Artificial

<220>
<223> A sequence of a probe contained in a probe set

<400> 193
gaattctaca tactaaatct ctctc 25

<210> 194
<211> 25
<212> DNA
<213> Artificial

<220>
<223> A sequence of a probe contained in a probe set

<400> 194
tctacatact aaatctctct ccttt 25

<210> 195
<211> 25
<212> DNA
<213> Artificial

<220>
<223> A sequence of a probe contained in a probe set

<400> 195
ggtgctactg tttatccgta ataat 25

<210> 196
<211> 25
<212> DNA
<213> Artificial

<220>
<223> A sequence of a probe contained in a probe set

<400> 196
cgtctttgtc tctagtgcag ttttt 25

<210> 197

<211> 25
<212> DNA
<213> Artificial

<220>
<223> A sequence of a probe contained in a probe set

<400> 197
agtgcagttt ttcgagatat tccgt 25

<210> 198
<211> 25
<212> DNA
<213> Artificial

<220>
<223> A sequence of a probe contained in a probe set

<400> 198
gtttttcgag atattccgta gtaca 25

<210> 199
<211> 25
<212> DNA
<213> Artificial

<220>
<223> A sequence of a probe contained in a probe set

<400> 199
agatattccg tagtacatat ttatt 25

<210> 200
<211> 25
<212> DNA
<213> Artificial

<220>
<223> A sequence of a probe contained in a probe set

<400> 200
aaagacactc ggcagcaaca tcagc 25

<210> 201
<211> 25
<212> DNA
<213> Artificial

<220>
<223> A sequence of a probe contained in a probe set

<400> 201
aaaggccctc atcaatagtc ggaaa 25

<210> 202
<211> 25
<212> DNA
<213> Artificial

<220>
<223> A sequence of a probe contained in a probe set

<400> 202
atgtgggtct tatgcttcca ttaac 25

<210> 203
<211> 25
<212> DNA
<213> Artificial

<220>
<223> A sequence of a probe contained in a probe set

<400> 203
attaacaaat gctctgtctt caatg 25

<210> 204
<211> 25
<212> DNA
<213> Artificial

<220>
<223> A sequence of a probe contained in a probe set

<400> 204
atgagcatgc attttcttgt atgat 25

<210> 205
<211> 25
<212> DNA
<213> Artificial

<220>
<223> A sequence of a probe contained in a probe set

<400> 205
attcaccttt tcttcaaaat cgata 25

<210> 206
<211> 25
<212> DNA
<213> Artificial

<220>
<223> A sequence of a probe contained in a probe set

<400> 206
aaatcgatat ggtggctgca aaact 25

<210> 207
<211> 25
<212> DNA
<213> Artificial

<220>
<223> A sequence of a probe contained in a probe set

<400> 207
aaactagaat tgttgcatcc ctcaa 25

<210> 208
<211> 25
<212> DNA
<213> Artificial

<220>
<223> A sequence of a probe contained in a probe set

<400> 208
gagggccata tccctgtggt attcc 25

<210> 209
<211> 25
<212> DNA
<213> Artificial

<220>
<223> A sequence of a probe contained in a probe set

<400> 209
tcctgctttg gggctttaga attct 25

<210> 210
<211> 25
<212> DNA
<213> Artificial

<220>
<223> A sequence of a probe contained in a probe set

<400> 210
gttccaaatc cacagctttt acgta 25

<210> 211
<211> 25
<212> DNA
<213> Artificial

<220>
<223> A sequence of a probe contained in a probe set

<400> 211
agcacagttt cactgccata tactc 25

<210> 212
<211> 25
<212> DNA
<213> Artificial

<220>
<223> A sequence of a probe contained in a probe set

<400> 212
aggactttct gaagcctcac ttatg 25

<210> 213
<211> 25
<212> DNA
<213> Artificial

<220>
<223> A sequence of a probe contained in a probe set

<400> 213
gaagcctcac ttatgagatg cctga 25

<210> 214
<211> 25
<212> DNA
<213> Artificial

<220>
<223> A sequence of a probe contained in a probe set

<400> 214
atgcctgaag ccaggccatg gctat 25

<210> 215
<211> 25
<212> DNA
<213> Artificial

<220>
<223> A sequence of a probe contained in a probe set

<900> 215
agagctgtcc tggtatctag accca 25

<210> 216
<211> 25
<212> DNA
<213> Artificial

<220>
<223> A sequence of a probe contained in a probe set

<400> 216
tatctagacc catcttcttt ttgaa 25

<210> 217
<211> 25
<212> DNA
<213> Artificial

<220>
<223> A sequence of a probe contained in a probe set

<400> 217
atgttactat ctgcttttgg ttata 25

<210> 218
<211> 25
<212> DNA

<213> Artificial

<220>
<223> A sequence of a probe contained in a probe set

<400> 218
ttttctgggg ttatgatggc catac 25

<210> 219
<211> 25
<212> DNA
<213> Artificial

<220>
<223> A sequence of a probe contained in a probe set

<400> 219
gagtttctct cacattactg tatat 25

<210> 220
<211> 25
<212> DNA
<213> Artificial

<220>
<223> A sequence of a probe contained in a probe set

<400> 220
aattcaggct ttggatgttt cttta 25

<210> 221
<211> 25
<212> DNA
<213> Artificial

<220>
<223> A sequence of a probe contained in a probe set

<400> 221
gatgtttctt tagcagtttt gtgat 25

<210> 222
<211> 25
<212> DNA
<213> Artificial

<220>
<223> A sequence of a probe contained in a probe set

<400> 222
aagccatagt cgtgcctgtt tgctt 25

<210> 223
<211> 25
<212> DNA
<213> Artificial

<220>

<223> A sequence of a probe contained in a probe set

<400> 223
atagtcgtgc ctgtttgctt agcat 25

<210> 224
<211> 25
<212> DNA
<213> Artificial

<220>
<223> A sequence of a probe contained in a probe set

<400> 224
gtttgcttag cattcctatt gacaa 25

<210> 225
<211> 25
<212> DNA
<213> Artificial

<220>
<223> A sequence of a probe contained in a probe set

<400> 225
cattcctatt gacaactctt ctggg 25

<210> 226
<211> 25
<212> DNA
<213> Artificial

<220>
<223> A sequence of a probe contained in a probe set

<400> 226
gttctgcttt aataagcgag accta 25

<210> 227
<211> 25
<212> DNA
<213> Artificial

<220>
<223> A sequence of a probe contained in a probe set

<400> 227
aaaacacatc tggcctaatg ttcca 25

<210> 228
<211> 25
<212> DNA
<213> Artificial

<220>
<223> A sequence of a probe contained in a probe set

<400> 228

gcctaatgtt ccagatcctt caaag 25

<210> 229
<211> 25
<212> DNA
<213> Artificial

<220>
<223> A sequence of a probe contained in a probe set

<400> 229
agagtcatat tgcccagtgg tcacc 25

<210> 230
<211> 25
<212> DNA
<213> Artificial

<220>
<223> A sequence of a probe contained in a probe set

<400> 230
ttcagatggc aatttcactg atgta 25

<210> 231
<211> 25
<212> DNA
<213> Artificial

<220>
<223> A sequence of a probe contained in a probe set

<400> 231
acaaaaagcc ttttccagaa gatct 25

<210> 232
<211> 25
<212> DNA
<213> Artificial

<220>
<223> A sequence of a probe contained in a probe set

<400> 232
gaagatctga aatcattgga cctgt 25

<210> 233
<211> 25
<212> DNA
<213> Artificial

<220>
<223> A sequence of a probe contained in a probe set

<400> 233
ggcatctcct gagcctaggc aatac 25

<210> 234

<211> 25
<212> DNA
<213> Artificial

<220>
<223> A sequence of a probe contained in a probe set

<400> 234
gtagggtgac ttctggagcc atccc 25

<210> 235
<211> 25
<212> DNA
<213> Artificial

<220>
<223> A sequence of a probe contained in a probe set

<400> 235
agagctgggg aaatgggccc gaga 25

<210> 236
<211> 25
<212> DNA
<213> Artificial

<220>
<223> A sequence of a probe contained in a probe set

<400> 236
tcatgggtgc ctcagagcag gacct 25

<210> 237
<211> 25
<212> DNA
<213> Artificial

<220>
<223> A sequence of a probe contained in a probe set

<400> 237
agcaggacct tggtatttcc ttgtt 25

<210> 238
<211> 25
<212> DNA
<213> Artificial

<220>
<223> A sequence of a probe contained in a probe set

<400> 238
gctttgggct acgtggatga ccagc 25

<210> 239
<211> 25
<212> DNA
<213> Artificial

<220>
<223> A sequence of a probe contained in a probe set

<400> 239
atgaccagct gttcgtgttc tatga 25

<210> 240
<211> 25
<212> DNA
<213> Artificial

<220>
<223> A sequence of a probe contained in a probe set

<400> 240
cccgaactcc atgggtttcc agtag 25

<210> 241
<211> 25
<212> DNA
<213> Artificial

<220>
<223> A sequence of a probe contained in a probe set

<400> 241
gcagctgagt cagagtctgc ccage 25

<210> 242
<211> 25
<212> DNA
<213> Artificial

<220>
<223> A sequence of a probe contained in a probe set

<400> 242
tctgcccagc cgtcaaaaga gtctt 25

<210> 243
<211> 25
<212> DNA
<213> Artificial

<220>
<223> A sequence of a probe contained in a probe set

<400> 243
agggcaggtg cttcaggata ccata 25

<210> 244
<211> 25
<212> DNA
<213> Artificial

<220>
<223> A sequence of a probe contained in a probe set

<400> 244
tccaggctgg aagtacttgg ccccc 25

<210> 245
<211> 25
<212> DNA
<213> Artificial

<220>
<223> A sequence of a probe contained in a probe set

<400> 245
gagagagtag ctgcagcctt catca 25

<210> 246
<211> 25
<212> DNA
<213> Artificial

<220>
<223> A sequence of a probe contained in a probe set

<400> 246
gccagcaagt ccttaggcta ctgta 25

<210> 247
<211> 25
<212> DNA
<213> Artificial

<220>
<223> A sequence of a probe contained in a probe set

<400> 247
gctactgtaa tgctgcctca ggacc 25

<210> 248
<211> 25
<212> DNA
<213> Artificial

<220>
<223> A sequence of a probe contained in a probe set

<400> 248
tcctctaggc cctgtgagca caaag 25

<210> 249
<211> 25
<212> DNA
<213> Artificial

<220>
<223> A sequence of a probe contained in a probe set

<400> 249
taatccattt caggactctc tccag 25

<210> 250
<211> 25
<212> DNA
<213> Artificial

<220>
<223> A sequence of a probe contained in a probe set

<400> 250
gctcggggtg tgtcatttct atatt 25

<210> 251
<211> 25
<212> DNA
<213> Artificial

<220>
<223> A sequence of a probe contained in a probe set

<400> 251
atattcctcc agctgggatt ggggg 25

<210> 252
<211> 25
<212> DNA
<213> Artificial

<220>
<223> A sequence of a probe contained in a probe set

<400> 252
gcaggcccaa tgctgctttt ggggg 25

<210> 253
<211> 25
<212> DNA
<213> Artificial

<220>
<223> A sequence of a probe contained in a probe set

<400> 253
gggggtcagc atccagtgtg agata 25

<210> 254
<211> 25
<212> DNA
<213> Artificial

<220>
<223> A sequence of a probe contained in a probe set

<400> 254
tgcaaataaa acgctttccc cgtca 25

<210> 255
<211> 25
<212> DNA

<213> Artificial

<220>
<223> A sequence of a probe contained in a probe set

<400> 255
gcctgaacct cagacctagt aattt 25

<210> 256
<211> 25
<212> DNA
<213> Artificial

<220>
<223> A sequence of a probe contained in a probe set

<400> 256
cagacctagt aatttttcat gcagt 25

<210> 257
<211> 25
<212> DNA
<213> Artificial

<220>
<223> A sequence of a probe contained in a probe set

<400> 257
caagtttgta acatgcagca gatta 25

<210> 258
<211> 25
<212> DNA
<213> Artificial

<220>
<223> A sequence of a probe contained in a probe set

<400> 258
gaaaacctta atgactcaga gagca 25

<210> 259
<211> 25
<212> DNA
<213> Artificial

<220>
<223> A sequence of a probe contained in a probe set

<400> 259
ggaagctgat taattagata tgcat 25

<210> 260
<211> 25
<212> DNA
<213> Artificial

<220>

<223> A sequence of a probe contained in a probe set

<400> 260
gcatctggca ttgttttatc ttatc 25

<210> 261
<211> 25
<212> DNA
<213> Artificial

<220>
<223> A sequence of a probe contained in a probe set

<400> 261
atcttatcag tattatcact cttat 25

<210> 262
<211> 25
<212> DNA
<213> Artificial

<220>
<223> A sequence of a probe contained in a probe set

<400> 262
atcactctta tgttggttta ttctt 25

<210> 263
<211> 25
<212> DNA
<213> Artificial

<220>
<223> A sequence of a probe contained in a probe set

<400> 263
gttggtttat tcttaagctg tacaa 25

<210> 264
<211> 25
<212> DNA
<213> Artificial

<220>
<223> A sequence of a probe contained in a probe set

<400> 264
aaacatatgc taaatccgct tcagt 25

<210> 265
<211> 25
<212> DNA
<213> Artificial

<220>
<223> A sequence of a probe contained in a probe set

<400> 265

atgctaaatc cgcttcagta tttta 25

<210> 266
<211> 25
<212> DNA
<213> Artificial

<220>
<223> A sequence of a probe contained in a probe set

<400> 266
ttctttcccc aaatatcatg tagca 25

<210> 267
<211> 25
<212> DNA
<213> Artificial

<220>
<223> A sequence of a probe contained in a probe set

<400> 267
gaaacattct tatgcatcat ttggt 25

<210> 268
<211> 25
<212> DNA
<213> Artificial

<220>
<223> A sequence of a probe contained in a probe set

<900> 268
tacgctatgg gatactggca acagt 25

<210> 269
<211> 25
<212> DNA
<213> Artificial

<220>
<223> A sequence of a probe contained in a probe set

<400> 269
gcaacagtgc acatatttca taacc 25

<210> 270
<211> 25
<212> DNA
<213> Artificial

<220>
<223> A sequence of a probe contained in a probe set

<400> 270
ttcataacca aattagcagc accgg 25

<210> 271

<211> 25
<212> DNA
<213> Artificial

<220>
<223> A sequence of a probe contained in a probe set

<400> 271
gcagcaccgg tcttaatttg atgtt 25

<210> 272
<211> 25
<212> DNA
<213> Artificial

<220>
<223> A sequence of a probe contained in a probe set

<400> 272
ggatatgtgt gtttactgta ctttt 25

<210> 273
<211> 25
<212> DNA
<213> Artificial

<220>
<223> A sequence of a probe contained in a probe set

<400> 273
tttgttttga tccgtttgta taaat 25

<210> 274
<211> 25
<212> DNA
<213> Artificial

<220>
<223> A sequence of a probe contained in a probe set

<400> 274
gatagcaata tcttggacac atttg 25

<210> 275
<211> 25
<212> DNA
<213> Artificial

<220>
<223> A sequence of a probe contained in a probe set

<400> 275
atacctagca atcactttta ctttt 25

<210> 276
<211> 25
<212> DNA
<213> Artificial

<220>
<223> A sequence of a probe contained in a probe set

<400> 276
ttttactttt tgtaattctg tctct 25

## Description

TECHNICAL FIELD

The present invention relates to a method and a program for determining sensitivity to breast cancer neoadjuvant chemotherapy More specifically, the present invention relates to a method and a program for determining sensitivity to breast cancer neoadjuvant chemotherapy that is useful for providing information assisting diagnosis of sensitivity to breast cancer neoadjuvant chemotherapy.

BACKGROUND

Recently, an increased number of patients undergo breast cancer neoadjuvant chemotherapy not only for improving the adaptability to a surgery of local advanced breast cancer, but also for improving the adaptability to a breast-conserving surgery for a patient who has relatively large tumor(s). It is also known that a patient who has had a pathological complete response (hereinafter, also referred to as "pCR") by a breast cancer neoadjuvant chemotherapy has a good prognosis.

At present, a sequential chemotherapy using taxane and anthracycline is commonly performed in clinical practice. However, the pCR rate (the number of cases achieving pCR/the total number of cases having undergone a chemotherapy) of the sequential chemotherapy is 10 to 30%, which is not necessarily high. Also, it is reported that the pCR rate is higher in estrogen receptor (hereinafter, referred to as "ER")-negative cases, when ER-positive cases and ER-negative cases are compared. However, in ER-negative cases, pCR is not necessarily achieved in every case by a breast cancer neoadjuvant chemotherapy, whereas in ER-positive cases, pCR is occasionally achieved by a breast cancer neoadjuvant chemotherapy. Therefore, there is a demand for a method capable of accurately determining sensitivity to breast cancer neoadjuvant chemotherapy irrespectively of, e.g., classification based on ER positivity and ER negativity.

As a method for predicting sensitivity to breast cancer neoadjuvant chemotherapy, for example, a method of determining sensitivity to breast cancer neoadjuvant chemotherapy by measuring and analyzing expression levels of a specified gene group using RNA extracted from a specimen collected from a subject is proposed (see, for example, WO 2011/065533 A). However, in the method described in WO 2011/065533 A, a gene group including ER gene is used as a gene group to be measured.

On the other hand, Iwamoto T et al.(Gene pathways associated with prognosis and chemotherapy sensitivity in molecular subtypes of breast cancer, Journal of the National Cancer Institute, 2011, Vol. 103, p. 264-272) discloses that a gene group involved in signal transduction of chemokine receptor-3, a gene group involved in signal transduction of chemokine receptor-5, a gene group involved in signal transduction of interleukin-8 and the like are related to chemotherapy responsibility in ER-positive breast cancer, from the gene expression profile obtained by a comprehensive gene expression analysis using a DNA microarray. However, predictive determination of sensitivity using a concretely specified gene group is not conducted.

Also, Schmidt M et al. (The humoral immune system has a key prognostic impact in node-negative breast cancer, Cancer research, 2008, Vol. 68, p. 5405-5413) discloses that B-cell metagene consisting of a gene group associated with a B cell responsible for humoral immune system is a prognostic factor for lymph node metastasis negative and highly proliferative breast cancer. Schmidt M et al. (A comprehensive analysis of human gene expression profiles identifies stromal immunoglobulin kappa C as a compatible prognostic marker in human solid tumors, Clinical Cancer Research, 2012, Vol. 18, p. 2695-2703) discloses that an expression level of immunoglobulin κC gene is correlated with B-cell metagene, and is useful for predicting sensitivity to chemotherapy using anthracycline in ER-negative breast cancer.

Further, Teschendorff AE et al. (An immune response gene expression module identifies a good prognosis subtype in estrogen receptor negative breast cancer, Genome Biology, 2007, Vol. 8, R157) discloses a gene group consisting of seven genes (respective genes of C1QA, XCL2, SPP1, TNFRSF17, LY9, IGLC2 and HLA-F) related with the immune responsiveness as a prognostic marker in ER-negative breast cancer cases.

WO 2013/014296 A1 discloses a method for predicting a response to chemotherapy, including neoadjuvant chemotherapy, in a patient suffering from or at risk of developing recurrent neoplastic disease, in particular breast cancer. This method is based on the measurement of gene expression levels (in particular of the genes UBE2C, RACGAP1, DHCR7, STC2, AZGP1, RBBP8, IL6ST and MGP) in tumor samples of the cancer patient. Though, this prognostic gene expression signature is limited to particular classifications of breast cancer.

SUMMARY OF INVENTION

However, the present inventors have found out that when predicting sensitivity to breast cancer neoadjuvant chemotherapy is predicted by using genes or gene groups described in WO 2011/065533 A, Schmidt M et al. (Cancer research, 2008, Vol. 68, p. 5405-5413), Schmidt M et al. (Clinical Cancer Research, 2012, Vol. 18, p. 2695-2703) and Teschendorff AE et al. (Genome Biology, 2007, Vol. 8, R157), the reliability of determination regarding the presence or absence of sensitivity to breast cancer neoadjuvant chemotherapy can be impaired in specimens of a subject group classified based on ER positivity and ER negativity, or in specimens of a subject group classified based on the regimen of breast cancer neoadjuvant chemotherapy, as described below.

It is an object of the present invention to provide a method and a program for determining sensitivity to breast cancer neoadjuvant chemotherapy capable of providing information that assists more accurate diagnosis of both the presence and absence of sensitivity to breast cancer neoadjuvant chemotherapy irrespectively of the background of the subject such as ER positivity or negativity, the regimen of chemotherapy and the like.

One aspect includes a method for determining sensitivity of a subject to breast cancer neoadjuvant chemotherapy comprising the steps of :

(1) preparing a measurement sample comprising RNA from a specimen collected from the subject,
(2) measuring an expression level of each gene of (A1) to (A19) below making use of a measurement sample obtained in step (1),
(3) analyzing an expression level of the each gene measured in step (2), and
(4) determining sensitivity to breast cancer neoadjuvant chemotherapy on the basis of an analysis result obtained in step (3),
wherein

(A1) is human caspase recruitment domain family, member 9 (CARD9) gene,
(A2) is human indoleamine-2,3-dioxygenase 1 (IDO1) gene,
(A3) is human chemokine (C-X-C motif) ligand 9 (CXCL9) gene,
(A4) is human purine nucleoside phosphorylase (PNP) gene,
(A5) is human chemokine (C-X-C motif) ligand 11 (CXCL11) gene,
(A6) is human CCAAT/enhancer binding protein (CEBPB) gene,
(A7) is human CD83 gene,
(A8) is human interleukin 6 signal transducer (IL6ST) gene,
(A9) is human chemokine (C-X3-C) receptor 1 (CX3CR1) gene,
(A10) is human CD1D gene,
(A11) is human cathepsin C (CTSC) gene,
(A12) is human chemokine (C-X-C motif) ligand 10 (CXCL10) gene,
(A13) is human immunoglobulin heavy chain genetic locus G1 isotype (IGHG1) gene,
(A14) is human zinc finger E-box-binding homeobox 1 (ZEB1) gene,
(A15) is human vascular endothelial growth factor A (VEGFA) gene,
(A16) is human semaphorin-3C precursor (SEMA3C) gene,
(A17) is human complement receptor (CR2) gene,
(A18) is human HFE gene, and
(A19) is human EDA gene.

Another aspect includes a computer program causing a computer to function as a control section for analyzing an expression level of each nucleic acid corresponding to the probes of (B1) to (B23) below in a measurement sample comprising RNA prepared from a specimen collected from a subject, and determining sensitivity to breast cancer neoadjuvant chemotherapy on the basis of obtained information of the expression level, the control section being configured to execute:

calculation of solution D based on a discriminant represented by formula (I):

$$D = \sum_i (w_i \times y_i) - 3.327217 \qquad (I)$$

(wherein, i represents a number assigned to each nucleic acid shown in Table B so as to correspond to SEQ ID NO of the targeted polynucleotide, $w_i$ represents a weighting factor of nucleic acid of number i shown in Table B, $y_i$ represents a standardized expression level of nucleic acid, the standardized expression level being obtained by standardizing an expression level of nucleic acid according to the formula represented by formula (II):

$$y_i = x_i - m_i \qquad (\mathrm{II})$$

(wherein, $x_i$ represents an expression level of nucleic acid of number i shown in Table B, and $m_i$ represents a mean value of expression level of nucleic acid of number i shown in Table B over the specimens), and $\Sigma_i$ represents a sum total over the respective nucleic acids), and
sensitivity determination that makes a determination of being sensitive to breast cancer neoadjuvant chemotherapy when solution D of the discriminant is a positive value, and makes a determination of being insensitive to breast cancer neoadjuvant chemotherapy when solution D is 0 or a negative value, wherein

(B1) is a probe targeting a polynucleotide having a nucleotide sequence of SEQ ID NO: 1,
(B2) is a probe targeting a polynucleotide having a nucleotide sequence of SEQ ID NO: 2,
(B3) is a probe targeting a polynucleotide having a nucleotide sequence of SEQ ID NO: 3,
(B4) is a probe targeting a polynucleotide having a nucleotide sequence of SEQ ID NO: 4,
(B5) is a probe targeting a polynucleotide having a nucleotide sequence of SEQ ID NO: 5,
(B6) is a probe targeting a polynucleotide having a nucleotide sequence of SEQ ID NO: 6,
(B7) is a probe targeting a polynucleotide having a nucleotide sequence of SEQ ID NO: 7,
(B8) is a probe targeting a polynucleotide having a nucleotide sequence of SEQ ID NO: 8,
(B9) is a probe targeting a polynucleotide having a nucleotide sequence of SEQ ID NO: 9,
(B10) is a probe targeting a polynucleotide having a nucleotide sequence of SEQ ID NO: 10,
(B11) is a probe targeting a polynucleotide having a nucleotide sequence of SEQ ID NO: 11,
(B12) is a probe targeting a polynucleotide having a nucleotide sequence of SEQ ID NO: 12,
(B13) is a probe targeting a polynucleotide having a nucleotide sequence of SEQ ID NO: 13,
(B14) is a probe targeting a polynucleotide having a nucleotide sequence of SEQ ID NO: 14,
(B15) is a probe targeting a polynucleotide having a nucleotide sequence of SEQ ID NO: 15,
(B16) is a probe targeting a polynucleotide having a nucleotide sequence of SEQ ID NO: 16,
(B17) is a probe targeting a polynucleotide having a nucleotide sequence of SEQ ID NO: 17,
(B18) is a probe targeting a polynucleotide having a nucleotide sequence of SEQ ID NO: 18,
(B19) is a probe targeting a polynucleotide having a nucleotide sequence of SEQ ID NO: 19,
(B20) is a probe targeting a polynucleotide having a nucleotide sequence of SEQ ID NO: 20,
(B21) is a probe targeting a polynucleotide having a nucleotide sequence of SEQ ID NO: 21,
(B22) is a probe targeting a polynucleotide having a nucleotide sequence of SEQ ID NO: 22, and
(B23) is a probe targeting a polynucleotide having a nucleotide sequence of SEQ ID NO: 23, wherein Table B is:

TABLE B

| Number i of nucleic acid | SEQ ID NO of targeted polynucleotide | Weighting factor |
|---|---|---|
| 1 | 1 | 2.361579818 |
| 2 | 2 | 0.527535817 |
| 3 | 3 | 0.53572137 |
| 4 | 4 | 1.296736029 |
| 5 | 5 | 0.437766376 |
| 6 | 6 | 1.09614395 |
| 7 | 7 | 1.154132786 |
| 8 | 8 | -0.997955474 |
| 9 | 9 | -0.84645569 |
| 10 | 10 | 0.703499669 |
| 11 | 11 | 1.262066324 |
| 12 | 12 | 0.481709248 |
| 13 | 13 | 0.784677171 |
| 14 | 14 | -1.056130291 |

(continued)

| Number i of nucleic acid | SEQ ID NO of targeted polynucleotide | Weighting factor |
|---|---|---|
| 15 | 15 | -0.90152985 |
| 16 | 16 | 0.941011796 |
| 17 | 17 | -0.580145259 |
| 18 | 18 | 0.797198448 |
| 19 | 19 | -0.963860205 |
| 20 | 20 | -1.352304026 |
| 21 | 21 | -1.231365097 |
| 22 | 22 | -0.637818166 |
| 23 | 23 | 0.449217729 |

ADVANTAGEOUS EFFECTS OF INVENTION

According to the method and the program for determining sensitivity to breast cancer neoadjuvant chemotherapy of the present embodiment, there is exhibited an excellent effect such that information assisting more accurate diagnosis of both the presence and absence of sensitivity to breast cancer neoadjuvant chemotherapy, irrespectively of the background of the subject such as ER positivity or negativity, the regimen of chemotherapy and the like.

BRIEF DESCRIPTION OF DRAWINGS

FIG. 1 is a schematic explanatory view of an apparatus for determining sensitivity to breast cancer neoadjuvant chemotherapy.
FIG. 2 is a block diagram illustrating a functional configuration of the determining apparatus shown in FIG. 1.
FIG. 3 is a block diagram illustrating a hardware configuration of the determining apparatus shown in FIG. 1.
FIG. 4 is a flowchart of determination of sensitivity to breast cancer neoadjuvant chemotherapy using the determining apparatus shown in FIG. 1.
FIG. 5 illustrates the result of examination for the relation between the number of probe sets and accuracy in Example 1.
FIG. 6 illustrates the result of comparison between the determination result by the discriminant represented by formula (I) and the pathological diagnosis result for a training set in Example 1.
FIG. 7 illustrates the result of comparison between the determination result by the discriminant represented by formula (I) and the pathological diagnosis result for a validation set in Example 1.
FIG. 8 illustrates the result of comparison between the determination result by the discriminant represented by formula (I) and the pathological diagnosis result acquired from a database in Example 2.
FIG. 9 illustrates the result of comparison between the determination result by the discriminant represented by formula (I) and the pathological diagnosis result acquired from the database in Example 3.
FIG. 10 illustrates the result of comparison between the determination result by the discriminant represented by formula (I) and the pathological diagnosis result acquired from the database in Example 4.
FIG. 11 illustrates the result of comparison between the determination result obtained by using the expression level of gene corresponding to 70 probe sets shown in Tables 1 and 2 of WO 2011/065533 A, and the pathological diagnosis result acquired from the database for subject groups 1-1 to 1-6 in Comparative Example 1.
FIG. 12 illustrates the result of comparison between the determination result obtained by using the expression level of gene corresponding to 70 probe sets shown in Tables 1 and 2 of WO 2011/065533 A, and the pathological diagnosis result acquired from the database for subject groups 2-1 to 2-3 in Comparative Example 1.
FIG. 13 illustrates the result of comparison between the determination result obtained by using an expression level of gene corresponding to 70 probe sets shown in Tables 1 and 2 of WO 2011/065533 A, and the pathological diagnosis result acquired from the database for subject groups 3-1 to 3-5 in Comparative Example 1.
FIG. 14 illustrates the result of comparison between the determination result obtained according to the method described in Schmidt M et al. (Cancer research, 2008, Vol. 68, p. 5405-5413), and the pathological diagnosis result acquired from the database for subject groups 1-1 to 1-6 in Comparative Example 2.

FIG. 15 illustrates the result of comparison between the determination result obtained according to the method described in Schmidt M et al. (Cancer research, 2008, Vol. 68, p. 5405-5413), and the pathological diagnosis result acquired from the database for subject groups 2-1 to 2-3 in Comparative Example 2.

FIG. 16 illustrates the result of comparison between the determination result obtained according to the method described in Schmidt M et al. (Cancer research, 2008, Vol. 68, p. 5405-5413), and the pathological diagnosis result acquired from the database for subject groups 3-1 to 3-5 in Comparative Example 2.

FIG. 17 illustrates the result of comparison between the determination result obtained according to the method described in Schmidt M et al. (Clinical Cancer Research, 2012, Vol. 18, p. 2695-2703), and the pathological diagnosis result acquired from the database for subject groups 1-1 to 1-6 in Comparative Example 3.

FIG. 18 illustrates the result of comparison between the determination result obtained according to the method described in Schmidt M et al. (Clinical Cancer Research, 2012, Vol. 18, p. 2695-2703), and the pathological diagnosis result acquired from the database for subject groups 2-1 to 2-3 in Comparative Example 3.

FIG. 19 illustrates the result of comparison between the determination result obtained according to the method described in Schmidt M et al. (Clinical Cancer Research, 2012, Vol. 18, p. 2695-2703), and the pathological diagnosis result acquired from the database for subject groups 3-1 to 3-5 in Comparative Example 3.

FIG. 20 illustrates the result of comparison between the determination result obtained according to the method described in Teschendorff AE et al. (Genome Biology, 2007, Vol. 8, R157), and the pathological diagnosis result acquired from the database for subject groups 1-1 to 1-6 in Comparative Example 4.

FIG. 21 illustrates the result of comparison between the determination result obtained according to the method described in Teschendorff AE et al. (Genome Biology, 2007, Vol. 8, R157), and the pathological diagnosis result acquired from the database for subject groups 2-1 to 2-3 in Comparative Example 4.

FIG. 22 illustrates the result of comparison between the determination result obtained according to the method described in Teschendorff AE et al. (Genome Biology, 2007, Vol. 8, R157), and the pathological diagnosis result acquired from the database for subject groups 3-1 to 3-5 in Comparative Example 4.

## DESCRIPTION OF EMBODIMENTS

In one aspect, the method for determining sensitivity to breast cancer neoadjuvant chemotherapy according to the present embodiment is a method for determining sensitivity to breast cancer neoadjuvant chemotherapy comprising the steps of:

(1) preparing a measurement sample comprising RNAfrom a specimen collected from a subject,
(2) measuring an expression level of each gene of (A1) to (A19) below making use of a measurement sample obtained in step (1),
(3) analyzing an expression level of the each gene measured in the step (2), and
(4) determining sensitivity to breast cancer neoadjuvant chemotherapy on the basis of an analysis result obtained in step (3), wherein

(A1) is human caspase recruitment domain family, member 9 (CARD9) gene,
(A2) is human indoleamine-2,3-dioxygenase 1 (IDO1) gene,
(A3) is human chemokine (C-X-C motif) ligand 9 (CXCL9) gene,
(A4) is human purine nucleoside phosphorylase (PNP) gene,
(A5) is human chemokine (C-X-C (motif) ligand 11 (CXCL11) gene,
(A6) is human CCAAT/enhancer binding protein (CEBPB) gene,
(A7) is human CD83 gene,
(A8) is human interleukin 6 signal transducer (IL6ST) gene,
(A9) is human chemokine (C-X3-C) receptor 1 (CX3CR1) gene,
(A10) is human CD1D gene,
(A11) is human cathepsin C (CTSC) gene,
(A12) is human chemokine (C-X-C motif) ligand 10 (CXCL10) gene,
(A13) is human immunoglobulin heavy chain genetic locus G1 isotype (IGHG1) gene,
(A14) is human zinc finger E-box-binding homeobox 1 (ZEB1) gene,
(A15) is human vascular endothelial growth factor A (VEGFA) gene,
(A16) is human semaphorin-3C precursor (SEMA3C) gene,
(A17) is human complement receptor (CR2) gene,
(A18) is human HFE gene, and
(A19) is human EDA gene.

In a particular embodiment, the method for determining sensitivity to breast cancer neoadjuvant chemotherapy according to the present embodiment is a method including the steps of:

(I-1) preparing a measurement sample including RNA from a specimen collected from a subject;
(I-2) measuring an expression level of each gene of (A1) to (A19) making use of the measurement sample obtained in step (I-1);
(I-3) analyzing the expression level of the each gene measured in the step (I-2), and
(I-4) determining sensitivity to breast cancer neoadjuvant chemotherapy on the basis of the analysis result obtained in step (I-3) (hereinafter, also referred to as "Method 1"). The step (I-2) corresponds to the step (1). The step (I-3) corresponds to the step (2). The step (I-4) corresponds to the step (3).

In another particular embodiment, the method for determining sensitivity to breast cancer neoadjuvant chemotherapy according to the present embodiment is a method including the steps of:

(II-1) preparing a measurement sample including RNA from a specimen collected from a subject;
(II-2) measuring an expression level of each nucleic acid detected by probes of (B1) to (B23) making use of the measurement sample obtained in step (II-1);
(II-3) analyzing the expression level of the each nucleic acid measured in step (II-2), and
(II-4) determining sensitivity to breast cancer neoadjuvant chemotherapy on the basis of the analysis result obtained in step (II-3), wherein

(B1) is a probe for targeting a polynucleotide having a nucleotide sequence of SEQ ID NO: 1,
(B2) is a probe for targeting a polynucleotide having a nucleotide sequence of SEQ ID NO: 2,
(B3) is a probe for targeting a polynucleotide having a nucleotide sequence of SEQ ID NO: 3,
(B4) is a probe for targeting a polynucleotide having a nucleotide sequence of SEQ ID NO: 4,
(B5) is a probe for targeting a polynucleotide having a nucleotide sequence of SEQ ID NO: 5,
(B6) is a probe for targeting a polynucleotide having a nucleotide sequence of SEQ ID NO: 6,
(B7) is a probe for targeting a polynucleotide having a nucleotide sequence of SEQ ID NO: 7,
(B8) is a probe for targeting a polynucleotide having a nucleotide sequence of SEQ ID NO: 8,
(B9) is a probe for targeting a polynucleotide having a nucleotide sequence of SEQ ID NO: 9,
(B10) is a probe for targeting a polynucleotide having a nucleotide sequence of SEQ ID NO: 10,
(B11) is a probe for targeting a polynucleotide having a nucleotide sequence of SEQ ID NO: 11,
(B12) is a probe for targeting a polynucleotide having a nucleotide sequence of SEQ ID NO: 12,
(B13) is a probe for targeting a polynucleotide having a nucleotide sequence of SEQ ID NO: 13,
(B14) is a probe for targeting a polynucleotide having a nucleotide sequence of SEQ ID NO: 14,
(B15) is a probe for targeting a polynucleotide having a nucleotide sequence of SEQ ID NO: 15,
(B16) is a probe for targeting a polynucleotide having a nucleotide sequence of SEQ ID NO: 16,
(B17) is a probe for targeting a polynucleotide having a nucleotide sequence of SEQ ID NO: 17,
(B18) is a probe for targeting a polynucleotide having a nucleotide sequence of SEQ ID NO: 18,
(B19) is a probe for targeting a polynucleotide having a nucleotide sequence of SEQ ID NO: 19,
(B20) is a probe for targeting a polynucleotide having a nucleotide sequence of SEQ ID NO: 20,
(B21) is a probe for targeting a polynucleotide having a nucleotide sequence of SEQ ID NO: 21,
(B22) is a probe for targeting a polynucleotide having a nucleotide sequence of SEQ ID NO: 22, and
(B23) is a probe for targeting a polynucleotide having a nucleotide sequence of SEQ ID NO: 23 (hereinafter, also referred to as "Method 2"). The step (II-2) corresponds to the step (1). The step (II-3) corresponds to the step (2). The step (II-4) corresponds to the step (3).

Hereinafter, "specified nucleic acid" encompasses each nucleic acid detected by the genes of (A1) to (A19) and the probes of (B1) to (B23). Hereinafter, "an expression level of a specified nucleic acid" encompasses an expression level of each of the gene of (A1) to (A19) and an expression level of each nucleic acids detected by the probes of (B1) to (B23).

According to the method according to the present embodiment, both the presence or absence of sensitivity to breast cancer neoadjuvant chemotherapy in ER-positive cases, and the presence or absence of sensitivity to breast cancer neoadjuvant chemotherapy in ER-negative cases can be accurately determined, since in the method, the operation of measuring and analyzing an expression level of a specified nucleic acid is employed. Therefore, according to the method of the present embodiment, it is possible to assist diagnosis of sensitivity to breast cancer neoadjuvant chemotherapy by providing a person who makes diagnosis (e.g., physician with the obtained determination result as diagnosis assisting information.

The present inventors found that it is possible to accurately determine the presence or absence of sensitivity to breast cancer neoadjuvant chemotherapy irrespectively of the classification based on ER positivity and ER negativity, the classification based on the regimen of the breast cancer neoadjuvant chemotherapy or the like, when sensitivity to breast cancer neoadjuvant chemotherapy is evaluated on the basis of a result that is obtained by using a probe set shown in Table 1, each probe for targeting a respective one of SEQ ID NOs: 1 to 23, and measuring an expression level of each nucleic acid detected by the probe set, and comprehensively analyzing the expression levels. The present invention was accomplished on the basis of this finding.

The "probe for targeting a polynucleotide" used herein refers to a probe designed for the purpose of detecting the polynucleotide. Typically, "probe for targeting a polynucleotide" has a partial sequence of the polynucleotide, or a sequence of the partial sequence in which one or several nucleotides are different from those in the nucleotide sequence of the polynucleotide. The "stringent condition" used herein refers to the condition that is commonly used by a person skilled in the art in conducting hybridization of polynucleotide. The "stringent condition" is not particularly limited as far as it allows hybridization between a probe and a nucleic acid which is to be detected. It is known that the stringency of the condition in conducting hybridization is a function of the temperature, the salt concentration of the hybridization buffer, the chain length of the probe, the GC content of the nucleotide sequence of the probe, and the concentration of the chaotropic agent in the hybridization buffer, and can be appropriately set by a person skilled in the art in consideration of these conditions. As the stringent condition, for example, the condition described in Molecular Cloning: A Laboratory Manual (2nd ed.) (Sambrook, J. et al., 1998, published by Cold Spring Harbor Laboratory Press) can be used.

The "neoadjuvant chemotherapy" used herein refers to an anticancer drug treatment performed on a patient suffering from breast cancer for the purpose of reducing the size of tumor tissues and the like prior to a surgery. The agent used for neoadjuvant chemotherapy is not particularly limited as far as it has an anticancer action. The agent includes, for example, paclitaxel, docetaxel, epirubicin, cyclophosphamide, 5-fluorouracil, adriamycin, ixabepilone, anthracycline and the like. In neoadjuvant chemotherapy, one or a combination of two or more of these agents is administered to a patient according to a prescribed medication schedule.

In the method according to the present embodiment, first, a measurement sample including RNA is prepared from a specimen collected from a subject (step (I-1) of Method 1 and step (II-2) of Method 2).

The specimen is preferably a specimen including breast cancer cells collected from a subject by a preoperative biopsy. Concrete examples of the specimen include a tissue collected from a subject by a preoperative biopsy and the like. Examples of biopsy include fine-needle aspiration biopsy, core-needle biopsy, and a biopsy using a vacuum-assisted core-biopsy instrument (for example, product of Johnson & Johnson K.K., trade name: Mammotome (registered trade name)) (called "Mammotome biopsy"). Among them, Mammotome biopsy is preferred, since a specimen can be obtained readily with low burden. RNA from a specimen can be extracted by a known method. Extraction of RNA from a specimen can be conducted by using a commercially available kit for extraction of RNA. Examples of the commercially available kit include kit of which trade name is Trizol (registered trade name) manufactured by Invitrogen, kit of which trade name is Qiagen RNeasy kit (registered trade name) manufactured by Qiagen, and the like.

Next, a measurement sample suited for measurement of an expression level of a gene, namely a production amount of a transcript (mRNA) corresponding to the gene or the like is prepared. For example, when an expression level of a specified nucleic acid is quantified by RT-PCR, mRNA that is purified from RNA extracted as described above, or RNA itself extracted as described above can be used as a measurement sample. The mRNA can be purified by a known purification method. For purification of mRNA, a commercially available purification kit can be used. On the other hand, when an expression level of a specified nucleic acid is quantified by a microarray, a measurement sample can be acquired by preparing corresponding cDNA or cRNA with a use of the extracted RNA.

The "cDNA" used herein includes not only DNA generated by reverse transcription from mRNA, but also a complementary strand of the DNA, and double-stranded DNA of cDNA and a complementary strand of the cDNA. Amplification of cDNA can be conducted by a known method. For amplification of cDNA, a commercially available kit for amplifying cDNA and a nucleic acid amplification device can be used. Here, examples of the commercially available kit include kit of which trade name is WT-Ovation™ FFPE System V2 manufactured by NuGEN Technologies.

cRNA can be synthesized from cDNA that is reversed transcribed from mRNA, by *in vitro* transcription reaction (IVT) using DNA-dependent RNA polymerase. DNA-dependent RNA polymerase includes, for example, T7 RNA polymerase and the like, but the present invention is not limited to such exemplification. Prior to application to a microarray, synthesized cRNA can be purified as is needed. For purification of cRNA, a method known in the art such as ethanol precipitation, or a commercially available nucleic acid purification kit can be used. Further, for facilitating hybridization between cRNA and a probe on a microarray, cRNA can be fragmented. Fragmentation of cRNA can be conducted by a method known in the art. Such a method includes, for example, a method of heating in the presence of mental ion, a method of using enzyme such as ribonuclease, and the like, but the present invention is not limited to this exemplification.

In the method described below for detecting nucleic acid by a microarray, when formation of a hybrid between cDNA or cRNA in the measurement sample and a probe is measured by detecting fluorescence, color, radiation or the like, it is preferred to label the cDNA or cRNA with a labelling substance such as a substance that generates a detectable signal, or a substance capable of binding with a substance generating a detectable signal. The labelling substance can be any substances that are commonly used in the art, and includes, for example, fluorescent substances such as Cy3, Cy5, Alexa Fluor (registered trade name), and fluorescein isothiocyanate (FITC); haptens such as biotin; radioactive substances, and the like, but the present invention is not limited to this exemplification. The method for labelling cDNA or cRNA with the labelling substance is known in the art. For example, by mixing biotinylated ribonucleotide or biotinylated deoxyribonucleotide as a substrate in the reaction liquid in the step of synthesizing cDNA or cRNA, it is possible to synthesize cDNA labelled with biotin or cRNA labelled with biotin.

Next, an expression level of a specified nucleic acid is measured by using the resulting measurement sample (step (I-2) of Method 1 and step (II-2) of Method 2).

In step (I-2) of Method 1 and step (II-2) of Method 2, an expression level of a specified nucleic acid can be measured, for example, by a microarray, quantitative RT-PCR, quantitative PCR, Northern blot analysis or the like. Among them, it is preferred to measure by using a microarray, since it enables rapid and simple measurement of an expression level of a specified nucleic acid. Here, the "expression level of a specified nucleic acid" includes the copy number of the specified nucleic acid contained in the measurement sample, concentration of the specified nucleic acid in the measurement sample, or a value indicating the copy number of the specified nucleic acid or concentration of the specified nucleic acid. The value indicating the copy number of the specified nucleic acid or concentration of the specified nucleic acid includes, for example, intensity of fluorescence that is measured after applying the measurement sample on the microarray and then allowing the specified nucleic acid and the probe on the microarray to hybridize them, and the like, but the present invention is not limited to this exemplification.

Measurement of the expression level of the specified nucleic acid by a microarray can be conducted by using a known method. Concretely, by using, for example, Human Genome U133 Plus 2.0 Array (trade name) manufactured by Affymetrix, Inc. which is a microarray capable of analyzing expression of human genome, it is possible to measure the expression level of the specified nucleic acid at once. Concretely, the specified nucleic acid can be detected by bringing the measurement sample into contact with the microarray, and hybridizing cDNA or cRNA in the measurement sample with the probe on the microarray.

The microarray used in the method according to the present embodiment is not particularly limited as far as the probes of (B1) to (B23) described below are arranged on a base material. The microarray is preferably a DNA microarray (DNA chip). As such a microarray, microarrays prepared by a method known in the art, and commercially available microarrays are exemplified.

Contact between the measurement sample and the microarray can be achieved by adding the measurement sample to the microarray. In this case, the measurement sample can be used as a dilution obtained by quantifying the concentration of nucleic acid in the measurement sample, and diluting the measurement sample so that the concentration of nucleic acid is a concentration suited for detection by the microarray. Contact between the measurement sample and the microarray can be performed usually at about 10 to 70°C for 2 to 20 hours depending on the kind of microarray being used. For contact between the measurement sample and the microarray, Hybridization Oven 640 (trade name) manufactured by Affymetrix, Inc. or the like can be used.

Further, after contact with the measurement sample, staining of cDNA or cRNA that is immobilized on the base material of the microarray via the probe, and washing of the microarray can be conducted. For example, when the cDNA or cRNA corresponding to the specified nucleic acid in the measurement sample is labelled with biotin, it is possible to stain the cDNA or cRNA hybridized with the probe of the microarray by binding a fluorescent substance or the like labelled with avidin or streptavidin to the biotin. The fluorescent substance includes, for example, FITC, Alexa Fluor (trademark), green-fluorescent protein (GFP), luciferin, phycoerythrin, and the like, but the present invention is not limited to this exemplification. In the present embodiment, after binding avidin or streptavidin with biotin, an antibody capable of binding with the avidin or streptavidin, which is labelled with a fluorescent substance or the like is brought into contact with the microarray, and thus the nucleic acid hybridized with the probe, which is to be measured, can be stained. Staining and washing of the microarray can be conducted by using a microarray washing and staining apparatus, trade name: Fluidic Station 450, manufactured by Affymetrix, Inc. or the like.

The human caspase recruitment domain family, member 9 (CARD9) gene of (A1) has a sequence corresponding to GenBank accession number: NM_022352.

The human indoleamine-2,3-dioxygenase 1 (IDO1) gene of (A2) has a sequence corresponding to GenBank accession number: M34455.

The human chemokine (C-X-C motif) ligand 9 (CXCL9) gene of (A3) has a sequence corresponding to GenBank accession number: NM_002416.

The human purine nucleoside phosphorylase (PNP) gene of (A4) has a sequence corresponding to GenBank accession number: NM_000270.

The human chemokine (C-X-C motif) ligand 11 (CXCL11) gene of (A5) has a sequence corresponding to GenBank accession number: AF002985 or AF030514.

The human CCAAT/enhancer binding protein (CEBPB) gene of (A6) has a sequence corresponding to GenBank accession number: AL564683.

The human CD83 gene of (A7) has a sequence corresponding to GenBank accession number: NM_004233.

The human interleukin 6 signal transducer (IL6ST) gene of (A8) has a sequence corresponding to GenBank accession number: NM_002184, AB015706 or BE856546.

The human chemokine (C-X3-C) receptor 1 (CX3CR1) gene of (A9) has a sequence corresponding to GenBank accession number: U20350.

The human CD1D gene of (A10) has a sequence corresponding to GenBank accession number: NM_001766.

The human cathepsin C (CTSC) gene of (A11) has a sequence corresponding to GenBank accession number: NM_001814.

The human chemokine (C-X-C motif) ligand 10 (CXCL10) gene of (A12) has a sequence corresponding to GenBank accession number: NM_001565.

The human immunoglobulin heavy chain genetic locus G1 isotype (IGHG1) gene of (A13) has a sequence corresponding to GenBank accession number: AJ275397.

The human zinc finger E-box-binding homeobox 1 (ZEB1) gene of (A14) has a sequence corresponding to GenBank accession number: AI373166 or U12170.

The human vascular endothelial growth factor A (VEGFA) gene of (A15) has a sequence corresponding to GenBank accession number: AF022375.

The human semaphorin-3C precursor (SEMA3C) gene of (A16) has a sequence corresponding to GenBank accession number: AI962897.

The human complement receptor (CR2) gene of (A17) has a sequence corresponding to human complement receptor (CR2) gene (GenBank accession number: NM_001877).

The human HFE gene of (A18) has a sequence corresponding to GenBank accession number: AF144243.

The human EDA gene of (A19) has a sequence corresponding to GenBank accession number: NM_001399.

The gene of (A1) includes a nucleic acid detected by a probe for targeting a polynucleotide having a nucleotide sequence of SEQ ID NO: 1.

The gene of (A2) includes a nucleic acid detected by a probe for targeting a polynucleotide having a nucleotide sequence of SEQ ID NO: 2.

The gene of (A3) includes a nucleic acid detected by a probe for targeting a polynucleotide having a nucleotide sequence of SEQ ID NO: 3.

The gene of (A4) includes a nucleic acid detected by a probe for targeting a polynucleotide having a nucleotide sequence of SEQ ID NO: 4.

The gene of (A5) includes a nucleic acid detected by a probe for targeting a polynucleotide having a nucleotide sequence of SEQ ID NO: 5 or SEQ ID NO: 23.

The gene of (A6) includes a nucleic acid detected by a probe for targeting a polynucleotide having a nucleotide sequence of SEQ ID NO: 6.

The gene of (A7) includes a nucleic acid detected by a probe for targeting a polynucleotide having a nucleotide sequence of SEQ ID NO: 7.

The gene of (A8) includes a nucleic acid detected by a probe for targeting a polynucleotide having a nucleotide sequence of SEQ ID NO: 8, SEQ ID NO: 14 or SEQ ID NO: 19.

The gene of (A9) includes a nucleic acid detected by a probe for targeting a polynucleotide having a nucleotide sequence of SEQ ID NO: 9.

The gene of (A10) includes a nucleic acid detected by a probe for targeting a polynucleotide having a nucleotide sequence of SEQ ID NO: 10.

The gene of (A11) includes a nucleic acid detected by a probe for targeting a polynucleotide having a nucleotide sequence of SEQ ID NO: 11.

The gene of (A12) includes a nucleic acid detected by a probe for targeting a polynucleotide having a nucleotide sequence of SEQ ID NO: 12.

The gene of (A13) includes a nucleic acid detected by a probe for targeting a polynucleotide having a nucleotide sequence of SEQ ID NO: 13.

The gene of (A14) includes a nucleic acid detected by a probe for targeting a polynucleotide having a nucleotide sequence of SEQ ID NO: 15 or SEQ ID NO: 22.

The gene of (A15) includes a nucleic acid detected by a probe for targeting a polynucleotide having a nucleotide sequence of SEQ ID NO: 16.

The gene of (A16) includes a nucleic acid detected by a probe for targeting a polynucleotide having a nucleotide sequence of SEQ ID NO: 17.

The gene of (A17) includes a nucleic acid detected by a probe for targeting a polynucleotide having a nucleotide sequence of SEQ ID NO: 18.

The gene of (A18) includes a nucleic acid detected by a probe for targeting a polynucleotide having a nucleotide sequence of SEQ ID NO: 20.

The gene of (A19) includes a nucleic acid detected by a probe for targeting a polynucleotide having a nucleotide sequence of SEQ ID NO: 21.

The probe of (B1) is a probe that targets a polynucleotide having a nucleotide sequence of SEQ ID NO: 1. The probe has a partial sequence of a nucleotide sequence of SEQ ID NO: 1 or a complementary sequence thereof.

The probe of (B2) is a probe that targets a polynucleotide having a nucleotide sequence of SEQ ID NO: 2. The probe has a partial sequence of a nucleotide sequence of SEQ ID NO: 2 or a complementary sequence thereof.

The probe of (B3) is a probe that targets a polynucleotide having a nucleotide sequence of SEQ ID NO: 3. The probe has a partial sequence of a nucleotide sequence of SEQ ID NO: 3 or a complementary sequence thereof.

The probe of (B4) is a probe that targets a polynucleotide having a nucleotide sequence of SEQ ID NO: 4. The probe has a partial sequence of a nucleotide sequence of SEQ ID NO: 4 or a complementary sequence thereof.

The probe of (B5) is a probe that targets a polynucleotide having a nucleotide sequence of SEQ ID NO: 5. The probe has a partial sequence of a nucleotide sequence of SEQ ID NO: 5 or a complementary sequence thereof.

The probe of (B6) is a probe that targets a polynucleotide having a nucleotide sequence of SEQ ID NO: 6. The probe has a partial sequence of a nucleotide sequence of SEQ ID NO: 6 or a complementary sequence thereof.

The probe of (B7) is a probe that targets a polynucleotide having a nucleotide sequence of SEQ ID NO: 7. The probe has a partial sequence of a nucleotide sequence of SEQ ID NO: 7 or a complementary sequence thereof.

The probe of (B8) is a probe that targets a polynucleotide having a nucleotide sequence of SEQ ID NO: 8. The probe has a partial sequence of a nucleotide sequence of SEQ ID NO: 8 or a complementary sequence thereof.

The probe of (B9) is a probe that targets a polynucleotide having a nucleotide sequence of SEQ ID NO: 9. The probe has a partial sequence of a nucleotide sequence of SEQ ID NO: 9 or a complementary sequence thereof.

The probe of (B10) is a probe that targets a polynucleotide having a nucleotide sequence of SEQ ID NO: 10. The probe has a partial sequence of a nucleotide sequence of SEQ ID NO: 10 or a complementary sequence thereof.

The probe of (B11) is a probe that targets a polynucleotide having a nucleotide sequence of SEQ ID NO: 11. The probe has a partial sequence of a nucleotide sequence of SEQ ID NO: 11 or a complementary sequence thereof.

The probe of (B12) is a probe that targets a polynucleotide having a nucleotide sequence of SEQ ID NO: 12. The probe has a partial sequence of a nucleotide sequence of SEQ ID NO: 12 or a complementary sequence thereof.

The probe of (B13) is a probe that targets a polynucleotide having a nucleotide sequence of SEQ ID NO: 13. The probe has a partial sequence of a nucleotide sequence of SEQ ID NO: 13 or a complementary sequence thereof.

The probe of (B14) is a probe that targets a polynucleotide having a nucleotide sequence of SEQ ID NO: 14. The probe has a partial sequence of a nucleotide sequence of SEQ ID NO: 14 or a complementary sequence thereof.

The probe of (B15) is a probe that targets a polynucleotide having a nucleotide sequence of SEQ ID NO: 15. The probe has a partial sequence of a nucleotide sequence of SEQ ID NO: 15 or a complementary sequence thereof.

The probe of (B16) is a probe that targets a polynucleotide having a nucleotide sequence of SEQ ID NO: 16. The probe has a partial sequence of a nucleotide sequence of SEQ ID NO: 16 or a complementary sequence thereof.

The probe of (B17) is a probe that targets a polynucleotide having a nucleotide sequence of SEQ ID NO: 17. The probe has a partial sequence of a nucleotide sequence of SEQ ID NO: 17 or a complementary sequence thereof.

The probe of (B18) is a probe that targets a polynucleotide having a nucleotide sequence of SEQ ID NO: 18. The probe has a partial sequence of a nucleotide sequence of SEQ ID NO: 18 or a complementary sequence thereof.

The probe of (B19) is a probe that targets a polynucleotide having a nucleotide sequence of SEQ ID NO: 19. The probe has a partial sequence of a nucleotide sequence of SEQ ID NO: 19 or a complementary sequence thereof.

The probe of (B20) is a probe that targets a polynucleotide having a nucleotide sequence of SEQ ID NO: 20. The probe has a partial sequence of a nucleotide sequence of SEQ ID NO: 20 or a complementary sequence thereof.

The probe of (B21) is a probe that targets a polynucleotide having a nucleotide sequence of SEQ ID NO: 21. The probe has a partial sequence of a nucleotide sequence of SEQ ID NO: 21 or a complementary sequence thereof.

The probe of (B22) is a probe that targets a polynucleotide having a nucleotide sequence of SEQ ID NO: 22. The probe has a partial sequence of a nucleotide sequence of SEQ ID NO: 22 or a complementary sequence thereof.

The probe of (B23) is a probe that targets a polynucleotide having a nucleotide sequence of SEQ ID NO: 23. The probe has a partial sequence of a nucleotide sequence of SEQ ID NO: 23 or a complementary sequence thereof.

The length of the probes of (B1) to (B23) has typically 10 to 60 nucleotide length, and has 15 to 50 nucleotide length, from the view point of determining the presence or absence of insensitivity to breast cancer neoadjuvant chemotherapy more accurately.

In the present invention, it is preferred to measure an expression level of the specified nucleic acid using a probe set shown in Table 1 from the view point of determining the presence or absence of sensitivity to breast cancer neoadjuvant chemotherapy accurately.

TABLE 1

| Number of nucleic acid to be measured | Probe set ID | Nucleotide sequence of polynucleotide targeted by probe | Nucleotide sequence of probe |
|---|---|---|---|
| 1 | 220162_s_at | SEQ ID NO:1 | SEQ ID NO:24~34 |
| 2 | 210029_at | SEQ ID NO:2 | SEQ ID NO:35~45 |
| 3 | 203915_at | SEQ ID NO:3 | SEQ ID NO:46~56 |
| 4 | 201695_s_at | SEQ ID NO:4 | SEQ ID NO:57~67 |
| 5 | 211122_s_at | SEQ ID NO:5 | SEQ ID NO:68~78 |
| 6 | 212501_at | SEQ ID NO:6 | SEQ ID NO:79~89 |
| 7 | 204440_at | SEQ ID NO:7 | SEQ ID NO:90~100 |
| 8 | 204864_s_at | SEQ ID NO:8 | SEQ ID NO:101~111 |
| 9 | 205898_at | SEQ ID NO:9 | SEQ ID NO:112~122 |
| 10 | 205789_at | SEQ ID NO:10 | SEQ ID NO:123~133 |
| 11 | 201487_at | SEQ ID NO:11 | SEQ ID NO:134~144 |
| 12 | 204533_at | SEQ ID NO:12 | SEQ ID NO:145~155 |
| 13 | 216541_x_at | SEQ ID NO:13 | SEQ ID NO:156~166 |
| 14 | 211000_s_at | SEQ ID NO:14 | SEQ ID NO:167~177 |
| 15 | 212758_s_at | SEQ ID NO:15 | SEQ ID NO:178~188 |
| 16 | 210512_s_at | SEQ ID NO:16 | SEQ ID NO:189~199 |
| 17 | 203788_s_at | SEQ ID NO:17 | SEQ ID NO:200~210 |
| 18 | 205544_s_at | SEQ ID NO:18 | SEQ ID NO:211~221, |
| 19 | 204863_s_at | SEQ ID NO:19 | SEQ ID NO:222~232 |
| 20 | 211331_x_at | SEQ ID NO:20 | SEQ ID NO:233~243 |
| 21 | 206217_at | SEQ ID NO:21 | SEQ ID NO:244~254 |
| 22 | 210875_s_at | SEQ ID NO:22 | SEQ ID NO:255~265 |
| 23 | 210163_at | SEQ ID NO:23 | SEQ ID NO:266~276 |

In Table 1, "Probe set ID" represents an ID number assigned to each probe set composed of 11 to 20 probes immobilized on the base material in a human genome expression analyzing array (trade name: Human Genome U133 Plus 2.0 Array) manufactured by Affymetrix, Inc.. Each probe set includes probes having nucleotide sequences of the SEQ ID NOs shown in Table 1.

Next, an expression level of the specified nucleic acid is analyzed (step (I-3) of Method 1 and step (II-3) of Method 2). Then, based on the obtained analysis result, sensitivity to breast cancer neoadjuvant chemotherapy is determined (step (I-3) of Method 1 and step (II-3) of Method 2).

In step (I-3) of Method 1 and step (II-3) of Method 2, an expression level of the specified nucleic acid can be analyzed, for example, by a classification technique, a scoring technique, a cluster analyzing technique and the like.

As the classification technique, a known method can be used. Examples of such a classification technique include Diagonal Linear Discriminant Analysis (DLDA), Between-group analysis (BGA), Support Vector Mashine (SVM), k nearest neighbor classification (kNN), decision tree, Random Forest, neural network and the like. Among them, from the view point of the ability to determine the presence or absence of sensitivity to breast cancer neoadjuvant chemotherapy with a simple operation, DLDA is preferred. When an expression level is analyzed by such a classification technique, specimens are classified into specimens that are sensitive to breast cancer neoadjuvant chemotherapy, and specimens that are insensitive on the basis of the expression level. Therefore, in this case, in step (1-3) of Method 1 and step (II-3) of Method 2, sensitivity to breast cancer neoadjuvant chemotherapy can be determined according to the result of the classification.

When analysis of an expression level of the specified nucleic acid is conducted by DLDA which is a classification technique in step (I-3) of Method 1 and step (II-3) of Method 2, a discriminant constructed by using DLDA can be used.

As the discriminant, a discriminant represented by formula (I):

$$D = \sum_i (w_i \times y_i) - 3.327217 \qquad (I)$$

(wherein, i represents a number assigned to each nucleic acid shown in Table 2, $w_i$ represents a weighting factor of nucleic acid of number i shown in Table 2, $y_i$ represents a standardized expression level of nucleic acid, the standardized expression level being obtained by standardizing an expression level of nucleic acid according to the formula represented by formula (II):

$$y_i = x_i - m_i \qquad (II)$$

(wherein, $x_i$ represents an expression level of nucleic acid of number i shown in Table 2, arid $m_i$ represents a mean value of expression level of nucleic acid of number i shown in Table 2 over the specimens), and $\sum_i$ represents the sum total over the respective nucleic acids) can be recited.

TABLE 2

| Number of nucleic acid to be measured | Weighting factor |
|---|---|
| 1 | 2.36157982 |
| 2 | 0.52753582 |
| 3 | 0.53572137 |
| 4 | 1.29673603 |
| 5 | 0.43776638 |
| 6 | 1.09614395 |
| 7 | 1.15413279 |
| 8 | -0.9979555 |
| 9 | -0.8464557 |
| 10 | 0.0349967 |
| 11 | 1.26206632 |
| 12 | 0.48170925 |
| 13 | 0.78467717 |
| 14 | -1.0561303 |
| 15 | -0.9015298 |
| 16 | 0.9410118 |
| 17 | -0.5801453 |
| 18 | 0.79719845 |
| 19 | -0.9638602 |
| 20 | -1.352304 |
| 21 | -1.2313651 |
| 22 | -0.6378182 |
| 23 | 0.44921773 |

In analyzing an expression level of each nucleic acid with a use of the discriminant represented by formula (I), a value of expression level of the nucleic acid in a specimen is sequentially substituted for $x_i$ (i=1, 2, ..., 23) in the discriminant represented by formula (I), to thereby find solution D. In this case, in step (D), a determination of being sensitive to breast cancer neoadjuvant chemotherapy can be made when solution D is a positive value, and a determination of being insensitive to breast cancer neoadjuvant chemotherapy can be made when solution D is zero or a negative value.

As described above, according to the method of the present embodiment, since the operation of measuring and analyzing an expression level of the specified nucleic acid is employed, it is possible to determine both the presence or absence of sensitivity to breast cancer neoadjuvant chemotherapy in ER-positive cases, and the presence or absence of sensitivity to breast cancer neoadjuvant chemotherapy in ER-negative cases accurately. Therefore, the method according to the present embodiment is able to determine the presence or absence of sensitivity to breast cancer neoadjuvant chemotherapy irrespectively of the classification of the subject, and thus is suited for providing information for assisting decision of whether execution of breast cancer neoadjuvant chemotherapy is adaptable, and more appropriate information for optimization of the therapy.

Determination of sensitivity to breast cancer neoadjuvant chemotherapy as described above can be performed, for example, by a determining apparatus 1 shown in FIG. 1. Hereinafter, a determining apparatus that can be used for determining sensitivity to breast cancer neoadjuvant chemotherapy will be described in more detail with reference to the attached drawings, however, it is to be noted that the methods of the present invention are not limited to such an embodiment. FIG. 1 is a schematic diagram of an exemplary determining apparatus for sensitivity to breast cancer neoadjuvant chemotherapy. The determining apparatus 1 illustrated in FIG. 1 includes a measurement device 2, and a computer system 3 connected with the measurement device 2.

The measurement device 2 may be a microarray scanner that detects a signal based on the specified nucleic acid bound to a probe on a microarray. The signal may be optical information. The optical information includes, for example, a fluorescent signal, and the like. In this case, as the microarray after contact with the measurement sample is set in the measurement device 2, the measurement device 2 acquires optical information based on the specified nucleic acid bound to the probe on the microarray, and transmits the resulting optical information to the computer system 3.

The microarray scanner is only required to be able to detect a signal based on the specified nucleic acid. Since the signal based on the specified nucleic acid differs depending on the labelling substance used for labelling cDNA or cRNA in the measurement sample, as the microarray scanner, the one appropriate for detecting a signal arising from a certain labelling substance can be appropriately selected depending on the kind of the labelling substance. For example, when the labelling substance is a radioactive substance, a microarray scanner capable of detecting the radiation arising from the radioactive substance can be used as the measurement device 2.

When an expression level of gene is detected by a nucleic acid amplification method, the measurement device 2 can be a nucleic acid amplification detection device. In this case, a reaction liquid including a measurement sample, enzyme for nucleic acid amplification, primers and the like is set in the measurement device 2. Thereafter, nucleic acid in the reaction liquid is amplified by the nucleic acid amplification method. The measurement device 2 acquires optical information such as fluorescence arising from the reaction liquid by amplification reaction, and turbidity of the reaction liquid, and transmits the optical information to the computer system 3.

The computer system 3 includes a computer main unit 3a, an input device 3b, and a display section 3c for displaying specimen information, determination result and the like. The computer system 3 receives optical information from the measurement device 2. Then a processor of the computer system 3 executes a program for determining sensitivity to breast cancer neoadjuvant chemotherapy on the basis of the optical information.

FIG. 2 is a block diagram showing a functional configuration of the determining apparatus shown in FIG. 1.

As shown in FIG. 2, the computer system 3 includes an acquiring section 301, a storage section 302, a calculation section 303, a determination section 304, and an output section 305. The acquiring section 301 is communicatably connected with the measurement device 2 via a network. The calculation section 303 and the determination section 304 form a control section 306.

The acquiring section 301 acquires information transmitted from the measurement device 2. The storage section 302 stores the discriminant represented by formula (I) and a determination criterion. The calculation section 303 calculates solution D of the discriminant according to the discriminant stored in the storage section 302 using the information acquired in the acquiring section 301. The determination section 304 determines the presence or absence of sensitivity to breast cancer neoadjuvant chemotherapy on the basis of solution D calculated by the calculation section 303 and the determination criterion stored in the storage section 302. The output section 305 outputs a determination result by the determination section 304.

FIG. 3 is a block diagram illustrating a hardware configuration of the determining apparatus shown in FIG. 1.

As shown in FIG. 3, the computer main unit 3a includes a CPU (Central Processing Unit) 30, a ROM (Read Only Memory) 121, a RAM (Random Access Memory) 32, a hard disc 33, an I/O interface 34, a reading device 35, a communication interface 36, and an image output interface 37. The CPU 30, the ROM 31, the RAM 32, the hard disc 33, the I/O interface 34, the reading device 35, the communication interface 36 and the image output interface 37 are connected by a bus 38 in data communicatable manner.

The CPU 30 is able to execute a computer program stored in the ROM 31 and a computer program loaded to the RAM 32. The CPU 30 executes an application program to realize each functional block as described above. As a result; the computer system functions as a terminal of the device for determining the presence or absence of sensitivity to breast cancer neoadjuvant chemotherapy.

The ROM 31 is formed of a mask ROM, a PROM, an EPROM, an EEPROM or the like. The ROM 31 stores a computer program to be executed by the CPU 30 and data used therefor.

The RAM 32 is formed of a SRAM, a DRAM or the like. The RAM 32 is used for reading out a computer program stored in the ROM 31 and the hard disc 33. The RAM 32 is also used as a working space for the CPU 30 in executing these computer programs.

In the hard disc 33, an operation system to be executed by the CPU 30, computer programs such as an application program (computer program for determining the presence or absence of sensitivity to breast cancer neoadjuvant chemotherapy), and data used for execution of the computer programs are installed.

The reading device 35 is formed of a flexible disc drive, a CD-ROM drive, a DVD-ROM drive or the like. The reading device 35 is able to read out a computer program or data stored in a portable recording medium 40.

The I/O interface 34 is made up of, for example, serial interfaces such as USB, IEEE1394, and RS-232C, parallel interfaces such as SCSI, IDE, and IEEE1284, and analogue interfaces formed of a D/A converter, an A/D converter or the like. To the I/O interface 34, the input device 3b such as a keyboard or mouse is connected. An operator is able to input data to the computer main unit 3a by using the input device 3b.

The communication interface 36 is, for example, an Ethernet (registered trade name) interface. The communication interface 36 enables the computer system 3 to transmit printing data to a printer.

The image output interface 37 is connected to the display section 3c formed of a LCD, a CRT or the like. As a result, the display section 3c is able to output a video signal corresponding to image data given from the CPU 30. The display section 3c displays an image (screen) according to the input video signal.

Next, a processing procedure of determining the presence or absence of sensitivity to breast cancer neoadjuvant chemotherapy by the determining apparatus 1 will be described. FIG. 4 is a flowchart of determining sensitivity to breast cancer neoadjuvant chemotherapy using the determining apparatus shown in FIG. 1. Here, description will be made while taking the case of conducting determination using fluorescence information based on a nucleic acid to be measured bound to a probe on a microarray that is brought into contact with a measurement sample as an example, but the present invention is not limited only to this embodiment.

First, in step S1-1, the acquiring section 301 of the determining apparatus 1 acquires fluorescence information from the measurement device 2. Then, in step S1-2, the calculation section 303 calculates fluorescence intensity from the fluorescence information acquired by the acquiring section 301, and transmits it to the storage section 302.

Next, in step S1-3, the calculation section 303 calculates solution D of formula (I) on the basis of the fluorescence intensity stored in the storage section 302 according to the discriminant (I) represented by the formula (I) stored in the storage section 302.

Thereafter, in step S1-4, the determination section 304 determines the presence or absence of sensitivity to breast cancer neoadjuvant chemotherapy (namely, sensitivity and insensitivity) by using the value of solution D calculated in the calculation section 303 and the determination criterion stored in the storage section 302.

Here, when solution D is a positive number, the process advances to step S1-4, and the determination section 304 transmits a determination result indicative of sensitivity to breast cancer neoadjuvant chemotherapy to the output section 305. On the other hand, when solution D is 0 or a negative number, it transmits a determination result indicative of insensitivity to breast cancer neoadjuvant chemotherapy to the output section 305.

Then, in step S1-7, the output section 305 outputs a determination result to make the display section 3c display the result or to make a printer print out the result. As a result, it is possible to provide information that assists a physician in deciding whether the subject is sensitive or insensitive to breast cancer neoadjuvant chemotherapy.

These steps can be conducted by using a computer program causing a computer to function as a control section for analyzing an expression level of each nucleic acid corresponding to the probes of (B1) to (B23) described the above in a measurement sample comprising RNA prepared from a specimen collected from a subject, and determining sensitivity to breast cancer neoadjuvant chemotherapy on the basis of obtained information of the expression level, the control section being configured to execute calculation of solution D based on a discriminant represented by the above formula (I), and sensitivity determination that makes a determination of being sensitive to breast cancer neoadjuvant chemotherapy when solution D of the discriminant is a positive value, and makes a determination of being insensitive to breast cancer neoadjuvant chemotherapy when solution D is 0 or a negative value. The computer program is provided as a recording medium storing the computer program or data.

The methodand program for determining sensitivity to breast cancer neoadjuvant chemotherapy envisaged herein involve determining sensitivity to breast cancer neoadjuvant chemotherapy on the basis of an analysis of the expression level of at least each of genes (A1) to (A19) described herein. In particular embodiments, the methodand program for determining sensitivity to breast cancer neoadjuvant chemotherapy envisaged herein involve determining sensitivity to breast cancer neoadjuvant chemotherapy on the basis of an analysis of the expression level of only genes (A1) to (A19) described herein. In further particular embodiments, the method and program for determining sensitivity to breast cancer neoadjuvant chemotherapy envisaged herein involve determining sensitivity to breast cancer neoadjuvant chemotherapy on the basis of an analysis of the expression level of genes using only probes (B1) to (B23) as described herein. In particular embodiments, the method and program for determining sensitivity to breast cancer neoadjuvant chemotherapy envisaged herein involve determining sensitivity to breast cancer neoadjuvant chemotherapy on the basis of an analysis of the expression level of genes using probes of SEQ ID NO: 24 to 272 described herein. In further particular embodiments, the method and program for determining sensitivity to breast cancer neoadjuvant chemotherapy envisaged herein involve determining sensitivity to breast cancer neoadjuvant chemotherapy on the basis of an analysis of the expression level of genes using only the probes corresponding to SEQ ID NO: 24 to 272 described herein.

[Examples]

Hereinafter, the present invention will be described in detail by way of examples that will not limit the present invention. In the following examples and the like, a preprocessing (normalization) of data of a CEL file was conducted by using a RMA statistical algorithm of analysis software (trade name: Affymetrix Expression Console software, manufactured by Affymetrix, Inc.) unless otherwise specified. Any other analyses were conducted by using statistical analysis software R (http://www.r-project.org/) and statistical analysis software Bioconductor (http://www.bioconductor.org/).

(Example 1)

(1) Collection of specimens from subjects

From each of 117 patients suffering from breast cancer who underwent neoadjuvant chemotherapy at Osaka University Hospital, in the period of 2002 to 2010, a specimen including a breast cancer cell was collected by using a vacuum-assisted core-biopsy instrument attached with a collection needle (size 8G) (manufactured by Johnson & Johnson K.K., trade name: Mammotome (registered trade name)). Immediately after collection of a specimen, the specimen was put into liquid nitrogen, and stored at -80°C until use.

(2) Classification of subjects

After collection of the specimens in the above (1), the 117 patients underwent, as breast cancer neoadjuvant chemotherapy, administration of 80 mg/m$^2$ of paclitaxel once a week for 12 weeks, followed by a total of four times of administrations of 75 mg/m$^2$ of epirubicin, 500 mg/m$^2$ of cyclophosphamide and 500 mg/m$^2$ of 5-fluorouracil (5-FU) every three weeks.

Thereafter, pathologic diagnosis and determination of effect of the anticancer agents were conducted by a histopathological examination, and the 117 patients were classified into a pathological complete response group (pCR group) and a non complete response group (npCR group). The "pCR" refers to the state that a tumor completely disappears, or a tumor remains only in a breast duct accompanied by no infiltration site, and no lymph node metastasis. The "npCR" refers to other states than the pCR.

(3) Extraction of RNA from specimen and preparation of cDNA

From a specimen obtained in the above (1) (about 20 mg), RNA was extracted by using a RNA extracting reagent (trade name: TRIzol (registered trade name) manufactured by Invitrogen, or trade name: RNeasy mini kit manufactured by QIAGEN Sciences), thereby giving a RNA sample.

Using the RNA sample (equivalent to 50 ng of RNA), and a random primer attached to a transcript amplification kit (trade name: WT-Ovation FFPE System V2, manufactured by NuGEN Technologies), first-strand cDNA and second-strand cDNA were synthesized, and thereafter cDNA was amplified by the Ribo-SPIA™ amplification technique. In this way, 117 kinds of cDNA corresponding to the specimens of 117 cases were obtained.

(4) Analysis of gene expression

Using a reagent for fragmentation and labelling (trade name: FL-Ovation™ cDNA Biotin Module V2, manufactured by NuGEN Technologies), the cDNA obtained in the above (3) was labelled with biotin and fragmented.

The resulting fragmented biotin-labelled cDNA was allowed to hybridize with nucleic acid (probe set) on an array for analysis of human genome expression (trade name: Human Genome U133 Plus 2.0 Array, manufactured by Affymetrix, Inc.) overnight. Hybridization between the fragmented biotin-labelled cDNA and nucleic acid (probe set) on the array was conducted according to the conditions recommended by the manufacture (Affymetrix, Inc.).

Next, the array after the hybridization was subjected to a machine specialized for a washing and staining treatment of microarray (trade name: GeneChip (registered trade name) Fluidics Station 450, manufactured by Affymetrix, Inc.) to fluorescently stain the cDNA hybridized with nucleic acid (probe set) on the array and wash the same.

Thereafter, the array was subjected to a microarray scanner (trade name: GeneChip (registered trade name) Scanner 3000, manufactured by Affymetrix, Inc.) to read a signal based on a fluorescent labelling substance of the cDNA hybridized with nucleic acid (probe set) on the array to quantify the fluorescence intensity. The resulting data of fluorescence intensity was processed by software (trade name: GeneChip (registered trade name) Operating Software, manufactured by Affymetrix, Inc.), thereby giving a CEL file. The CEL file was used for gene expression analysis. In this way, CEL files were obtained for the data of fluorescence intensity based on the nucleic acids corresponding to the probes of the probe set in each of the specimens of 117 cases. (5) Selection of probe set, and construction of discriminant for determination of sensitivity to breast cancer neoadjuvant chemotherapy

Among the data corresponding to a total of 54675 probe sets mounted on the trade name: Human Genome U133 Plus 2.0 Array manufactured by Affymetrix, Inc., those of 22283 probe sets that are common to the trade name: Human Genome U133A Array manufactured by Affymetrix, Inc. were used in the following analysis.

Further, among the 22283 probe sets, 934 probe sets that are classified as "Immune response" on the gene ontology biological process were selected by referring information of each probe set published from Affymetrix, Inc. (version na32, http://www.Affymetrix.com/).

In data of respective CEL files of the acquired 117 cases, for each of the 934 probe sets, from an expression level of the nucleic acid detected by the probe set, a mean value of expression level of the nucleic acid in 117 cases was subtracted to thereby standardize the expression level (mean-centering).

Next, respective fluorescence intensity data of the specimens of 117 cases was randomly grouped into 58 cases of a training set and 59 cases of a validation set. At this time, the grouping was conducted so that the number of pCR cases in the training set is about twice the number of pCR cases in the validation set so as to increase the detectability of gene corresponding to the probe set whose expression level differs between pCR and npCR cases. Every subsequent analysis other than those described separately was applied to the data of the training set.

The fact that the expression level of the gene corresponding to the probe set differs between pCR and npCR was evaluated by a Welch's t-test, and thereafter a probe set in which p value is less than 0.01 was selected.

Then, discriminants were constructed by increasing the number of selected probe sets one by one in increasing order of p value in the Welch's t-test using a Diagonal Linear Discriminant Analysis (DLDA) as an algorithm of the discriminant.

Then, the number of probe sets with which the accuracy is maximized was determined by using a Leave-One-Out Cross-Validation method. In the Leave-One-Out Cross-Validation method, using a discriminant constructed of data of the training set excluding one case, a result of the excluded one case was predicted, and this operation was repeated 58 times while varying data of one case that is excluded, and the pathologic diagnosis results of 58 cases and the results of 58 cases predicted by discriminants were aggregated. The accuracy was determined by dividing a sum of "the number of specimens exhibiting pCR as a pathologic diagnosis result and predicted as being pCR", and "the number of specimens exhibiting npCR as a pathologic diagnosis result and predicted as being npCR" by the total specimen number. FIG. 5 illustrates the result of examining the relation between the probe set number and the accuracy in Example 1.

The result shown in FIG. 5 reveals that accuracy is maximum when 23 probe sets including the top 23rd probe sets in increasing order of p value in the Welch's t-test (see Table 3) are used. These 23 probe sets respectively target the polynucleotides represented SEQ ID NO: 1 to 23. For these 23 probe sets, a final discriminant was constructed by using all training set data.

TABLE 3

| Number of nucleic acid to be measured | Probe set ID | Nucleotide sequence of polynucleotide targeted by probe | Gene symbol | Nucleotide sequence of probe | Weighting factor | Welch's t-test | p value | High expression |
|---|---|---|---|---|---|---|---|---|
| 1 | 220162_s_at | SEQ ID NO:1 | CARD9 | SEQ ID NO:24~34 | 2.36157982 | 4.323 | 0.0001 | pCR |
| 2 | 210029_at | SEQ ID NO:2 | ID01 | SEQ ID NO:35~45 | 0.52753582 | 3.835 | 0.0004 | pCR |
| 3 | 203915_at | SEQ ID NO:3 | CXCL9 | SEQ ID NO:46~56 | 0.53572137 | 3.760 | 0.0005 | pCR |
| 4 | 201695_s_at | SEQ ID NO:4 | PNP | SEQ ID NO:57~67 | 1.29673603 | 3.804 | 0.0006 | pCR |
| 5 | 211122_s_at | SEQ ID NO:5 | CXCL11 | SEQ ID NO:68~78 | 0.43776638 | 3.686 | 0.0007 | pCR |
| 6 | 212501_at | SEQ ID NO:6 | CEBPB | SEQ ID NO:79~89 | 1.09614395 | 3.631 | 0.0009 | pCR |
| 7 | 204440_at | SEQ ID NO:7 | CD83 | SEQ ID NO:90~100 | 1.15413279 | 3.397 | 0.0013 | pCR |
| 8 | 204864_s_at | SEQ ID NO:8 | IL6ST | SEQ ID NO:101~111 | -0.9979555 | -3.444 | 0.0015 | npCR |
| 9 | 205898_at | SEQ ID NO:9 | CX3CR1 | SEQ ID NO:112~122 | -0.8464557 | -3.327 | 0.0020 | npCR |
| 10 | 205789_at | SEQ ID NO:10 | CD1D | SEQ ID NO: 123~133 | 0.70349967 | 3.263 | 0.0021 | pCR |
| 11 | 201487_at | SEQ ID NO:11 | CTSC | SEQ ID NO: 134~144 | 1.26206632 | 3.314 | 0.0022 | pCR |
| 12 | 204533_at | SEQ ID NO:12 | CXCL10 | SEO ID NO:145~155 | 0.48170925 | 3.165 | 0.0030 | pCR |
| 13 | 216541_x_at | SEQ ID NO:13 | IGHG1 | SEQ ID NO:156~166 | 0.78467717 | 3.158 | 0.0031 | pCR |
| 14 | 211000_s_at | SEQ ID NO:14 | IL6ST | SEQ ID NO:167~177 | -1.0561303 | -3.189 | 0.0031 | npCR |
| 15 | 212758_s_at | SEQ ID NO:15 | ZEB1 | SEQ ID NO:178~188 | -0.9015298 | -3.130 | 0.0034 | npCR |
| 16 | 210512_s_at | SEQ ID NO:16 | VEGFA | SEQ ID NO: 189~199 | 0.9410118 | 3.102 | 0.0036 | pCR |
| 17 | 203788_s_at | SEQ ID NO:17 | SEMA3C | SEQ ID NO:200~210 | -0.5801453 | -3.133 | 0.0040 | npCR |
| 18 | 205544_s_at | SEQ ID NO:18 | CR2 | SEQ ID NO:211~221 | 0.79719845 | 3.149 | 0.0040 | pCR |
| 19 | 204863_s_at | SEQ ID NO:19 | IL6ST | SEQ ID NO:222~232 | -0.9638602 | -3.083 | 0.0043 | npCR |
| 20 | 211331_x_at | SEQ ID NO:20 | HFE | SEQ ID NO:233~243 | -1.352304 | -2.993 | 0.0047 | npCR |
| 21 | 206217_at | SEQ ID NO:21 | EDA | SEQ ID NO:244~254 | -1.2313651 | -3.001 | 0.0049 | npCR |
| 22 | 210875_s_at | SEQ ID NO:22 | ZEB1 | SEQ ID NO:255~265 | -0.6378182 | -2.930 | 0.0054 | npCR |
| 23 | 210163_at | SEQ ID NO:23 | CXCL11 | SEQ ID NO:266~276 | 0.44921773 | 2.919 | 0.0061 | pCR |

The resulting discriminant is a discriminant represented by formula (I):

$$D = \sum_i (w_i \times y_i) - 3.327217 \qquad (I)$$

(wherein, i represents a number assigned to each nucleic acid shown in Table 2, $w_i$ represents a weighting factor of nucleic acid of number i shown in Table 2, $y_i$ represents a standardized expression level of nucleic acid, the standardized expression level being obtained by standardizing an expression level of nucleic acid according to the formula represented by formula (II):

$$y_i = x_i - m_i \qquad (II)$$

(wherein, xi represents an expression level of nucleic acid of number i shown in Table 2, and $m_i$ represents a mean value of expression level of nucleic acid of number i shown in Table 2 over the specimens), and Ei represents the sum total over the respective nucleic acids). When solution D of the discriminant represented by formula (I) is a positive value, it can be determined that the specimen is sensitive to breast cancer neoadjuvant chemotherapy, and when solution D is 0 or a negative value, it can be determined that the specimen is insensitive to breast cancer neoadjuvant chemotherapy.

(6) Comparison between determination result by discriminant and pathologic diagnosis result

Using the data of expression level measured for the specimens of 58 cases grouped in the training set (data of fluorescence intensity), and the discriminant represented by formula (I), to which one of the pCR group and the npCR group of breast cancer patient specimens each specimen of the 58 cases corresponds was determined. The performance of the discriminant was evaluated by comparing the determination result by the discriminant represented by formula (I) and the result of the pathologic diagnosis with a use of the result of the pathologic diagnosis as a true value, FIG. 6 illustrates the result of comparison between the determination result by the discriminant represented by formula (I) and the pathologic diagnosis result for the training set in Example 1. In the figure, "Gp-R" denotes a specimen determined as "a specimen of a subject sensitive to breast cancer neoadjuvant chemotherapy" by the discriminant, and the "Gp-NR" denotes a specimen determined as "a specimen of a subject insensitive to breast cancer neoadjuvant chemotherapy" by the discriminant.

Next, using the data of expression levels measured for the specimens of 59 cases grouped in the validation set (data of fluorescence intensity), and the discriminant, sensitivity to breast cancer neoadjuvant chemotherapy was determined by determining to which one of the pCR group and the npCR group of breast cancer patient specimens each specimen of the 59 cases is allocated was determined. By comparing the pathologic diagnosis result and the determination result by the discriminant using the pathologic diagnosis result as a true value, the performance of the discriminant was evaluated. FIG. 7 illustrates the result of comparison between the determination result by the discriminant represented by formula (I) and the pathologic diagnosis result for the validation set in Example 1. In the figure, "Gp-R" denotes a specimen determined as "a specimen of a subject sensitive to breast cancer neoadjuvant chemotherapy" by the discriminant, and the "Gp-NR" denotes a specimen determined as "a specimen of a subject insensitive to breast cancer neoadjuvant chemotherapy" by the discriminant.

The result shown in FIG. 6 reveals that among the specimens of 58 cases grouped in the training set, 26 cases are determined as Gp-R and 32 cases are determined as Gp-NR by the discriminant represented by formula (I). Also, the result shown in FIG. 6 reveals that among the specimens determined as Gp-R, 16 cases are specimens of breast cancer patients of the pCR group, and among the specimens determined as Gp-NR, 30 cases are specimens of breast cancer patients of the npCR group. Therefore, these results demonstrate that the specimens sensitive to breast cancer neoadjuvant chemotherapy and the specimens insensitive to breast cancer neoadjuvant chemotherapy can be discriminated in the training set according to the discriminant represented by formula (I). Further, the result shown in FIG. 7 reveals that among the specimens of 59 cases grouped in the validation set, 24 cases are determined as Gp-R and 35 cases are determined as Gp-NR by the discriminant represented by formula (I). Also, the result shown in FIG. 7 reveals that among the specimens determined as Gp-R, 9 cases are specimens of breast cancer patients of the pCR group, and all of the specimens determined as Gp-NR are specimens of breast cancer patients of the npCR group. Therefore, these results demonstrate that the specimens sensitive to breast cancer neoadjuvant chemotherapy and the specimens insensitive to breast cancer neoadjuvant chemotherapy can be discriminated in the validation set according to the discriminant represented by formula (I).

These results suggest that the presence or absence of sensitivity to breast cancer neoadjuvant chemotherapy can be determined accurately by using the expression level of each nucleic acid detected by the probes of (1) to (23) in a specimen collected from a subject.

(Example 2)

From data sets of six subject groups of accession numbers: GSE16446 (subject groups 1-1), GSE20194 (subject groups 1-2), GSE20271 (subject groups 1-3), GSE22093 (subject groups 1-4), GSE23988 (subject groups 1-5) and GSE41998 (subject groups 1-6) in gene expression information database of microarray experiments (NCBI Gene Expression Omnibus (http://www.ncbi.nlm.nih.gov/geo/)), data of 901 cases of breast cancer cases having undergone neoadjuvant chemotherapy was extracted. For the extracted data, RMA normalization and mean-centering were conducted for each data set. Using the expression level of each nucleic acid detected by the probes of (1) to (23) in the resulting data, and the discriminant represented by formula (I), the presence or absence of sensitivity to breast cancer neoadjuvant chemotherapy was determined.

Next, for each data set, the determination result by the discriminant represented by formula (I) was compared with the pathologic diagnosis result acquired from the database, to thereby evaluate the performance of the discriminant. FIG. 8 illustrates the result of comparison between the determination result by the discriminant represented by formula (I) and the pathologic diagnosis result acquired from the database in Example 2.

Since the odds ratio exceeds 1 (greater than or equal to 3.09) and the minimum value in the 95% confidence interval exceeds 1 for all of the subject groups 1-1 to 1-6 as can be seen from the result shown in FIG. 8, it can be recognized that the determination by the discriminant represented by formula (I) is not an accidental result, but is a significant result in any cases.

Therefore, these results suggest that sensitivity to breast cancer neoadjuvant chemotherapy can be determined accurately by using the expression level of each nucleic acid detected by the probes of (1) to (23).

(Example 3)

The data of 901 cases extracted in Example 2 was classified into the following three data sets, subject groups 2-1 to 2-3, based on the presence or absence of each of ER and HER2:

subject group 2-1: group consisting of subjects showing ER positivity and HER2 negativity ($ER^+$, $HER2^-$),
subject group 2-2: group consisting of subjects showing ER positivity or ER negativity and HER2 positivity ($ER^{+-}$, $HER2^+$), and
subject group 2-3: group consisting of subjects showing ER negativity and HER2 negativity ($ER^-$, $HER2^-$). Since the "group consisting of subjects showing ER positivity and HER2 positivity ($ER^+$, $HER2^+$)" and the "group consisting of subjects showing ER negativity and HER2 positivity ($ER^-$, $HER2^+$)" are common in that they are sensitive to Herceptin, they are collected in subject group 2-2.

Next, for each data set, the determination result by the discriminant represented by formula (I) was compared with the pathologic diagnosis result acquired from the database, to thereby evaluate the performance of the discriminant. FIG. 9 illustrates the result of comparison between the determination result by the discriminant represented by formula (I) and the pathologic diagnosis result acquired from the database in Example 3.

Since the odds ratio exceeds 1 (greater than or equal to 3.41) and the minimum value in the 95% confidence interval exceeds 1 for all of the subject groups 2-1 to 2-3 as can be seen from the result shown in FIG. 9, it can be recognized that the determination by the discriminant represented by formula (I) is not an accidental result, but is a significant result in any cases. Therefore, these results suggest that sensitivity to breast cancer neoadjuvant chemotherapy can be determined accurately by using the expression level of each nucleic acid detected by the probes of (1) to (23) also in the data sets classified according to the presence or absence of each of ER and HER2. Also, these results suggest that the presence or absence of sensitivity to breast cancer neoadjuvant chemotherapy can be accurately determined irrespectively of the classification based on ER positivity and ER negativity by using the expression level of each nucleic acid detected by the probes of (1) to (23).

(Example 4)

The data of 901 cases extracted in Example 2 was classified into five data sets based on the kind of regimen of breast cancer neoadjuvant chemotherapy.

Then, for each data set, the determination result by the discriminant represented by formula (I) was compared with the pathologic diagnosis result acquired from the database, to thereby evaluate the performance of the discriminant. FIG. 10 illustrates the result of comparison between the determination result by the discriminant represented by formula (I) and the pathologic diagnosis result acquired from the database in Example 4. In the figure, "Epirubicin" denotes a group consisting of subjects administered with epirubicin (subject group 3-1), "FAC or FEC" denotes a group consisting of subjects administered with a combination of 5-fluorouracil, Adriamycin and cyclophosphamide, or a combination of 5-fluorouracil, epirubicin and cyclophosphamide (subject group 3-2), "A. paclitaxel" denotes a group consisting of subjects administered with anthracycline and paclitaxel (subject group 3-3), "A. docetaxel" denotes a group consisting of subjects administered with anthracycline and docetaxel (subject group 3-4), and "A. Ixabepilone" denotes a group consisting of subjects administered with anthracycline and ixabepilone (subject group 3-5).

Since the odds ratio exceeds 1 (greater than or equal to 3.45) and the minimum value in the 95% confidence interval exceeds 1 for all of the subject groups 3-1 to 3-5 as can be seen from the result shown in FIG. 10, it can be recognized that the determination by the discriminant represented by formula (I) is not an accidental result, but is a significant result in any cases. Therefore, these results suggest that sensitivity to breast cancer neoadjuvant chemotherapy can be determined accurately by using the expression level of each nucleic acid detected by the probes of (1) to (23) also in the data sets classified according to the used regimen of breast cancer neoadjuvant chemotherapy. Also, these results suggest that the presence or absence of sensitivity to breast cancer neoadjuvant chemotherapy can be accurately determined irrespectively of the classification based on the regimen of breast cancer neoadjuvant chemotherapy by using the expression level of each nucleic acid detected by the probes of (1) to (23).

(Comparative Example 1)

Using the expression levels of genes corresponding to 70 probe sets listed in Tables 1 and 2 of WO 2011/065533 A in the data of 901 cases extracted in Example 2, the presence or absence of sensitivity to breast cancer neoadjuvant chemotherapy was determined according to the method described in WO 2011/065533 A.

Next, for each data set of the subject groups 1-1 to 1-6, the determination result obtained by using the expression levels of genes corresponding to 70 probe sets listed in Tables 1 and 2 of WO 2011/065533 A was compared with the pathologic diagnosis result acquired from the database, to thereby evaluate the performance of the discriminant. FIG. 11 illustrates the result of comparison between the determination result obtained by using the expression levels of genes corresponding to 70 probe sets listed in Tables 1 and 2 of WO 2011/065533 A and the pathologic diagnosis result acquired from the database for the subject groups 1-1 to 1-6 in Comparative Example 1.

Also for each data set of the subject groups 2-1 to 2-3, the determination result obtained by using the expression levels of genes corresponding to 70 probe sets listed in Tables 1 and 2 of WO 2011/065533 A was compared with the pathologic diagnosis result acquired from the database, to thereby evaluate the performance of the discriminant. FIG. 12 illustrates the result of comparison between the determination result obtained by using the expression levels of genes corresponding to 70 probe sets listed in Tables 1 and 2 of WO 2011/065533 A and the pathologic diagnosis result acquired from the database for the subject groups 2-1 to 2-3 in Comparative Example 1.

Further, for each data set of the subject groups 3-1 to 3-5, the determination result obtained by using the expression levels of genes corresponding to 70 probe sets listed in Tables 1 and 2 of WO 2011/065533 A was compared with the pathologic diagnosis result acquired from the database, to thereby evaluate the performance of the discriminant. FIG. 13 illustrates the result of comparison between the determination result obtained by using the expression levels of genes corresponding to 70 probe sets listed in Tables 1 and 2 of WO 2011/065533 A and the pathologic diagnosis result acquired from the database for the subject groups 3-1 to 3-5 in Comparative Example 1. In the figure, "Epirubicin", "FAC or FEC", "A. paclitaxel", "A. docetaxel" and "A. Ixabepilone" are identical to "Epirubicin", "FAC or FEC", "A. paclitaxel", "A. docetaxel" and "A. Ixabepilone" in FIG. 10.

The result shown in FIG. 11 reveals that the reliability of the determination result can be poor depending on the kind of population of the subject group, since there is a case that both the odds ratio regarding the determination result and the minimum value in the 95% confidence interval are less than or equal to 1. Also the result shown in FIG. 12 reveals that the reliability of the determination result in each of the ER-positive cases and the ER-negative cases is poor when the expression levels of genes corresponding to 70 probe sets listed in Tables 1 and 2 of WO 2011/065533 A are used, since there is a case that the minimum value in the 95% confidence interval of odds ratio regarding the determination result is less than or equal to 1, also in the determination result for each data set of the subject groups classified according to the presence or absence of each of ER and HER2. Further, the result shown in FIG. 13 reveals that the reliability of the determination result can be poor depending on the kind of the administered anticancer agent when the expression levels of genes corresponding to 70 probe sets listed in Tables 1 and 2 of WO 2011/065533 A are used, since there is a case that both the odds ratio regarding the determination result and the minimum value in the 95% confidence interval are less than or equal to 1, also in the determination result for each data set of the subject groups classified according to the kind of the used regimen of breast cancer neoadjuvant chemotherapy.

(Comparative Example 2)

Using the expression level of B-cell metagene described in Schmidt M et al. (Cancer research, 2008, Vol. 68, p. 5405-5413) in the data of 901 cases extracted in Example 2, the presence or absence of sensitivity to breast cancer neoadjuvant chemotherapy was determined according to the method described in Schmidt M et al. (Cancer research, 2008, Vol. 68, p. 5405-5413).

Next, for each data set of the subject groups 1-1 to 1-6, the determination result obtained according to the method described in Schmidt M et al. (Cancer research, 2008, Vol. 68, p. 5405-5413) was compared with the pathologic diagnosis result acquired from the database, to thereby evaluate the performance of the discriminant. FIG. 14 illustrates the result of comparison between the determination result obtained according to the method described in Schmidt M et al. (Cancer research, 2008, Vol. 68, p. 5405-5413) and the pathologic diagnosis result acquired from the database for the subject groups 1-1 to 1-6 in Comparative Example 2.

For each data set of the subject groups 2-1 to 2-3, the determination result obtained according to the method described in Schmidt M et al. (Cancer research, 2008, Vol. 68, p. 5405-5413) was compared with the pathologic diagnosis result acquired from the database, to thereby evaluate the performance of the discriminant. FIG. 15 illustrates the result of comparison between the determination result obtained according to the method described in Schmidt M et al. (Cancer research, 2008, Vol. 68, p. 5405-5413) and the pathologic diagnosis result acquired from the database for the subject groups 2-1 to 2-3 in Comparative Example 2.

Further, for each data set of the subject groups 3-1 to 3-5, the determination result obtained according to the method described in Schmidt M et al. (Cancer research, 2008, Vol. 68, p. 5405-5413) was compared with the pathologic diagnosis result acquired from the database, to thereby evaluate the performance of the discriminant. FIG. 16 illustrates the result of comparison between the determination result obtained according to the method described in Schmidt M et al. (Cancer research 2008, Vol. 68, p. 5405-5413) and the pathologic diagnosis result acquired from the database for the subject groups 3-1 to 3-5 in Comparative Example 2. In the figure, "Epirubicin", "FAC or FEC", "A. paclitaxel", "A. docetaxel" and "A. Ixabepilone" are identical to "Epirubicin", "FAC or FEC", "A. paclitaxel", "A. docetaxel" and "A. Ixabepilone" in FIG. 10.

The result shown in FIG. 14 reveals that the reliability of the determination result can be poor depending on the kind of population of the subject group according to the method described in Schmidt M et al. (Cancer research, 2008, Vol. 68, p. 5405-5413), since there is a case that the minimum value in the 95% confidence interval of odds ratio regarding the determination result is less than or equal to 1. Also the result shown in FIG. 15 reveals that the method described in Schmidt M et al. (Cancer research, 2008, Vol. 68, p. 5405-5413) is poor in reliability of the determination result in each of the ER-positive cases and the ER-negative cases, since there is a case that the minimum value in the 95% confidence interval of odds ratio regarding the determination result is less than or equal to 1, also in the determination result for each data set of the subject groups classified according to the presence or absence of each of ER and HER2. Further, the result shown in FIG. 16 reveals that according to the method described in Iwamoto T et al. (Journal of the National Cancer Institute, 2011, Vol. 103, p. 264-272), the reliability of the determination result can be poor depending on the kind of the administered anticancer agent, since there is a case that the minimum value in the 95% confidence interval of odds ratio regarding the determination result is less than or equal to 1, also in the determination result for each data set of the subject groups classified according to the kind of the used regimen of breast cancer neoadjuvant chemotherapy.

(Comparative Example 3)

Using the expression level of immunogloblin κC gene described in Schmidt M et al. (Clinical Cancer Research, 2012, Vol. 18, p. 2695-2703) in the data of 901 cases extracted in Example 2, the presence or absence of sensitivity to breast cancer neoadjuvant chemotherapy was determined according to the method described in Schmidt M et al. (Clinical' Cancer Research, 2012, Vol. 18, p. 2695-2703).

Next, for each data set of the subject groups 1-1 to 1-6, the determination result obtained according to the method described in Schmidt M et al. (Clinical Cancer Research, 2012, Vol. 18, p. 2695-2703) was compared with the pathologic diagnosis result acquired from the database, to thereby evaluate the performance of the discriminant. FIG. 17 illustrates the result of comparison between the determination result obtained according to the method described in Schmidt M et al. (Clinical Cancer Research, 2012, Vol. 18, p. 2695-2703) and the pathologic diagnosis result acquired from the database for the subject groups 1-1 to 1-6 in Comparative Example 3.

For each data set of the subject groups 2-1 to 2-3, the determination result obtained according to the method described in Schmidt M et al. (Clinical Cancer Research, 2012, Vol. 18, p. 2695-2703) was compared with the pathologic diagnosis result acquired from the database, to thereby evaluate the performance of the discriminant. FIG. 18 illustrates the result of comparison between the determination result obtained according to the method described in Schmidt M et al. (Clinical Cancer Research, 2012, Vol. 18, p. 2695-2703) and the pathologic diagnosis result acquired from the database for the subject groups 2-1 to 2-3 in Comparative Example 3.

Further, for each data set of the subject groups 3-1 to 3-5, the determination result obtained according to the method described in Schmidt M et al. (Clinical Cancer Research, 2012, Vol. 18, p. 2695-2703) was compared with the pathologic diagnosis result acquired from the database, to thereby evaluate the performance of the discriminant. FIG. 19 illustrates the result of comparison between the determination result obtained according to the method described in Schmidt M et al. (Clinical Cancer. Research, 2012, Vol. 18, p. 2695-2703) and the pathologic diagnosis result acquired from the database for the subject groups 3-1 to 3-5 in Comparative Example 3. In the figure, "Epirubicin", "FAC or FEC", "A. paclitaxel", "A. docetaxel" and "A. Ixabepilone" are identical to "Epirubicin", "FAC or FEC", "A. paclitaxel", "A. docetaxel" and "A. Ixabepilone" in FIG. 10.

The result shown in FIG. 17 reveals that the reliability of the determination result can be poor depending on the kind of population of the subject group according to the method described in Schmidt M et al. (Clinical Cancer Research, 2012, Vol. 18, p. 2695-2703), since there is a case that the minimum value in the 95% confidence interval of odds ratio regarding the determination result is less than or equal to 1. The result shown in FIG. 18 reveals that the reliability of the determination result in each of the ER-positive cases and the ER-negative cases is poorer by the method described in Schmidt M et al. (Clinical Cancer Research, 2012, Vol. 18, p. 2695-2703) than by the method according to the present embodiment using the expression level of each nucleic acid detected by the probes of (1) to (23), since the odds ratio regarding the determination result is smaller in comparison with the case where sensitivity to breast cancer neoadjuvant chemotherapy is determined by the method according to the present embodiment using an expression level of each nucleic acid detected by the probes (1) to (23). Further, the result shown in FIG. 19 reveals that the reliability of the determination result can be poor depending on the kind of the administered anticancer agent according to the method described in Schmidt M et al. (Clinical Cancer Research, 2012, Vol. 18, p. 2695-2703), since there is a case that the minimum value in the 95% confidence interval of odds ratio regarding the determination result is less than or equal to 1, also in the determination result for each data set of the subject groups classified according to the kind of the used regimen of breast cancer neoadjuvant chemotherapy.

(Comparative Example 4)

Using the expression level of each gene of C1QA, XCL2, SPP1, TNFRSF17, LY9, IGLC2 and HLA-F described in Teschendorff AE et al. (Genome Biology, 2007, Vol. 8, R157) in the data of 901 cases extracted in Example 2, the presence or absence of sensitivity to breast cancer neoadjuvant chemotherapy was determined according to the method described in Teschendorff AE et al. (Genome Biology, 2007, Vol. 8, R157).

Next, for each data set of the subject groups 1-1 to 1-6, the determination result obtained according to the method described in Teschendorff AE et al. (Genome Biology, 2007, Vol. 8, R157) was compared with the pathologic diagnosis result acquired from the database, to thereby evaluate the performance of the discriminant. FIG. 20 illustrates the result of comparison between the determination result obtained according to the method described in Teschendorff AE et al. (Genome Biology, 2007, Vol. 8, R157) and the pathologic diagnosis result acquired from the database for the subject groups 1-1 to 1-6 in Comparative Example 4.

For each data set of the subject groups 2-1 to 2-3, the determination result obtained according to the method described in Teschendorff AE et al. (Genome Biology, 2007, Vol. 8, R157) was compared with the pathologic diagnosis result acquired from the database, to thereby evaluate the performance of the discriminant. FIG. 21 illustrates the result of comparison between the determination result obtained according to the method described in Thechendorff AE et al. (Genome Biology, 2007, Vol. 8, R157) and the pathologic diagnosis result acquired from the database for the subject groups 2-1 to 2-3 in Comparative Example 4.

Further, for each data set of the subject groups 3-1 to 3-5, the determination result obtained according to the method described in Teschendorff AE et al. (Genome Biology, 2007, Vol. 8, R157) was compared with the pathologic diagnosis result acquired from the database, to thereby evaluate the performance of the discriminant. FIG. 22 illustrates the result of comparison between the determination result obtained according to the method described in Teschendorff AE et al. (Genome Biology, 2007, Vol. 8, R157) and the pathologic diagnosis result acquired from the database for the subject groups 3-1 to 3-5 in Comparative Example 4. In the figure, "Epirubicin", "FAC or FEC", "A. paclitaxel", "A. docetaxel" and "A. Ixabepilone" are identical to "Epirubicin", "FAC or FEC", "A. paclitaxel", "A. docetaxel" and "A. Ixabepilone" in FIG. 10.

The result shown in FIG. 20 reveals that the reliability of the determination result can be poor depending on the kind of population of the subject group according to the method described in Teschendorff AE et al. (Genome Biology, 2007, Vol. 8, R157), since there is a case that the minimum value in the 95% confidence interval of odds ratio regarding the determination result is less than or equal to 1. Also the result shown in FIG. 21 reveals that the method described in Teschendorff AE et al. (Genome Biology, 2007, Vol. 8, R157) is poor in reliability of the determination result in each of the ER-positive cases and the ER-negative cases, since there is a case that the minimum value in the 95% confidence interval of odds ratio regarding the determination result is less than or equal to 1, also in the determination result for each data set of the subject groups classified according to the presence or absence of each of ER and HER2. Further, the result shown in FIG. 22 reveals that according to the method described in Teschendorff AE et al. (Genome Biology, 2007, Vol. 8, R157), the reliability of the determination result can be poor depending on the kind of the administered anticancer agent, since there is a case that the minimum value in the 95% confidence interval of odds ratio regarding the determination result is less than or equal to 1, also in the determination result for each data set of the subject groups classified according to the kind of the used regimen of breast cancer neoadjuvant chemotherapy.

These results suggest that the presence or absence of sensitivity to breast cancer neoadjuvant chemotherapy can be determined more accurately irrespectively of the classification based on ER positivity and ER negativity, and the classification based on the kind of regimen of breast cancer neoadjuvant chemotherapy by using the expression level of each nucleic acid detected by the probes of (1) to (23). Therefore, the method according to the present embodiment is suited for providing information for assisting decision of adaptability to execution of breast cancer neoadjuvant chemotherapy or more appropriate information for optimizing the therapy.

SEQ ID NOs: 24 to 272 are sequences contained in probe sets.

## Claims

1. A method for determining sensitivity to breast cancer neoadjuvant chemotherapy comprising the steps of:

    (1) preparing a measurement sample comprising RNA from a.specimen collected from a subject,
    (2) measuring an expression level of each gene of (A1) to (A19) below making use of a measurement sample obtained in step (1),
    (3) analyzing an expression level of each gene measured in step (2), and
    (4) determining sensitivity to breast cancer neoadjuvant chemotherapy on the basis of an analysis result obtained in step (3), wherein

        (A1) is human caspase recruitment domain family, member 9 (CARD9) gene,
        (A2) is human indoleamine-2,3-dioxygenase 1 (IDO1) gene,
        (A3) is human chemokine (C-X-C motif) ligand 9 (CXCL9) gene,
        (A4) is human purine nucleoside phosphorylase (PNP) gene,
        (A5) is human chemokine (C-X-C motif) ligand 11 (CXCL11) gene,
        (A6) is human CCAAT/enhancer binding protein (CEBPB) gene,
        (A7) is human CD83 gene,
        (A8) is human interleukin 6 signal transducer (IL6ST) gene,
        (A9) is human chemokine (C-X3-C) receptor 1 (CX3CR1) gene,
        (A10) is human CD1D gene,
        (A11) is human cathepsin C (CTSC) gene,
        (A12) is human chemokine (C-X-C motif) ligand 10 (CXCL10) gene,
        (A13) is human immunoglobulin heavy chain genetic locus G1 isotype (IGHG1) gene,
        (A14) is human zinc finger E-box-binding homeobox 1 (ZEB1) gene,
        (A15) is human vascular endothelial growth factor A (VEGFA) gene,
        (A16) is human semaphorin-3C precursor (SEMA3C) gene,
        (A17) is human complement receptor (CR2) gene,
        (A18) is human HFE gene, and
        (A19) is human EDA gene.

2. The method according to claim 1, wherein
the gene (A1) comprises a nucleic acid detected by a probe targeting a polynucleotide having a nucleotide sequence of SEQ ID NO: 1,
the gene (A2) comprises a nucleic acid detected by a probe targeting a polynucleotide having a nucleotide sequence of SEQ ID NO: 2,
the gene (A3) comprises a nucleic acid detected by a probe targeting a polynucleotide having a nucleotide sequence of SEQ ID NO: 3,
the gene (A4) comprises a nucleic acid detected by a probe targeting a polynucleotide having a nucleotide sequence of SEQ ID NO: 4,
the gene (A5) comprises a nucleic acid detected by a probe targeting a polynucleotide having a nucleotide sequence of SEQ ID NO: 5 or SEQ ID NO: 23,
the gene (A6) comprises a nucleic acid detected by a probe targeting a polynucleotide having a nucleotide sequence of SEQ ID NO: 6,
the gene (A7) comprises a nucleic acid detected by a probe targeting a polynucleotide having a nucleotide sequence of SEQ ID NO: 7,
the gene (A8) comprises a nucleic acid detected by a probe targeting a polynucleotide having a nucleotide sequence of SEQ ID NO: 8, SEQ ID NO: 14 or SEQ ID NO: 19,
the gene (A9) comprises a nucleic acid detected by a probe targeting a polynucleotide having a nucleotide sequence of SEQ ID NO: 9,
the gene (A10) comprises a nucleic acid detected by a probe targeting a polynucleotide having a nucleotide sequence of SEQ ID NO: 10,
the gene (A11) comprises a nucleic acid detected by a probe targeting a polynucleotide having a nucleotide sequence of SEQ ID NO: 11,
the gene (A12) comprises a nucleic acid detected by a probe targeting a polynucleotide having a nucleotide sequence of SEQ ID NO: 12,
the gene (A13) comprises a nucleic acid detected by a probe targeting a polynucleotide having a nucleotide sequence of SEQ ID NO: 13,
the gene (A14) comprises a nucleic acid detected by a probe targeting a polynucleotide having a nucleotide sequence of SEQ ID NO: 15 or SEQ ID NO: 22,
the gene (A15) comprises a nucleic acid detected by a probe targeting a polynucleotide having a nucleotide sequence of SEQ ID NO: 16,
the gene (A16) comprises a nucleic acid detected by a probe targeting a polynucleotide having a nucleotide sequence of SEQ ID NO: 17,
the gene (A17) comprises a nucleic acid detected by a probe targeting a polynucleotide having a nucleotide sequence of SEQ ID NO: 18,
the gene (A18) comprises a nucleic acid detected by a probe targeting a polynucleotide having a nucleotide sequence of SEQ ID NO: 20, and
the gene (A19) comprises a nucleic acid detected by a probe targeting a polynucleotide having a nucleotide sequence of SEQ ID NO: 21.

**3.** The method according to claim 2, wherein the expression level is measured by using a probe set shown in Table A.

TABLE A

| SEQ ID NO of targeted polynucleotide | SEQ ID NO of probe |
|---|---|
| 1 | 24~34 |
| 2 | 35~45 |
| 3 | 46~56 |
| 4 | 57~67 |
| 5 | 68~78 |
| 6 | 79~89 |
| 7 | 90~100 |
| 8 | 101~111 |
| 9 | 112~122 |
| 10 | 123~133 |
| 11 | 134~144 |
| 12 | 145~155 |
| 13 | 156~166 |
| 14 | 167~177 |
| 15 | 178~188 |
| 16 | 189~199 |
| 17 | 200~210 |
| 18 | 211~-221 |
| 19 | 222~232 |
| 20 | 233~243 |
| 21 | 244~254 |
| 22 | 255~265 |

(continued)

| SEQ ID NO of targeted polynucleotide | SEQ ID NO of probe |
|---|---|
| 23 | 266~276 |

**4.** The method according to claim 2 or 3, wherein analysis of the expression level is conducted by using a classification technique, a scoring technique or a cluster analyzing technique.

**5.** The method according to claim 4, wherein analysis of the expression level is conducted by using a classification technique.

**6.** The method according to claim 4, wherein the classification technique is a diagonal linear discriminant analysis.

**7.** The method according to claim 5, wherein in analysis of the expression level, solution D of the discriminant is determined by using the expression level and a discriminant represented by formula (I):

$$D = \sum_i (w_i \times y_i) - 3.327217 \qquad (\text{I})$$

(wherein, i represents a number assigned to each nucleic acid shown in Table B so as to correspond to SEQ ID NO of the targeted polynucleotide, $w_i$ represents a weighting factor of nucleic acid of number i shown in Table B, $y_i$ represents a standardized expression level of nucleic acid, the standardized expression level being obtained by standardizing an expression level of nucleic acid according to the formula represented by formula (II):

$$y_i = x_i - m_i \qquad (\text{II})$$

(wherein, $x_i$ represents an expression level of nucleic acid of number i shown in Table B, and $m_i$ represents a mean value of expression level of nucleic acid of number i shown in Table B over the specimens), and $\sum$i represents a sum total over the respective nucleic acids), and
wherein a determination of being sensitive to breast cancer neoadjuvant chemotherapy is made when solution D of the discriminant is a positive value, and a determination of being insensitive to breast cancer neoadjuvant chemotherapy is made when solution D is 0 or a negative value in the step (4).

TABLE B

| Number i of nucleic acid | SEQ ID NO of targeted polynucleotide | Weighting factor |
|---|---|---|
| 1 | 1 | 2.361579818 |
| 2 | 2 | 0.527535817 |
| 3 | 3 | 0.53572137 |
| 4 | 4 | 1.296736029 |
| 5 | 5 | 0.437766376 |
| 6 | 6 | 1.09614395 |
| 7 | 7 | 1.154132786 |
| 8 | 8 | -0.997955474 |
| 9 | 9 | -0.84645569 |
| 10 | 10 | 0.703499669 |
| 11 | 11 | 1.262066324 |
| 12 | 12 | 0.481709248 |
| 13 | 13 | 0.784677171 |

(continued)

| Number i of nucleic acid | SEQ ID NO of targeted polynucleotide | Weighting factor |
|---|---|---|
| 14 | 14 | -1.056130291 |
| 15 | 15 | -0.90152985 |
| 16 | 16 | 0.941011796 |
| 17 | 17 | -0.580145259 |
| 18 | 18 | 0.797198448 |
| 19 | 19 | -0.963860205 |
| 20 | 20 | -1.352304026 |
| 21 | 21 | -1.231365097 |
| 22 | 22 | -0.637818166 |
| 23 | 23 | 0.449217729 |

**8.** The method according to any one of claims 1 to 7, wherein the specimen comprises a breast cancer cell.

**9.** The method according to any one of claims 1 to 8, wherein the expression level is measured by using a microarray, a nucleic acid amplification method or a Northern blot analysis.

**10.** The method according to claim 9, wherein the expression level is measured by using a microarray.

**11.** The method according to claim 10, wherein the microarray is a microarray having a probe set shown in the Table A arranged on a base material.

**12.** The method according to claim 9, wherein the expression level is measured by using a nucleic acid amplification method.

**13.** The method according to claim 12, wherein the nucleic acid amplification method is quantitative RT-PCR or quantitative PCR.

**14.** A computer program causing a computer to function as a control section for analyzing an expression level of each nucleic acid corresponding to the probes of (B1) to (B23) below in a measurement sample comprising RNA prepared from a specimen collected from a subject, and determining sensitivity to breast cancer neoadjuvant chemotherapy on the basis of obtained information of the expression level, the control section being configured to execute:

calculation of solution D based on a discriminant represented by formula (I):

$$D = \sum_i (w_i \times y_i) - 3.327217 \qquad (\mathrm{I})$$

(wherein, i represents a number assigned to each nucleic acid shown in Table B so as to correspond to SEQ ID NO of the targeted polynucleotide, $w_i$ represents a weighting factor of nucleic acid of number i shown in Table B, $y_i$ represents a standardized expression level of nucleic acid, the standardized expression level being obtained by standardizing an expression level of nucleic acid according to the formula represented by formula (II):

$$y_i = x_i - m_i \qquad (\mathrm{II})$$

(wherein, $x_i$ represents an expression level of nucleic acid of number i shown in Table B, and $m_i$ represents a mean value of expression level of nucleic acid of number i shown in Table B over the specimens), and $\sum_i$ represents a sum total over the respective nucleic acids), and

sensitivity determination that makes a determination of being sensitive to breast cancer neoadjuvant chemotherapy when solution D of the discriminant is a positive value, and makes a determination of being insensitive to breast cancer neoadjuvant chemotherapy when solution D is 0 or a negative value, wherein

(B1) is a probe targeting a polynucleotide having a nucleotide sequence of SEQ ID NO: 1,
(B2) is a probe targeting a polynucleotide having a nucleotide sequence of SEQ ID NO: 2,
(B3) is a probe targeting a polynucleotide having a nucleotide sequence of SEQ ID NO: 3,
(B4) is a probe targeting a polynucleotide having a nucleotide sequence of SEQ ID NO: 4,
(B5) is a probe targeting a polynucleotide having a nucleotide sequence of SEQ ID NO: 5,
(B6) is a probe targeting a polynucleotide having a nucleotide sequence of SEQ ID NO: 6,
(B7) is a probe targeting a polynucleotide having a nucleotide sequence of SEQ ID NO: 7,
(B8) is a probe targeting a polynucleotide having a nucleotide sequence of SEQ ID NO: 8,
(B9) is a probe targeting a polynucleotide having a nucleotide sequence of SEQ ID NO: 9,
(B10) is a probe targeting a polynucleotide having a nucleotide sequence of SEQ ID NO: 10,
(B11) is a probe targeting a polynucleotide having a nucleotide sequence of SEQ ID NO: 11,
(B12) is a probe targeting a polynucleotide having a nucleotide sequence of SEQ ID NO: 12,
(B13) is a probe targeting a polynucleotide having a nucleotide sequence of SEQ ID NO: 13,
(B14) is a probe targeting a polynucleotide having a nucleotide sequence of SEQ ID NO: 14,
(B15) is a probe targeting a polynucleotide having a nucleotide sequence of SEQ ID NO: 15,
(B16) is a probe targeting a polynucleotide having a nucleotide sequence of SEQ ID NO: 16,
(B17) is a probe targeting a polynucleotide having a nucleotide sequence of SEQ ID NO: 17,
(B18) is a probe targeting a polynucleotide having a nucleotide sequence of SEQ ID NO: 18,
(B19) is a probe targeting a polynucleotide having a nucleotide sequence of SEQ ID NO: 19,
(B20) is a probe targeting a polynucleotide having a nucleotide sequence of SEQ ID NO: 20,
(B21) is a probe targeting a polynucleotide having a nucleotide sequence of SEQ ID NO: 21,
(B22) is a probe targeting a polynucleotide having a nucleotide sequence of SEQ ID NO: 22, and
(B23) is a probe targeting a polynucleotide having a nucleotide sequence of SEQ ID NO: 23, wherein Table B is:

TABLE B

| Number i of nucleic acid | SEQ ID NO of targeted polynucleotide | Weighting factor |
|---|---|---|
| 1 | 1 | 2.361579818 |
| 2 | 2 | 0.527535817 |
| 3 | 3 | 0.53572137 |
| 4 | 4 | 1.296736029 |
| 5 | 5 | 0.437766376 |
| 6 | 6 | 1.09614395 |
| 7 | 7 | 1.154132786 |
| 8 | 8 | -0.997955474 |
| 9 | 9 | -0.84645569 |
| 10 | 10 | 0.703499669 |
| 11 | 11 | 1.262066324 |
| 12 | 12 | 0.481709248 |
| 13 | 13 | 0.784677171 |
| 14 | 14 | -1.056130291 |
| 15 | 15 | -0.90152985 |
| 16 | 16 | 0.941011796 |
| 17 | 17 | -0.580145259 |
| 18 | 18 | 0.797198448 |
| 19 | 19 | -0.963860205 |

(continued)

| Number i of nucleic acid | SEQ ID NO of targeted polynucleotide | Weighting factor |
|---|---|---|
| 20 | 20 | -1.352304026 |
| 21 | 21 | -1.231365097 |
| 22 | 22 | -0.637818166 |
| 23 | 23 | 0.449217729 |

**Patentansprüche**

1. Verfahren zur Bestimmung der Empfindlichkeit gegenüber einer neoadjuvanten Chemotherapie für Brustkrebs, das die folgenden Schritte umfasst:

   (1) Herstellen einer Messprobe, die RNA aus einem Präparat umfasst, das einem Subjekt entnommen wurde,
   (2) Messen eines Expressionsniveaus von jedem der nachfolgenden Gene (A1) bis (A19), wobei Verwendung von einer in Schritt (1) erhaltenen Messprobe gemacht wird,
   (3) Analysieren eines Expressionsniveaus von jedem in Schritt (2) gemessenen Gen, und
   (4) Bestimmung der Empfindlichkeit gegenüber einer neoadjuvanten Chemotherapie für Brustkrebs auf der Basis eines in Schritt (3) erhaltenen Analyseergebnisses, wobei

   (A1) das Mitglied-9(CARD9)-Gen der menschlichen Caspase-Rekrutierungdomänen-Familie ist,
   (A2) das menschliche Indolamin-2,3-Dioxygenase-1(IDO1)-Gen ist,
   (A3) das menschliche Chemokin(CXC-Motiv)-Ligand-9(CXCL9)-Gen ist,
   (A4) das menschliche Purinnukleosidphosphorylase(PNP)-Gen ist,
   (A5) das menschliche Chemokin(CXC-Motiv)-Ligand-11(CXCL11)-Gen ist,
   (A6) das menschliche CCAAT/Enhancer-Bindungsprotein(CEBPB)-Gen ist,
   (A7) das menschliche CD83-Gen ist,
   (A8) das menschliche Interleukin-6-Signalüberträger(IL6ST)-Gen ist,
   (A9) das menschliche Chemokin(C-X3-C)-Rezeptor-1 (CX3CR1)-Gen ist,
   (A10)das menschliche CD1D-Gen ist,
   (A11)das menschliche Cathepsin-C(CTSC)-Gen ist,
   (A12)das menschliche Chemokin(CXC-Motiv)-Ligand-10(CXCL10)-Gen ist,
   (A13)das menschliche Immunglobulin-schwere-Kette-Genort-G1-Isotyp(IGHG1)-Gen ist,
   (A14)das menschliche Zinkfinger-E-Box-bindende Homeobox-1(ZEB1)-Gen ist,
   (A15)das menschliche Gen des vaskulären endothelialen Wachstumsfaktors A (VEGFA) ist,
   (A16)das menschliche Semaphorin-3C-Vorläufer(SEMA3C)-Gen ist,
   (A17)das menschliche Komplement-Rezeptor(CR2)-Gen ist,
   (A18)das menschliche HFE-Gen ist, und
   (A19)das menschliche EDA-Gen ist.

2. Verfahren nach Anspruch 1, wobei
   das Gen (A1) eine Nukleinsäure umfasst, die mit einer Sonde nachgewiesen wird, welche auf ein Polynukleotid abzielt, das eine Nukleotidsequenz von SEQ ID NO: 1 aufweist,
   das Gen (A2) eine Nukleinsäure umfasst, die mit einer Sonde nachgewiesen wird, welche auf ein Polynukleotid abzielt, das eine Nukleotidsequenz von SEQ ID NO: 2 aufweist,
   das Gen (A3) eine Nukleinsäure umfasst, die mit einer Sonde nachgewiesen wird, welche auf ein Polynukleotid abzielt, das eine Nukleotidsequenz von SEQ ID NO: 3 aufweist,
   das Gen (A4) eine Nukleinsäure umfasst, die mit einer Sonde nachgewiesen wird, welche auf ein Polynukleotid abzielt, das eine Nukleotidsequenz von SEQ ID NO: 4 aufweist,
   das Gen (A5) eine Nukleinsäure umfasst, die mit einer Sonde nachgewiesen wird, welche auf ein Polynukleotid abzielt, das eine Nukleotidsequenz von SEQ ID NO: 5 oder SEQ ID NO: 23 aufweist,
   das Gen (A6) eine Nukleinsäure umfasst, die mit einer Sonde nachgewiesen wird, welche auf ein Polynukleotid abzielt, das eine Nukleotidsequenz von SEQ ID NO: 6 aufweist,
   das Gen (A7) eine Nukleinsäure umfasst, die mit einer Sonde nachgewiesen wird, welche auf ein Polynukleotid abzielt, das eine Nukleotidsequenz von SEQ ID NO: 7 aufweist,

das Gen (A8) eine Nukleinsäure umfasst, die mit einer Sonde nachgewiesen wird, welche auf ein Polynukleotid abzielt, das eine Nukleotidsequenz von SEQ ID NO: 8, SEQ ID NO: 14 oder SEQ ID NO: 19 aufweist,
das Gen (A9) eine Nukleinsäure umfasst, die mit einer Sonde nachgewiesen wird, welche auf ein Polynukleotid abzielt, das eine Nukleotidsequenz von SEQ ID NO: 9 aufweist,
das Gen (A10) eine Nukleinsäure umfasst, die mit einer Sonde nachgewiesen wird, welche auf ein Polynukleotid abzielt, das eine Nukleotidsequenz von SEQ ID NO: 10 aufweist,
das Gen (A11) eine Nukleinsäure umfasst, die mit einer Sonde nachgewiesen wird, welche auf ein Polynukleotid abzielt, das eine Nukleotidsequenz von SEQ ID NO: 11 aufweist,
das Gen (A12) eine Nukleinsäure umfasst, die mit einer Sonde nachgewiesen wird, welche auf ein Polynukleotid abzielt, das eine Nukleotidsequenz von SEQ ID NO: 12 aufweist,
das Gen (A13) eine Nukleinsäure umfasst, die mit einer Sonde nachgewiesen wird, welche auf ein Polynukleotid abzielt, das eine Nukleotidsequenz von SEQ ID NO: 13 aufweist,
das Gen (A14) eine Nukleinsäure umfasst, die mit einer Sonde nachgewiesen wird, welche auf ein Polynukleotid abzielt, das eine Nukleotidsequenz von SEQ ID NO: 15 oder SEQ ID NO: 22 aufweist,
das Gen (A15) eine Nukleinsäure umfasst, die mit einer Sonde nachgewiesen wird, welche auf ein Polynukleotid abzielt, das eine Nukleotidsequenz von SEQ ID NO: 16 aufweist,
das Gen (A16) eine Nukleinsäure umfasst, die mit einer Sonde nachgewiesen wird, welche auf ein Polynukleotid abzielt, das eine Nukleotidsequenz von SEQ ID NO: 17 aufweist,
das Gen (A17) eine Nukleinsäure umfasst, die mit einer Sonde nachgewiesen wird, welche auf ein Polynukleotid abzielt, das eine Nukleotidsequenz von SEQ ID NO: 18 aufweist,
das Gen (A18) eine Nukleinsäure umfasst, die mit einer Sonde nachgewiesen wird, welche auf ein Polynukleotid abzielt, das eine Nukleotidsequenz von SEQ ID NO: 20 aufweist, und
das Gen (A19) eine Nukleinsäure umfasst, die mit einer Sonde nachgewiesen wird, welche auf ein Polynukleotid abzielt, das eine Nukleotidsequenz von SEQ ID NO: 21 aufweist.

**3.** Verfahren nach Anspruch 2, wobei das Expressionsniveau durch die Verwendung eines in Tabelle A gezeigten Sondensatzes gemessen wird.

TABELLE A

| SEQ ID NO des Zielpolynukleotids | SEQ ID NO der Sonde |
|---|---|
| 1 | 24-34 |
| 2 | 35-45 |
| 3 | 46-56 |
| 4 | 57-67 |
| 5 | 68-78 |
| 6 | 79-89 |
| 7 | 90-100 |
| 8 | 101-111 |
| 9 | 112-122 |
| 10 | 123-133 |
| 11 | 134-144 |
| 12 | 145-155 |
| 13 | 156-166 |
| 14 | 167-177 |
| 15 | 178-188 |
| 16 | 189-199 |
| 17 | 200-210 |
| 18 | 211-221 |

(fortgesetzt)

| SEQ ID NO des Zielpolynukleotids | SEQ ID NO der Sonde |
|---|---|
| 19 | 222-232 |
| 20 | 233-243 |
| 21 | 244-254 |
| 22 | 255-265 |
| 23 | 266-276 |

**4.** Verfahren nach Anspruch 2 oder 3, wobei die Analyse des Expressionsniveaus unter Verwendung einer Klassifizierungstechnik, einer Bewertungstechnik oder einer Cluster-Analysetechnik durchgeführt wird.

**5.** Verfahren nach Anspruch 4, wobei die Analyse des Expressionsniveaus unter Verwendung einer Klassifizierungstechnik durchgeführt wird.

**6.** Verfahren nach Anspruch 4, worin die Klassifizierungstechnik eine diagonale lineare Diskriminanzanalyse ist.

**7.** Verfahren nach Anspruch 5, wobei bei der Analyse des Expressionsniveaus Lösung D der Diskriminante unter Verwendung des Expressionsniveaus und einer durch Formel (I) dargestellten Diskriminante bestimmt wird:

$$D = \sum_i (w_i \times y_i) - 3{,}327217 \qquad \text{(I)}$$

(wobei i eine Nummer darstellt, die jeder in Tabelle B dargestellten Nukleinsäure zugeordnet wird, so dass sie der SEQ ID NO des Zielpolynukleotids entspricht, $w_i$ für einen Wichtungsfaktor der in Tabelle B gezeigten Nukleinsäure mit der Nummer i steht, $y_i$ für ein standardisiertes Expressionsniveau der Nukleinsäure steht, wobei das standardisierte Expressionsniveau durch die Standardisierung eines Expressionsniveaus der Nukleinsäure gemäß der durch die Formel (II) dargestellten Formel erhalten wird:

$$y_i = x_i - m_i \qquad \text{(II)}$$

(wobei $x_i$ für ein Expressionsniveau der in Tabelle B gezeigten Nukleinsäure mit der Nummer i steht, und $m_i$ für einen Mittelwert des Expressionsniveaus der in Tabelle B gezeigten Nukleinsäure mit der Nummer i über die Präparate steht) und $\sum_i$ für eine Gesamtsumme über die entsprechenden Nukleinsäuren steht) und
wobei eine Bestimmung der Empfindlichkeit gegenüber einer neoadjuvanten Chemotherapie für Brustkrebs durchgeführt wird, wenn die Lösung D der Diskriminante einen positiven Wert annimmt, und eine Bestimmung der Unempfindlichkeit gegenüber einer neoadjuvanten Chemotherapie für Brustkrebs durchgeführt wird, wenn die Lösung D 0 oder einen negativen Wert in Schritt (4) annimmt.

TABELLE B

| Nummer i der Nukleinsäure | SEQ ID NO des Zielpolynukleotids | Wichtungsfaktor |
|---|---|---|
| 1 | 1 | 2,361579818 |
| 2 | 2 | 0,527535817 |
| 3 | 3 | 0,53572137 |
| 4 | 4 | 1,296736029 |
| 5 | 5 | 0,437766376 |
| 6 | 6 | 1,09614395 |
| 7 | 7 | 1,154132786 |
| 8 | 8 | -0,997955474 |

(fortgesetzt)

| Nummer i der Nukleinsäure | SEQ ID NO des Zielpolynukleotids | Wichtungsfaktor |
|---|---|---|
| 9 | 9 | -0,84645569 |
| 10 | 10 | 0,703499669 |
| 11 | 11 | 1,262066324 |
| 12 | 12 | 0,481709248 |
| 13 | 13 | 0,784677171 |
| 14 | 14 | -1,056130291 |
| 15 | 15 | -0,90152985 |
| 16 | 16 | 0,941011796 |
| 17 | 17 | -0,580145259 |
| 18 | 18 | 0,797198448 |
| 19 | 19 | -0,963860205 |
| 20 | 20 | -1,352304026 |
| 21 | 21 | -1,231365097 |
| 22 | 22 | -0,637818166 |
| 23 | 23 | 0,449217729 |

**8.** Verfahren nach einem der Ansprüche 1 bis 7, wobei das Präparat eine Brustkrebszelle umfasst.

**9.** Verfahren nach einem der Ansprüche 1 bis 8, wobei das Expressionsniveau unter Verwendung eines Mikroarrays, eines Nukleinsäureamplifikationsverfahrens oder einer Northern-Blot-Analyse gemessen wird.

**10.** Verfahren nach Anspruch 9, wobei das Expressionsniveau unter Verwendung eines Mikroarrays gemessen wird.

**11.** Verfahren nach Anspruch 10, wobei der Mikroarray ein Mikroarray ist, der einen auf einem Grundmaterial angeordneten in der Tabelle A gezeigten Sondensatz aufweist.

**12.** Verfahren nach Anspruch 9, wobei das Expressionsniveau unter Verwendung eines Nukleinsäureamplifikationsverfahrens gemessen wird.

**13.** Verfahren nach Anspruch 12, wobei das Nukleinsäureamplifikationsverfahren eine quantitative RT-PCR oder quantitative PCR ist.

**14.** Computerprogramm, das einen Computer veranlasst, als Steuerteil zum Analysieren eines Expressionsniveaus jeder Nukleinsäure, die den nachfolgenden Sonden von (B1) bis (B23) entspricht, in einer Messprobe, die RNA umfasst, welche aus einem Präparat hergestellt wurde, das einem Subjekt entnommen wurde, und zum Bestimmen der Empfindlichkeit gegenüber einer neoadjuvanten Chemotherapie für Brustkrebs auf der Basis der erhaltenen Information des Expressionsniveaus zu funktionieren, wobei der Steuerteil konfiguriert ist, um Folgendes auszuführen:

eine Berechnung der Lösung D auf der Basis einer Diskriminante, die durch die Formel (I) dargestellt wird:

$$D = \sum_i (w_i \times y_i) - 3,327217 \qquad (I)$$

(wobei i eine Nummer darstellt, die jeder in Tabelle B dargestellten Nukleinsäure zugeordnet wird, so dass sie der SEQ ID NO des Zielpolynukleotids entspricht, $w_i$ für einen Wichtungsfaktor der in Tabelle B gezeigten Nukleinsäure mit der Nummer i steht, $y_i$ für ein standardisiertes Expressionsniveau der Nukleinsäure steht, wobei das standardisierte Expressionsniveau durch die Standardisierung eines Expressionsniveaus der Nukleinsäure gemäß der durch die Formel (II) dargestellten Formel erhalten wird:

$$y_i = x_i - m_i \qquad \text{(II)}$$

(wobei $x_i$ für ein Expressionsniveau der in Tabelle B gezeigten Nukleinsäure mit der Nummer i steht, und $m_i$ für einen Mittelwert des Expressionsniveaus der in Tabelle B gezeigten Nukleinsäure mit der Nummer i über die Präparate steht) und $\sum_i$ für eine Gesamtsumme über die entsprechenden Nukleinsäuren steht) und eine Empfindlichkeitsbestimmung, die eine Bestimmung der Empfindlichkeit gegenüber einer neoadjuvanten Chemotherapie für Brustkrebs durchführt, wenn die Lösung D der Diskriminante einen positiven Wert annimmt, und eine Bestimmung der Unempfindlichkeit gegenüber einer neoadjuvanten Chemotherapie für Brustkrebs durchführt, wenn die Lösung D 0 oder einen negativen Wert annimmt, wobei

(B1) eine Sonde ist, die auf ein Polynukleotid abzielt, das eine Nukleotidsequenz von SEQ ID NO: 1 aufweist,
(B2) eine Sonde ist, die auf ein Polynukleotid abzielt, das eine Nukleotidsequenz von SEQ ID NO: 2 aufweist,
(B3) eine Sonde ist, die auf ein Polynukleotid abzielt, das eine Nukleotidsequenz von SEQ ID NO: 3 aufweist,
(B4) eine Sonde ist, die auf ein Polynukleotid abzielt, das eine Nukleotidsequenz von SEQ ID NO: 4 aufweist,
(B5) eine Sonde ist, die auf ein Polynukleotid abzielt, das eine Nukleotidsequenz von SEQ ID NO: 5 aufweist,
(B6) eine Sonde ist, die auf ein Polynukleotid abzielt, das eine Nukleotidsequenz von SEQ ID NO: 6 aufweist,
(B7) eine Sonde ist, die auf ein Polynukleotid abzielt, das eine Nukleotidsequenz von SEQ ID NO: 7 aufweist,
(B8) eine Sonde ist, die auf ein Polynukleotid abzielt, das eine Nukleotidsequenz von SEQ ID NO: 8 aufweist,
(B9) eine Sonde ist, die auf ein Polynukleotid abzielt, das eine Nukleotidsequenz von SEQ ID NO: 9 aufweist,
(B10)eine Sonde ist, die auf ein Polynukleotid abzielt, das eine Nukleotidsequenz von SEQ ID NO: 10 aufweist,
(B11)eine Sonde ist, die auf ein Polynukleotid abzielt, das eine Nukleotidsequenz von SEQ ID NO: 11 aufweist,
(B12)eine Sonde ist, die auf ein Polynukleotid abzielt, das eine Nukleotidsequenz von SEQ ID NO: 12 aufweist,
(B13)eine Sonde ist, die auf ein Polynukleotid abzielt, das eine Nukleotidsequenz von SEQ ID NO: 13 aufweist,
(B14)eine Sonde ist, die auf ein Polynukleotid abzielt, das eine Nukleotidsequenz von SEQ ID NO: 14 aufweist,
(B15)eine Sonde ist, die auf ein Polynukleotid abzielt, das eine Nukleotidsequenz von SEQ ID NO: 15 aufweist,
(B16)eine Sonde ist, die auf ein Polynukleotid abzielt, das eine Nukleotidsequenz von SEQ ID NO: 16 aufweist,
(B17)eine Sonde ist, die auf ein Polynukleotid abzielt, das eine Nukleotidsequenz von SEQ ID NO: 17 aufweist,
(B18)eine Sonde ist, die auf ein Polynukleotid abzielt, das eine Nukleotidsequenz von SEQ ID NO: 18 aufweist,
(B19)eine Sonde ist, die auf ein Polynukleotid abzielt, das eine Nukleotidsequenz von SEQ ID NO: 19 aufweist,
(B20)eine Sonde ist, die auf ein Polynukleotid abzielt, das eine Nukleotidsequenz von SEQ ID NO: 20 aufweist,
(B21)eine Sonde ist, die auf ein Polynukleotid abzielt, das eine Nukleotidsequenz von SEQ ID NO: 21 aufweist,
(B22)eine Sonde ist, die auf ein Polynukleotid abzielt, das eine Nukleotidsequenz von SEQ ID NO: 22, aufweist, und
(B23)eine Sonde ist, die auf ein Polynukleotid abzielt, das eine Nukleotidsequenz von SEQ ID NO: 23 aufweist, wobei Tabelle B wie folgt ist:

TABELLE B

| Nummer i der Nukleinsäure | SEQ ID NO des Zielpolynukleotids | Wichtungsfaktor |
|---|---|---|
| 1 | 1 | 2,361579818 |

(fortgesetzt)

| Nummer i der Nukleinsäure | SEQ ID NO des Zielpolynukleotids | Wichtungsfaktor |
|---|---|---|
| 2 | 2 | 0,527535817 |
| 3 | 3 | 0,53572137 |
| 4 | 4 | 1,296736029 |
| 5 | 5 | 0,437766376 |
| 6 | 6 | 1,09614395 |
| 7 | 7 | 1,154132786 |
| 8 | 8 | -0,997955474 |
| 9 | 9 | -0,84645569 |
| 10 | 10 | 0,703499669 |
| 11 | 11 | 1,262066324 |
| 12 | 12 | 0,481709248 |
| 13 | 13 | 0,784677171 |
| 14 | 14 | -1,056130291 |
| 15 | 15 | -0,90152985 |
| 16 | 16 | 0,941011796 |
| 17 | 17 | -0,580145259 |
| 18 | 18 | 0,797198448 |
| 19 | 19 | -0,963860205 |
| 20 | 20 | -1,352304026 |
| 21 | 21 | -1,231365097 |
| 22 | 22 | -0,637818166 |
| 23 | 23 | 0,449217729 |

**Revendications**

**1.** Procédé de détermination de la sensibilité à une chimiothérapie du cancer du sein par néo-adjuvant, comprenant les étapes consistant à :

(1) préparer un échantillon de mesure comprenant de l'ARN qui provient d'un spécimen prélevé à partir d'un sujet,
(2) mesurer un niveau d'expression de chacun des gènes de (A1) à (A19) ci-dessous en utilisant un échantillon de mesure obtenu à l'étape (1),
(3) analyser un niveau d'expression de chaque gène mesuré à l'étape (2), et
(4) déterminer la sensibilité à une chimiothérapie du cancer du sein par néo-adjuvant sur la base d'un résultat d'analyse obtenu à l'étape (3), dans lequel :

(A1) est le gène du membre 9 de la famille des domaines de recrutement des caspases (CARD9) humaines,
(A2) est le gène de l'indoléamine-2,3-dioxygénase 1 (IDO1) humaine,
(A3) est le gène du ligand 9 d'une chimiokine à (motif C-X-C) (CXCL9) humaine,
(A4) est le gène de la purine nucléoside phosphorylase (PNP),
(A5) est le gène du ligand 11 d'une chimiokine à (motif C-X-C) (CXCL11) humaine,
(A6) est le gène de la protéine de liaison à la CCAAT/amplificateur (CEBPB) humaine,
(A7) est le gène d'un CD83 humain,
(A8) est le gène du transducteur du signal de l'interleukine 6 (IL6ST) humaine,
(A9) est le gène du récepteur 1 d'une chimiokine (C-X3-C) (CX3CR1) humaine,

(A10) est le gène du CD1D humain,
(A11) est le gène de la cathepsine C (CTSC) humaine,
(A12) est le gène du ligand 10 d'une chimiokine à (motif C-X-C) (CXCL10) humaine,
(A13) est le gène de l'isotype du locus génétique G1 d'une chaîne lourde d'immunoglobuline (IGHG1) humaine,
(A14) est le gène de l'homéo-boîte 1 de liaison à la boite E en doigt de zinc (ZEB1) humaine,
(A15) est le gène du facteur A de croissance endothéliale vasculaire (VEGFA) humaine,
(A16) est le gène du précurseur de la sémaphorine 3C (SEMA3C) humaine,
(A17) est le gène du récepteur au complément (CR2) humain,
(A18) est le gène de l'HFE humaine, et
(A19) est le gène de l'EDA humaine.

**2.** Procédé selon la revendication 1, dans laquelle
le gène (A1) comprend un acide nucléique détecté par une sonde ciblant un polynucléotide, qui a une séquence nucléotidique de SEQ ID n° : 1,
le gène (A2) comprend un acide nucléique détecté par une sonde ciblant un polynucléotide, qui a une séquence nucléotidique de SEQ ID n° : 2,
le gène (A3) comprend un acide nucléique détecté par une sonde ciblant un polynucléotide, qui a une séquence nucléotidique de SEQ ID n° : 3,
le gène (A4) comprend un acide nucléique détecté par une sonde ciblant un polynucléotide, qui a une séquence nucléotidique de SEQ ID n° : 4,
le gène (A5) comprend un acide nucléique détecté par une sonde ciblant un polynucléotide, qui a une séquence nucléotidique de SEQ ID n° : 5 ou SEQ ID n° : 23,
le gène (A6) comprend un acide nucléique détecté par une sonde ciblant un polynucléotide, qui a une séquence nucléotidique de SEQ ID n° : 6,
le gène (A7) comprend un acide nucléique détecté par une sonde ciblant un polynucléotide, qui a une séquence nucléotidique de SEQ ID n° : 7,
le gène (A8) comprend un acide nucléique détecté par une sonde ciblant un polynucléotide, qui a une séquence nucléotidique de SEQ ID n° : 8, SEQ ID n° : 14 ou SEQ ID n° : 19,
le gène (A9) comprend un acide nucléique détecté par une sonde ciblant un polynucléotide, qui a une séquence nucléotidique de SEQ ID n° : 9,
le gène (A10) comprend un acide nucléique détecté par une sonde ciblant un polynucléotide, qui a une séquence nucléotidique de SEQ ID n° : 10,
le gène (A11) comprend un acide nucléique détecté par une sonde ciblant un polynucléotide, qui a une séquence nucléotidique de SEQ ID n° : 11,
le gène (A12) comprend un acide nucléique détecté par une sonde ciblant un polynucléotide, qui a une séquence nucléotidique de SEQ ID n° : 12,
le gène (A13) comprend un acide nucléique détecté par une sonde ciblant un polynucléotide, qui a une séquence nucléotidique de SEQ ID n° : 13,
le gène (A14) comprend un acide nucléique détecté par une sonde ciblant un polynucléotide, qui a une séquence nucléotidique de SEQ ID n° : 15 ou SEQ ID n° : 22,
le gène (A15) comprend un acide nucléique détecté par une sonde ciblant un polynucléotide, qui a une séquence nucléotidique de SEQ ID n° : 16,
le gène (A16) comprend un acide nucléique détecté par une sonde ciblant un polynucléotide, qui a une séquence nucléotidique de SEQ ID n° : 17,
le gène (A17) comprend un acide nucléique détecté par une sonde ciblant un polynucléotide, qui a une séquence nucléotidique de SEQ ID n° : 18,
le gène (A18) comprend un acide nucléique détecté par une sonde ciblant un polynucléotide, qui a une séquence nucléotidique de SEQ ID n° : 20, et
le gène (A19) comprend un acide nucléique détecté par une sonde ciblant un polynucléotide, qui a une séquence nucléotidique de SEQ ID n° : 21.

**3.** Procédé selon la revendication 2, dans laquelle le niveau d'expression est mesuré en utilisant un ensemble de sondes présenté dans le Tableau A.

TABLEAU A

| SEQ ID n° du polynucléotide ciblé | SEQ ID n° de la sonde |
|---|---|
| 1 | 24~34 |
| 2 | 35~45 |
| 3 | 46~56 |
| 4 | 57~67 |
| 5 | 68~78 |
| 6 | 79~89 |
| 7 | 90~100 |
| 8 | 101~111 |
| 9 | 112~122 |
| 10 | 123~133 |
| 11 | 134~144 |
| 12 | 145~155 |
| 13 | 156~166 |
| 14 | 167~177 |
| 15 | 178~188 |
| 16 | 189~199 |
| 17 | 200~210 |
| 18 | 211~221 |
| 19 | 222~232 |
| 20 | 233~243 |
| 21 | 244~254 |
| 22 | 255~265 |
| 23 | 266~276 |

**4.** Procédé selon la revendication 2 ou 3, dans laquelle l'analyse du niveau d'expression est conduite en utilisant une technique de classification, une technique de scores ou une technique d'analyse de groupes.

**5.** Procédé selon la revendication 4, dans laquelle l'analyse du niveau d'expression est conduite en utilisant une technique de classification.

**6.** Procédé selon la revendication 4, dans laquelle la technique de classification est une analyse discriminante linéaire par diagonale.

**7.** Procédé selon la revendication 5, dans laquelle l'analyse du niveau d'expression, solution D du discriminant, est déterminée en utilisant le niveau d'expression et un discriminant représenté par la formule (I) :

$$D = \Sigma_i (w_i \text{ x } y_i) - 3{,}327217 \qquad (I)$$

(dans laquelle i représente un nombre attribué à chaque acide nucléique présenté dans le Tableau B, de sorte à correspondre à une SEQ ID n° du polynucléotide ciblé, $w_i$ représente un facteur pondéral d'acide nucléique de nombre i présenté dans le Tableau B, $y_i$ représente un niveau d'expression standardisé d'un acide nucléique, le niveau d'expression standardisé étant obtenu en standardisant un niveau d'expression d'un acide nucléique selon la formule représentée par la formule (II) :

$$y_i = x_i - m_i \qquad (II)$$

(dans laquelle $x_i$ représente un niveau d'expression d'un acide nucléique de nombre i présenté dans le Tableau B, et $m_i$ représente une valeur moyenne du niveau d'expression d'un acide nucléique de nombre i présenté dans le Tableau B sur les spécimens), et $\sum_i$ représente un total des sommes sur les acides nucléiques respectifs), et dans lequel une détermination du fait d'être sensible à une chimiothérapie du cancer du sein par néo-adjuvant est réalisée quand la solution D du discriminant est une valeur positive, et une détermination du fait d'être insensible à une chimiothérapie du cancer du sein par néo-adjuvant est réalisée quand la solution D est 0 ou une valeur négative dans l'étape (4).

TABLEAU B

| Nombre i d'acides nucléiques | SEQ ID n° du polynucleotide ciblé | Facteur pondéral |
|---|---|---|
| 1 | 1 | 2,361579818 |
| 2 | 2 | 0,527535817 |
| 3 | 3 | 0,53572137 |
| 4 | 4 | 1,296736029 |
| 5 | 5 | 0,437766376 |
| 6 | 6 | 1,09614395 |
| 7 | 7 | 1,154132786 |
| 8 | 8 | -0,997955474 |
| 9 | 9 | -0,84645569 |
| 10 | 10 | 0,703499669 |
| 11 | 11 | 1,262066324 |
| 12 | 12 | 0,481709248 |
| 13 | 13 | 0,784677171 |
| 14 | 14 | -1,056130291 |
| 15 | 15 | -0,90152985 |
| 16 | 16 | 0,941011796 |
| 17 | 17 | -0,580145259 |
| 18 | 18 | 0,797198448 |
| 19 | 19 | -0,963860205 |
| 20 | 20 | -1,352304026 |
| 21 | 21 | -1,231365097 |
| 22 | 22 | -0,637818166 |
| 23 | 23 | 0,449217729 |

**8.** Procédé selon l'une quelconque des revendications 1 à 7, dans laquelle le spécimen comprend une cellule de cancer du sein.

**9.** Procédé selon l'une quelconque des revendications 1 à 8, dans laquelle le niveau d'expression est mesuré en utilisant un micro-arrangement, un procédé par amplification d'acide nucléique ou une analyse par transfert de Northern.

**10.** Procédé selon la revendication 9, dans laquelle le niveau d'expression est mesuré en utilisant un micro-arrangement.

**11.** Procédé selon la revendication 10, dans laquelle le micro-arrangement est un micro-arrangement ayant un ensemble de sondes présenté dans le Tableau A, arrangé sur un matériau de base.

**12.** Procédé selon la revendication 9, dans laquelle le niveau d'expression est mesuré en utilisant un procédé d'amplification d'acide nucléique.

**13.** Procédé selon la revendication 12, dans laquelle le procédé d'amplification d'acide nucléique est une ACP-TI quantitative ou une ACP quantitative.

**14.** Programme informatique faisant qu'un ordinateur fonctionne comme partie de contrôle pour analyser un niveau d'expression de chaque acide nucléique correspondant aux sondes (B1) à (B23) ci-dessous dans un échantillon de mesure, comprenant de l'ARN préparé à partir d'un spécimen prélevé à partir d'un sujet, et déterminer la sensibilité à une chimiothérapie du cancer du sein par néo-adjuvant sur la base de l'information obtenue du niveau d'expression, la partie de contrôle étant configurée pour exécuteur :

le calcul d'une solution D basée sur un discriminant représenté par la formule (I) :

$$D = \Sigma_i (w_i \text{ x } y_i) - 3,327217 \qquad (I)$$

(dans laquelle i représente un nombre attribué à chaque acide nucléique présenté dans le Tableau B, de sorte à correspondre à une SEQ ID n° du polynucléotide ciblé, wi représente un facteur pondéral d'acide nucléique de nombre i présenté dans le Tableau B, yi représente un niveau d'expression standardisé d'un acide nucléique, le niveau d'expression standardisé étant obtenu en standardisant un niveau d'expression d'un acide nucléique selon la formule représentée par la formule (II) :

$$y_i = x_i - m_i \qquad (II)$$

(dans laquelle xi représente un niveau d'expression d'un acide nucléique de nombre i présenté dans le Tableau B, et $m_i$ représente une valeur moyenne du niveau d'expression d'un acide nucléique de nombre i présenté dans le Tableau B sur les spécimens), et $\Sigma_i$ représente un total des sommes sur les acides nucléiques respectifs), et

une détermination de la sensibilité, qui effectue une détermination du fait d'être sensible à une chimiothérapie du cancer du sein par néo-adjuvant quand la solution D du discriminant est une valeur positive, et effectue une détermination du fait d'être insensible à une chimiothérapie du cancer du sein par néo-adjuvant quand la solution D est 0 ou une valeur négative, dans laquelle

(B1) est une sonde qui cible un polynucléotide ayant une séquence nucléotidique de SEQ ID n° : 1,
(B2) est une sonde qui cible un polynucléotide ayant une séquence nucléotidique de SEQ ID n° : 2,
(B3) est une sonde qui cible un polynucléotide ayant une séquence nucléotidique de SEQ ID n° : 3,
(B4) est une sonde qui cible un polynucléotide ayant une séquence nucléotidique de SEQ ID n° : 4,
(B5) est une sonde qui cible un polynucléotide ayant une séquence nucléotidique de SEQ ID n° : 5,
(B6) est une sonde qui cible un polynucléotide ayant une séquence nucléotidique de SEQ ID n° : 6,
(B7) est une sonde qui cible un polynucléotide ayant une séquence nucléotidique de SEQ ID n° : 7,
(B8) est une sonde qui cible un polynucléotide ayant une séquence nucléotidique de SEQ ID n° : 8,
(B9) est une sonde qui cible un polynucléotide ayant une séquence nucléotidique de SEQ ID n° : 9,
(B10) est une sonde qui cible un polynucléotide ayant une séquence nucléotidique de SEQ ID n° : 10,
(B11) est une sonde qui cible un polynucléotide ayant une séquence nucléotidique de SEQ ID n° : 11,
(B12) est une sonde qui cible un polynucléotide ayant une séquence nucléotidique de SEQ ID n° : 12,
(B13) est une sonde qui cible un polynucléotide ayant une séquence nucléotidique de SEQ ID n° : 13,
(B14) est une sonde qui cible un polynucléotide ayant une séquence nucléotidique de SEQ ID n° : 14,
(B15) est une sonde qui cible un polynucléotide ayant une séquence nucléotidique de SEQ ID n° : 15,
(B16) est une sonde qui cible un polynucléotide ayant une séquence nucléotidique de SEQ ID n° : 16,
(B17) est une sonde qui cible un polynucléotide ayant une séquence nucléotidique de SEQ ID n° : 17,
(B18) est une sonde qui cible un polynucléotide ayant une séquence nucléotidique de SEQ ID n° : 18,
(B19) est une sonde qui cible un polynucléotide ayant une séquence nucléotidique de SEQ ID n° : 19,

(B20) est une sonde qui cible un polynucléotide ayant une séquence nucléotidique de SEQ ID n° : 20, (B21) est une sonde qui cible un polynucléotide ayant une séquence nucléotidique de SEQ ID n° : 21, (B22) est une sonde qui cible un polynucléotide ayant une séquence nucléotidique de SEQ ID n° : 22, et (B23) est une sonde qui cible un polynucléotide ayant une séquence nucléotidique de SEQ ID n° : 23, dans laquelle le Tableau B est :

TABLEAU B

| Nombre i d'acides nucléiques | SEQ ID n° du polynucleotide ciblé | Facteur pondéral |
|---|---|---|
| 1 | 1 | 2,361579818 |
| 2 | 2 | 0,527535817 |
| 3 | 3 | 0,53572137 |
| 4 | 4 | 1,296736029 |
| 5 | 5 | 0,437766376 |
| 6 | 6 | 1,09614395 |
| 7 | 7 | 1,154132786 |
| 8 | 8 | -0,997955474 |
| 9 | 9 | -0,84645569 |
| 10 | 10 | 0,703499669 |
| 11 | 11 | 1,262066324 |
| 12 | 12 | 0,481709248 |
| 13 | 13 | 0,784677171 |
| 14 | 14 | -1,056130291 |
| 15 | 15 | -0,90152985 |
| 16 | 16 | 0,941011796 |
| 17 | 17 | -0,580145259 |
| 18 | 18 | 0,797198448 |
| 19 | 19 | -0,963860205 |
| 20 | 20 | -1,352304026 |
| 21 | 21 | -1,231365097 |
| 22 | 22 | -0,637818166 |
| 23 | 23 | 0,449217729 |

FIG. 1

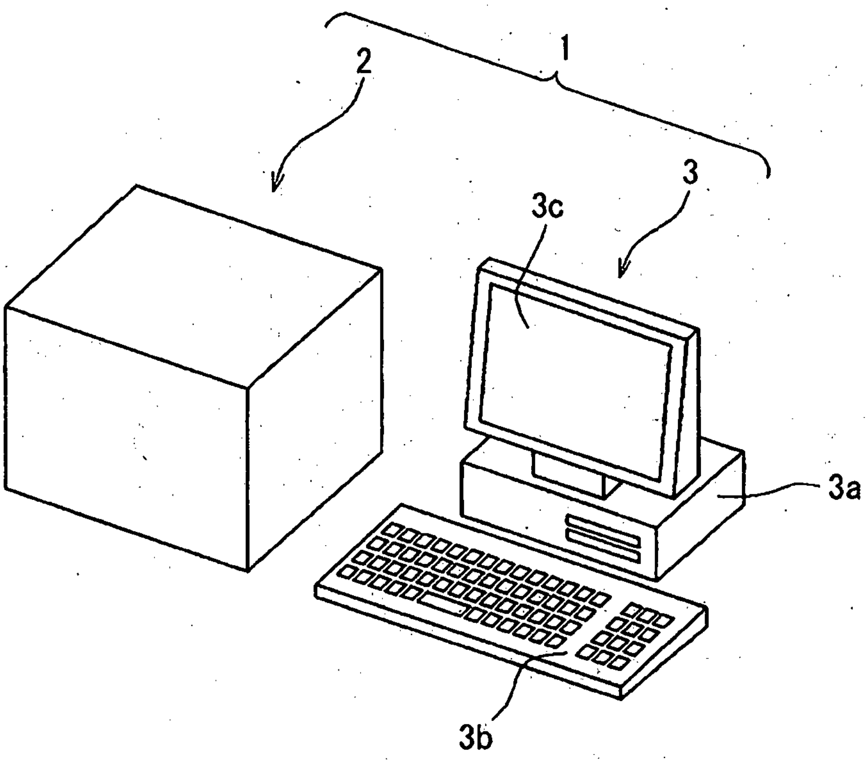

FIG. 2

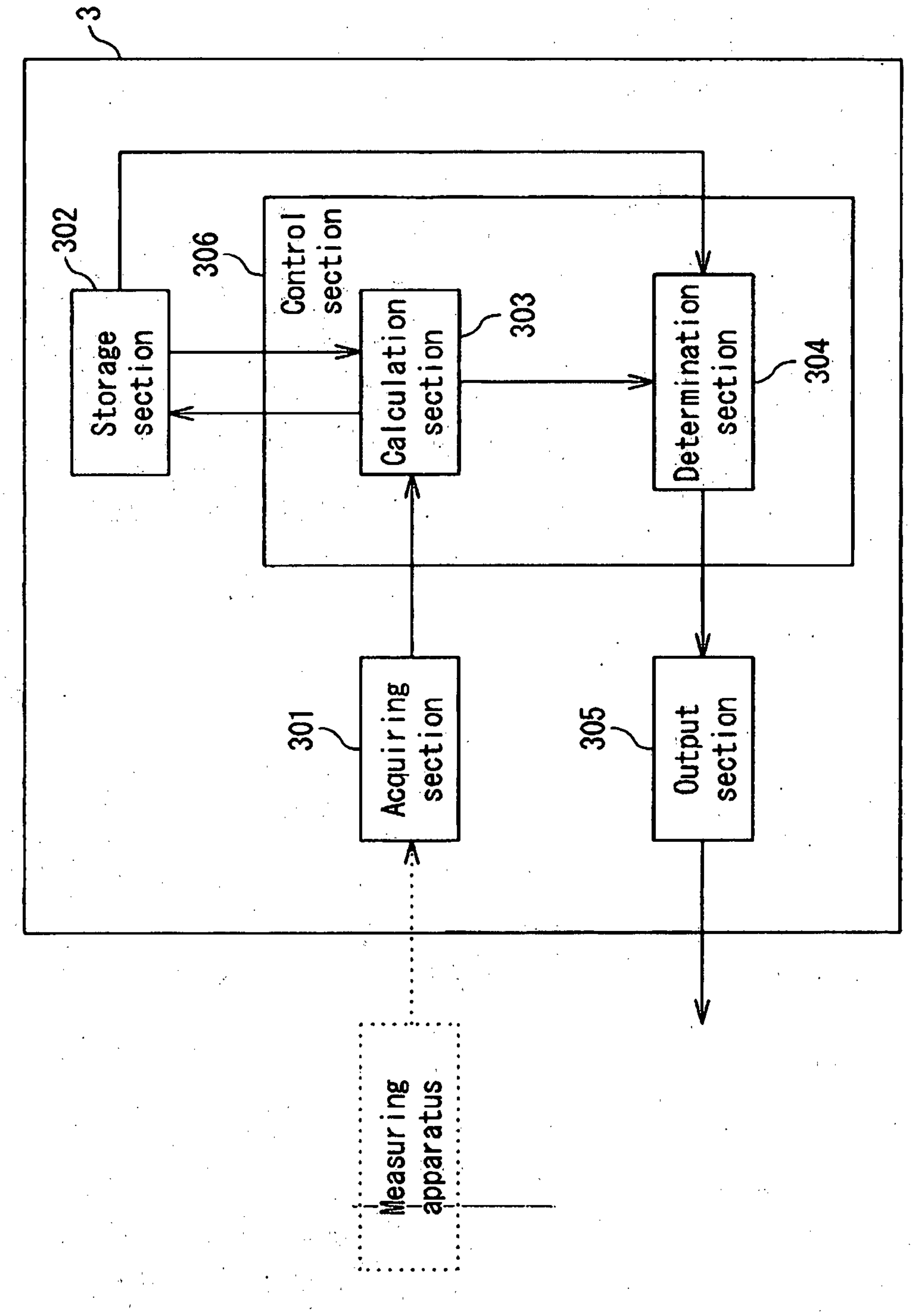
3
302
Storage section
306
Control section
Calculation section
303
Determination section
304
301
Acquiring section
305
Output section
Measuring apparatus

FIG. 3

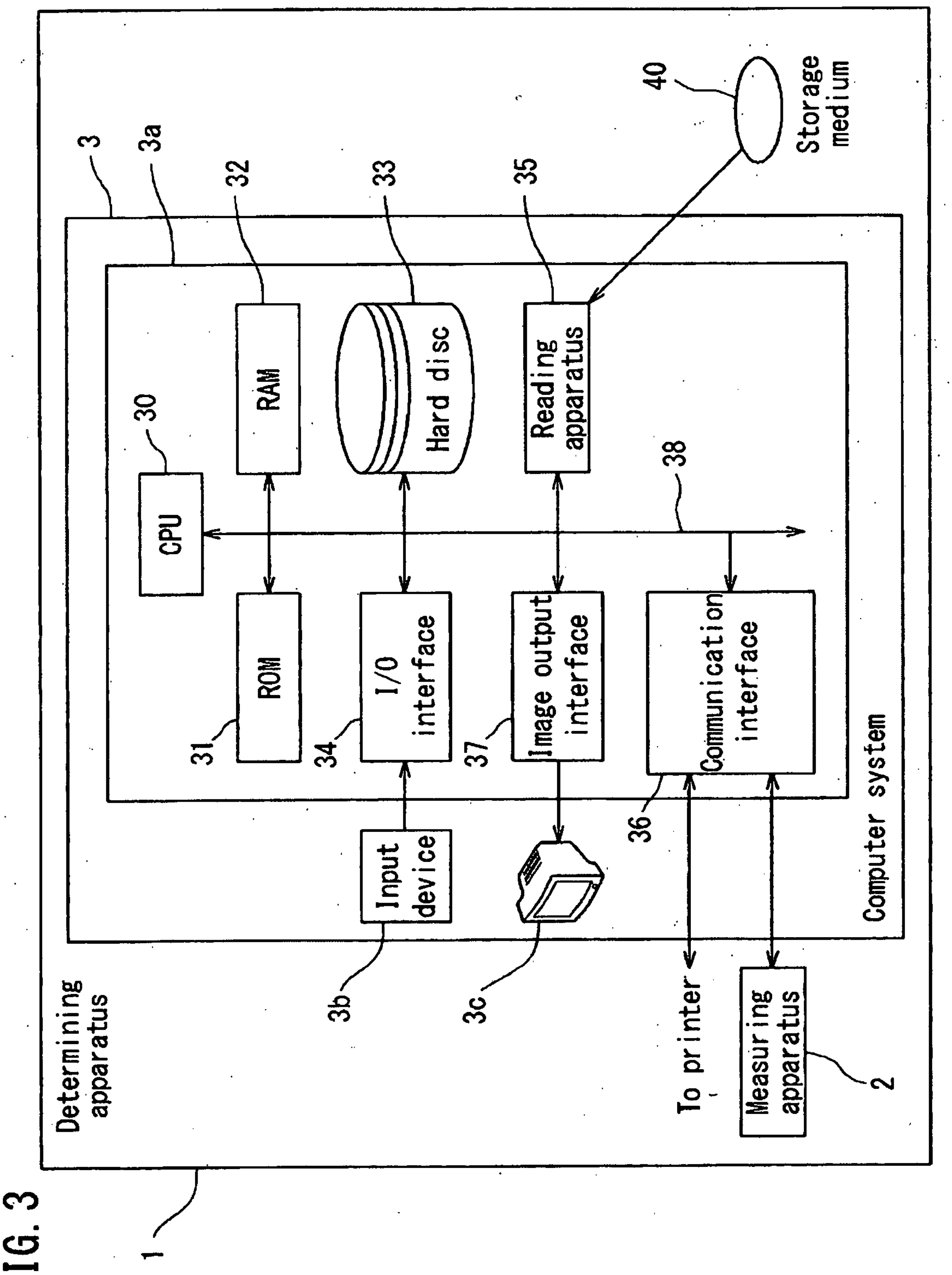
1
Determining apparatus
3
3a
Computer system
CPU
30
RAM
32
ROM
31
Hard disc
33
I/O interface
34
Reading apparatus
35
Image output interface
37
Communication interface
36
38
40
Storage medium
Input device
3b
3c
To printer
Measuring apparatus
2

FIG. 4

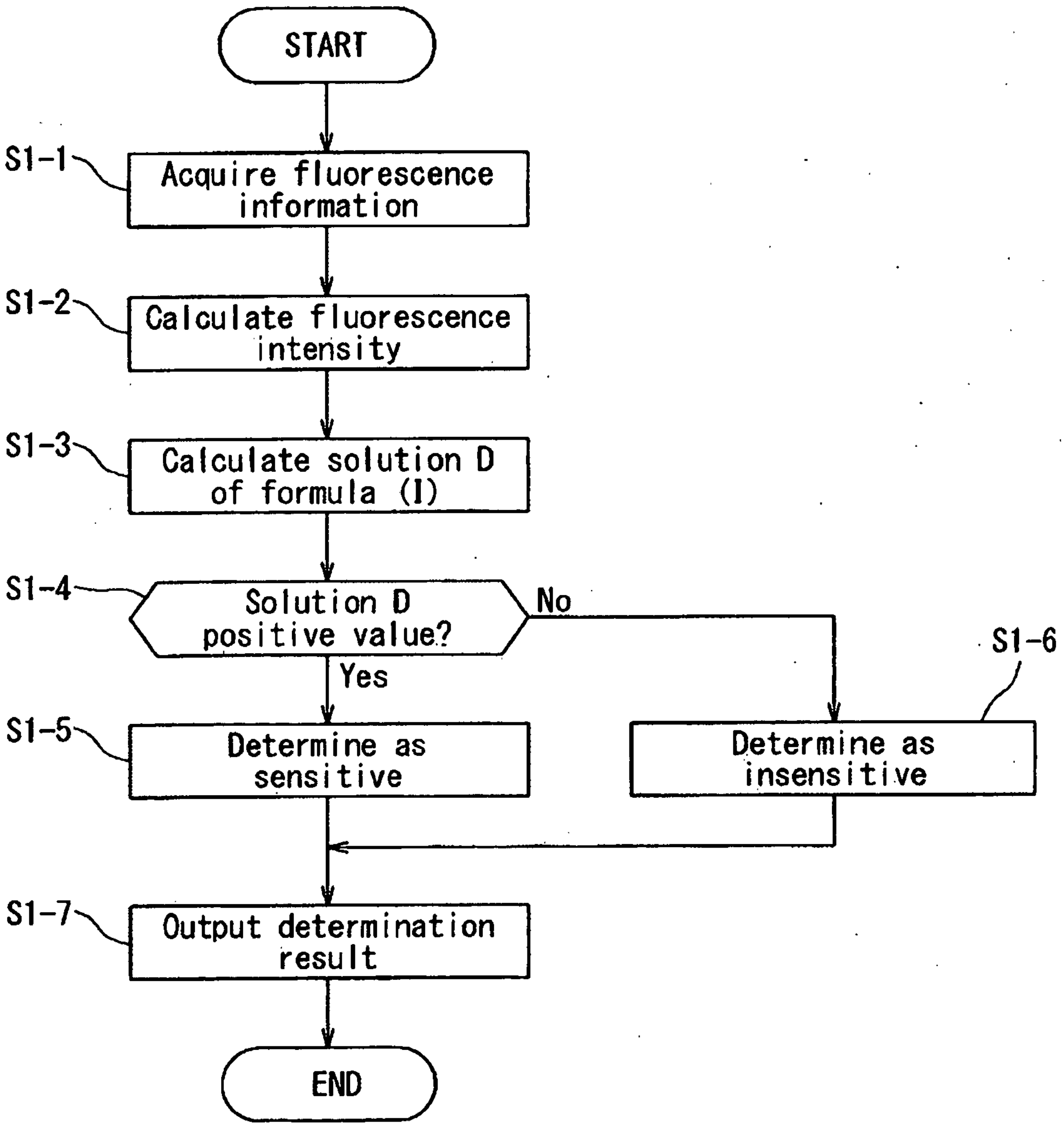
START
S1-1
Acquire fluorescence information
S1-2
Calculate fluorescence intensity
S1-3
Calculate solution D of formula (I)
S1-4
Solution D positive value?
No
Yes
S1-5
Determine as sensitive
S1-6
Determine as insensitive
S1-7
Output determination result
END

FIG. 5

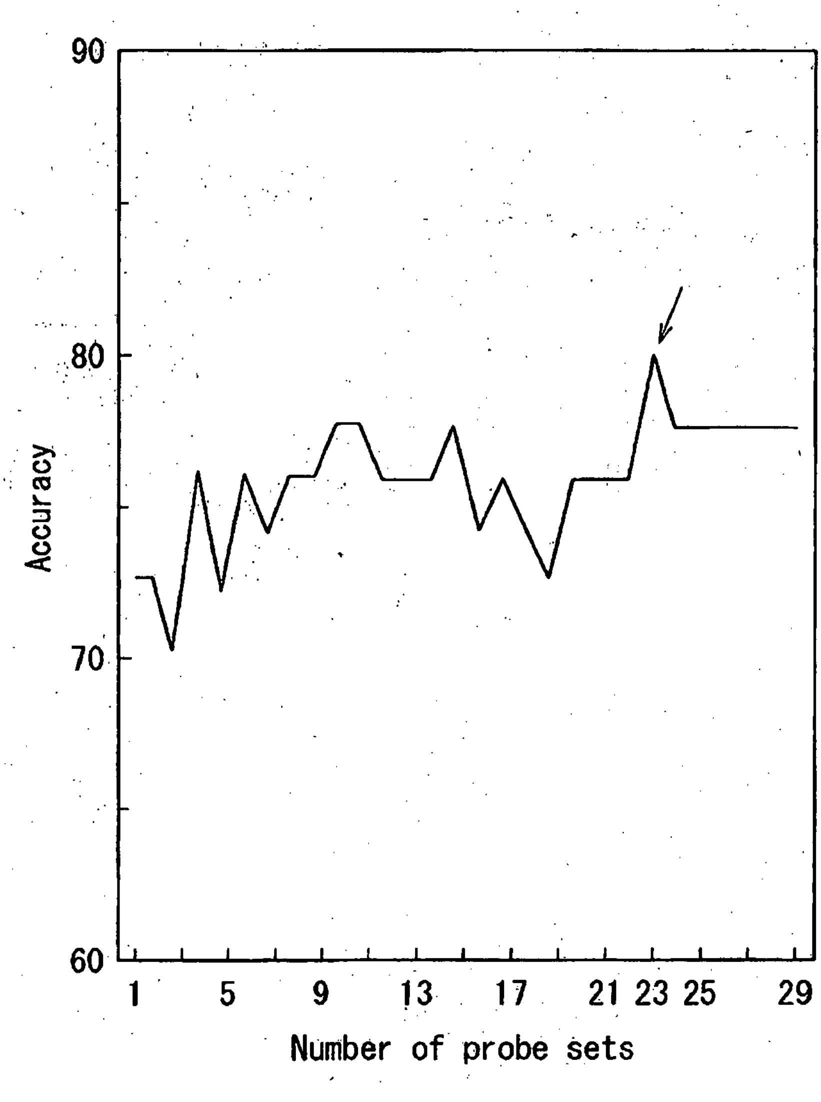

FIG. 6

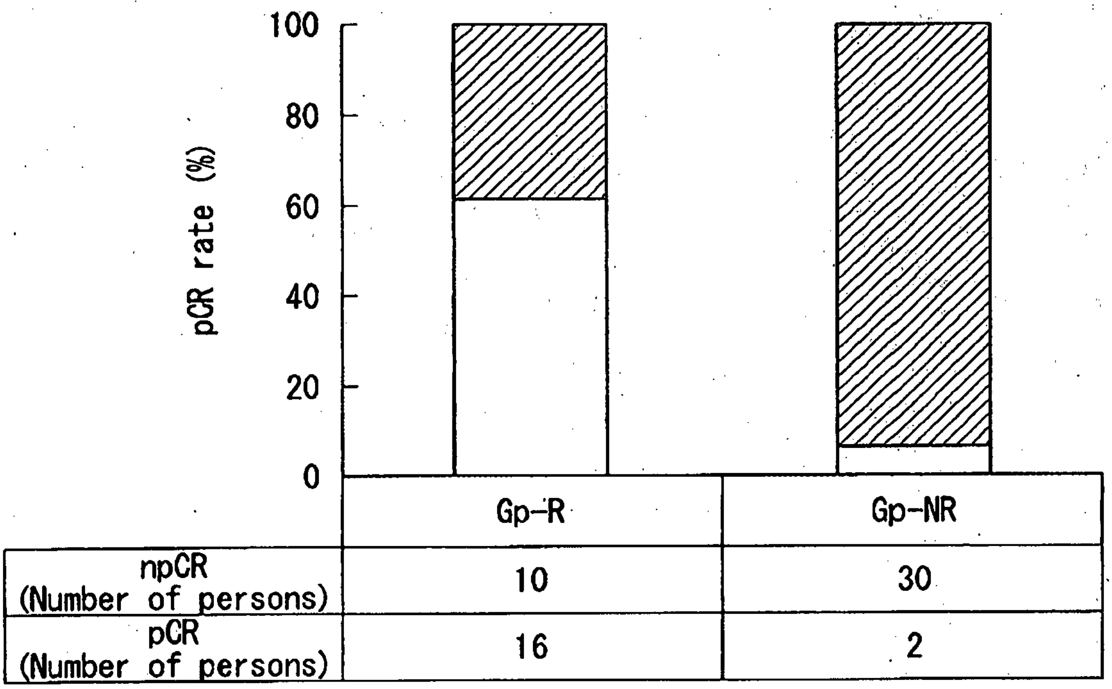


| | Gp-R | Gp-NR |
|---|---|---|
| npCR (Number of persons) | 10 | 30 |
| pCR (Number of persons) | 16 | 2 |

FIG. 7

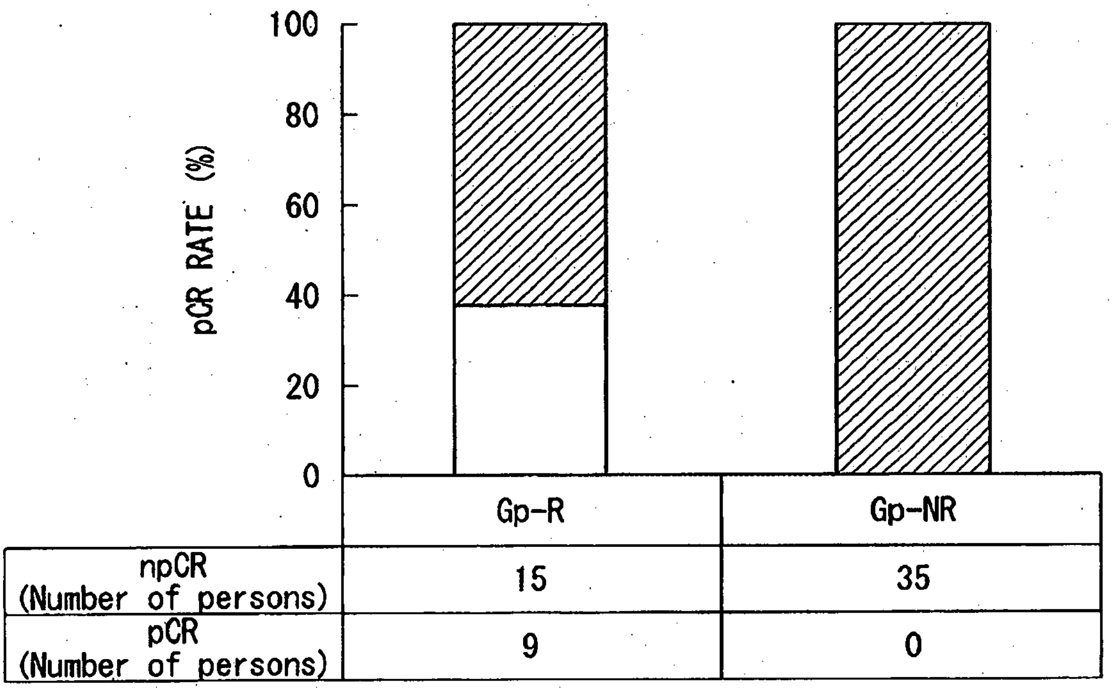


| | Gp-R | Gp-NR |
|---|---|---|
| npCR (Number of persons) | 15 | 35 |
| pCR (Number of persons) | 9 | 0 |

FIG. 8

| Subject group number | Classification | pCR (Number of persons) | Total of Gp-R (Number of persons) | pCR (Number of persons) | Total of Gp-NR (Number of persons) | Odds ratio | Odds ratio | 95% confidence interval |
|---|---|---|---|---|---|---|---|---|
| 1-1 | GSE16446 | 11 | 38 | 5 | 76 | 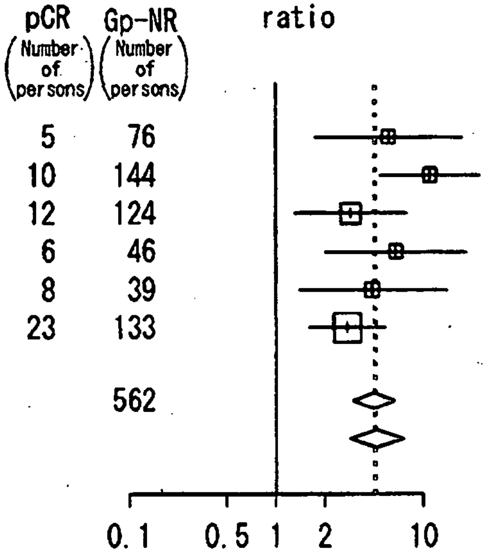 | 5.79 | [1.84;18.20] |
| 1-2 | GSE20194 | 35 | 77 | 10 | 144 | | 11.17 | [5.10;24.45] |
| 1-3 | GSE20271 | 14 | 54 | 12 | 124 | | 3.27 | [1.39;7.65] |
| 1-4 | GSE22093 | 18 | 36 | 6 | 46 | | 6.67 | [2.27;19.60] |
| 1-5 | GSE23988 | 12 | 22 | 8 | 39 | | 4.65 | [1.48;14.60] |
| 1-6 | GSE41998 | 44 | 112 | 23 | 133 | | 3.09 | [1.72;5.57] |
| Fixed effect model | | | 339 | | 562 | | 4.83 | [3.43;6.81] |
| Random effect model | | | | | | | 5.01 | [3.18;7.88] |

FIG. 9

| Subject group number | Classification | pCR (Number of persons) | Total of Gp-R (Number of persons) | pCR (Number of persons) | Total of Gp-NR (Number of persons) | Odds ratio | Odds ratio | 95% confidence interval |
|---|---|---|---|---|---|---|---|---|
| 2-1 | $ER^{+}$, $HER2^{-}$ | 12 | 45 | 26 | 330 | 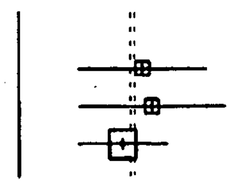 | 4.25 | [1.96;9.21] |
| 2-2 | $ER^{+-}$, $HER2^{+}$ | 21 | 40 | 14 | 74 | | 4.74 | [2.02;11.09] |
| 2-3 | $ER^{-}$, $HER2^{-}$ | 99 | 243 | 24 | 143 | | 3.41 | [2.05;5.66] |

FIG. 10

| Subject group number | Classification | pCR (Number of persons) | Total of Gp-R (Number of persons) | pCR (Number of persons) | Total of Gp-NR (Number of persons) | Odds ratio | Odds ratio | 95% confidence interval |
|---|---|---|---|---|---|---|---|---|
| 3-1 | Epirubicin | 11 | 38 | 5 | 76 | 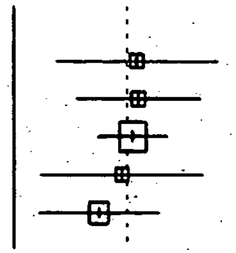 | 5. 79 | [1. 84;18. 20] |
| 3-2 | FAC or FEC | 22 | 62 | 9 | 107 | | 5. 99 | [2. 54;14. 13] |
| 3-3 | A. paclitaxel | 66 | 156 | 32 | 273 | | 5. 52 | [3. 39;8. 99] |
| 3-4 | A. docetaxel | 12 | 22 | 8 | 39 | | 4. 65 | [1. 48;14. 60] |
| 3-5 | A. Ixabepilone | 23 | 61 | 10 | 67 | | 3. 45 | [1. 48;8. 06] |

FIG. 11

| Subject group number | Classification | pCR (Number of persons) | Total of pCR (Number of persons) | pCR (Number of persons) | Total of npCR (Number of persons) | Odds ratio | 95% confidence interval |
|---|---|---|---|---|---|---|---|
| 1-1 | GSE16446 | 7 | 67 | 9 | 47 | 0.49 | [0.17; 1.43] |
| 1-2 | GSE20194 | 41 | 127 | 4 | 94 | 10.73 | [3.69; 31.22] |
| 1-3 | GSE20271 | 22 | 103 | 4 | 75 | 4.82 | [1.59; 14.66] |
| 1-4 | GSE22093 | 19 | 45 | 5 | 37 | 4.68 | [1.54; 14.23] |
| 1-5 | GSE23988 | 14 | 36 | 6 | 25 | 2.02 | [0.65; 6.28] |
| 1-6 | GSE41998 | 55 | 144 | 12 | 101 | 4.58 | [2.30; 9.14] |
| Fixed effect model | | | 522 | | 379 | 3.67 | [2.52; 5.36] |
| Random effect model | | | | | | 3.25 | [1.44; 7.31] |

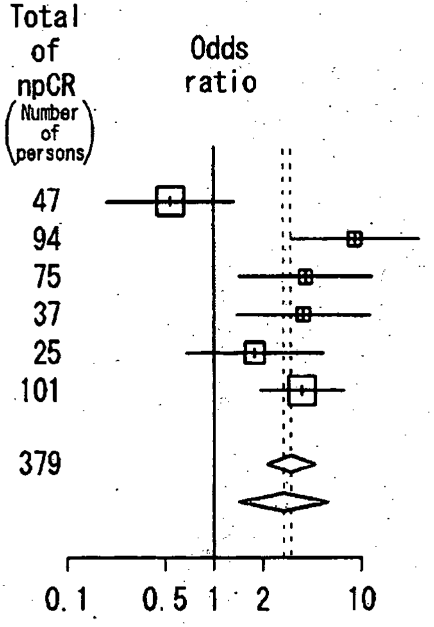

FIG. 12

| Subject group number | Classification | pCR (Number of persons) | Total of pCR (Number of persons) | pCR (Number of persons) | Total of npCR (Number of persons) | Odds ratio | Odds ratio | 95% confidence interval |
|---|---|---|---|---|---|---|---|---|
| 2-1 | $ER^{+}$, $HER2^{-}$ | 14 | 98 | 24 | 277 | 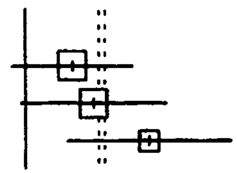 | 1.76 | [0.87;3.55] |
| 2-2 | $ER^{+-}$, $HER2^{+}$ | 24 | 63 | 11 | 51 | | 2.24 | [0.97;5.18] |
| 2-3 | $ER^{-}$, $HER2^{-}$ | 118 | 340 | 5 | 46 | | 4.36 | [1.68;11.33] |

FIG. 13

| Subject group number | Classification | pCR (Number of persons) | Total of pCR (Number of persons) | pCR (Number of persons) | Total of npCR (Number of persons) | Odds ratio | Odds ratio | 95% confidence interval |
|---|---|---|---|---|---|---|---|---|
| 3-1 | Epirubicin | 7 | 67 | 9 | 47 | 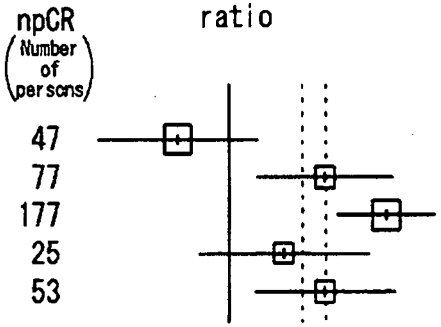 | 0.49 | [0.17;1.43] |
| 3-2 | FAC or FEC | 24 | 92 | 7 | 77 | | 3.53 | [1.43;8.73] |
| 3-3 | A. paclitaxel | 87 | 252 | 11 | 177 | | 7.96 | [4.10;15.44] |
| 3-4 | A. docetaxel | 14 | 36 | 6 | 25 | | 2.02 | [0.65;6.28] |
| 3-5 | A. Ixabepilone | 26 | 75 | 7 | 53 | | 3.49 | [1.38;8.81] |

# FIG. 14

| Subject group number | Classification | Higher than mean value: pCR (Number of persons) | Higher than mean value: Total (Number of persons) | Not Higher than mean value: pCR (Number of persons) | Not Higher than mean value: Total (Number of persons) | Odds ratio | 95% confidence interval |
|---|---|---|---|---|---|---|---|
| 1-1 | GSE16446 | 10 | 55 | 6 | 59 | 1.96 | [0.66;5.82] |
| 1-2 | GSE20194 | 26 | 88 | 19 | 133 | 2.52 | [1.29;4.90] |
| 1-3 | GSE20271 | 19 | 74 | 7 | 104 | 4.79 | [1.89;12.10] |
| 1-4 | GSE22093 | 14 | 31 | 10 | 51 | 3.38 | [1.26;9.08] |
| 1-5 | GSE23988 | 9 | 23 | 11 | 38 | 1.58 | [0.53;4.70] |
| 1-6 | GSE41998 | 40 | 106 | 27 | 139 | 2.51 | [1.41;4.47] |
| Fixed effect model | | | 377 | | 524 | 2.65 | [1.91;3.69] |
| Random effect model | | | | | | 2.64 | [1.90;3.68] |

Odds ratio

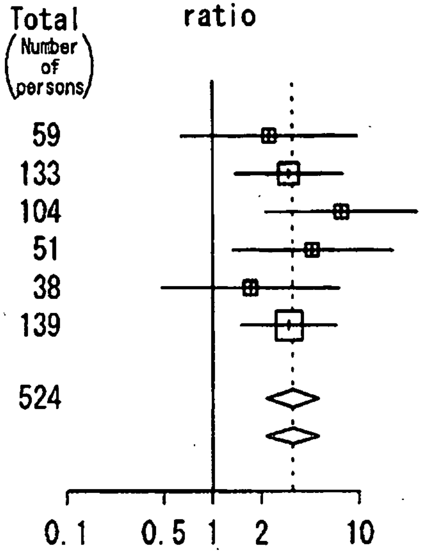

FIG. 15

| Subject group number | Classification | Higher than mean value: pCR (Number of persons) | Higher than mean value: Total (Number of persons) | Not Higher than mean value: pCR (Number of persons) | Not Higher than mean value: Total (Number of persons) | Odds ratio | Odds ratio | 95% confidence interval |
|---|---|---|---|---|---|---|---|---|
| 2-1 | $ER^+$, $HER2^-$ | 18 | 116 | 20 | 259 | 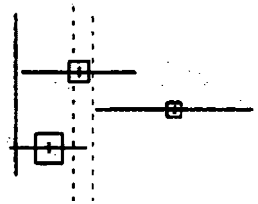 | 2.19 | [1.11;4.33] |
| 2-2 | $ER^{+-}$, $HER2^+$ | 28 | 57 | 7 | 57 | | 6.90 | [2.68;17.76] |
| 2-3 | $ER^-$, $HER2^-$ | 70 | 193 | 53 | 193 | | 1.50 | [0.98;2.31] |

FIG. 16

| Subject group number | Classification | Higher than mean value: pCR (Number of persons) | Higher than mean value: Total (Number of persons) | Not Higher than mean value: pCR (Number of persons) | Not Higher than mean value: Total (Number of persons) | Odds ratio | 95% confidence interval |
|---|---|---|---|---|---|---|---|
| 3-1 | Epirubicin | 10 | 55 | 6 | 59 | 1.96 | [0.66;5.82] |
| 3-2 | FAC or FEC | 19 | 65 | 12 | 104 | 3.17 | [1.42;7.08] |
| 3-3 | A. paclitaxel | 56 | 175 | 42 | 254 | 2.38 | [1.50;3.76] |
| 3-4 | A. docetaxel | 9 | 23 | 11 | 38 | 1.58 | [0.53;4.70] |
| 3-5 | A. Ixabepilone | 24 | 59 | 9 | 69 | 4.57 | [1.91;10.94] |

Odds ratio

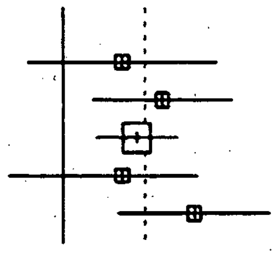

FIG. 17

| Subject group number | Classification | Higher than mean value pCR (Number of persons) | Higher than mean value Total (Number of persons) | Not Higher than mean value pCR (Number of persons) | Not Higher than mean value Total (Number of persons) | Odds ratio | 95% confidence interval |
|---|---|---|---|---|---|---|---|
| 1-1 | GSE16446 | 10 | 64 | 6 | 50 | 1. 36 | [0. 46; 4. 03] |
| 1-2 | GSE20194 | 27 | 85 | 18 | 136 | 3. 05 | [1. 56; 5. 99] |
| 1-3 | GSE20271 | 20 | 67 | 6 | 111 | 7. 45 | [2. 81; 19. 74] |
| 1-4 | GSE22093 | 15 | 33 | 9 | 49 | 3. 70 | [1. 37; 10. 03] |
| 1-5 | GSE23988 | 7 | 18 | 13 | 43 | 1. 47 | [0. 47; 4. 64] |
| 1-6 | GSE41998 | 44 | 119 | 23 | 126 | 2. 63 | [1. 46; 4. 72] |
| Fixed effect model | | | 386 | | 515 | 2. 89 | [2. 07; 4. 04] |
| Random effect model | | | | | | 2. 86 | [1. 88; 4. 36] |

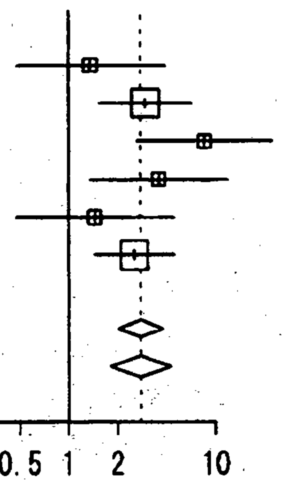

# FIG. 18

| Subject group number | Classification | Higher than mean value: pCR (Number of persons) | Higher than mean value: Total (Number of persons) | Not Higher than mean value: pCR (Number of persons) | Not Higher than mean value: Total (Number of persons) | Odds ratio | Odds ratio | 95% confidence interval |
|---|---|---|---|---|---|---|---|---|
| 2-1 | $ER^{+}$, $HER2^{-}$ | 18 | 114 | 20 | 261 | | 2.26 | [1.15;4.46] |
| 2-2 | $ER^{+-}$, $HER2^{+}$ | 27 | 64 | 8 | 50 | | 3.83 | [1.55;9.46] |
| 2-3 | $ER^{-}$, $HER2^{-}$ | 76 | 195 | 47 | 191 | | 1.96 | [1.26;3.03] |

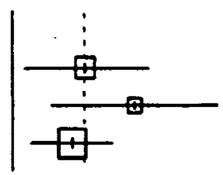

FIG. 19

| Subject group number | Classification | Higher than mean value pCR (Number of persons) | Higher than mean value Total (Number of persons) | Not Higher than mean value pCR (Number of persons) | Not Higher than mean value Total (Number of persons) | Odds ratio | Odds ratio | 95% confidence interval |
|---|---|---|---|---|---|---|---|---|
| 3-1 | Epirubicin | 10 | 64 | 6 | 50 | 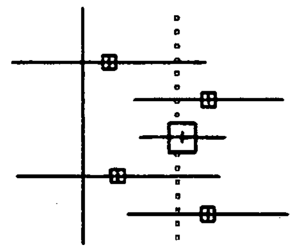 | 1.36 | [0.46;4.03] |
| 3-2 | FAC or FEC | 20 | 62 | 11 | 107 | | 4.16 | [1.83;9.44] |
| 3-3 | A. paclitaxel | 61 | 176 | 37 | 253 | | 3.10 | [1.94;4.94] |
| 3-4 | A. docetaxel | 7 | 18 | 13 | 43 | | 1.47 | [0.47;4.64] |
| 3-5 | A. Ixabepilone | 25 | 66 | 8 | 62 | | 4.12 | [1.68;10.06] |

# FIG. 20

| Subject group number | Classification | Higher than mean value: pCR (Number of persons) | Higher than mean value: Total (Number of persons) | Not Higher than mean value: pCR (Number of persons) | Not Higher than mean value: Total (Number of persons) | Odds ratio | 95% confidence interval |
|---|---|---|---|---|---|---|---|
| 1-1 | GSE16446 | 10 | 58 | 6 | 56 | 1.74 | [0.59;5.15] |
| 1-2 | GSE20194 | 23 | 92 | 22 | 129 | 1.62 | [0.84;3.13] |
| 1-3 | GSE20271 | 16 | 72 | 10 | 106 | 2.74 | [1.17;6.46] |
| 1-4 | GSE22093 | 15 | 33 | 9 | 49 | 3.70 | [1.37;10.03] |
| 1-5 | GSE23988 | 10 | 25 | 10 | 36 | 1.73 | [0.59;5.12] |
| 1-6 | GSE41998 | 40 | 115 | 27 | 130 | 2.03 | [1.15;3.60] |
| Fixed effect model | | | 395 | | 506 | 2.08 | [1.51;2.88] |
| Random effect model | | | | | | 2.08 | [1.50;2.88] |

Odds ratio

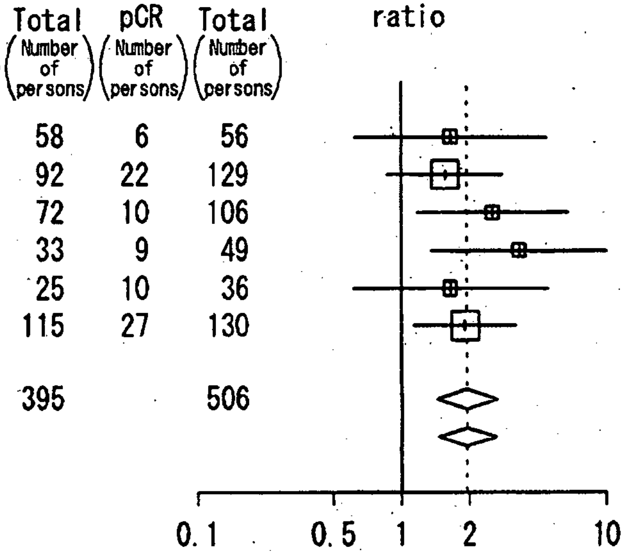

FIG. 21

| Subject group number | Classification | Higher than mean value pCR (Number of persons) | Higher than mean value Total (Number of persons) | Not Higher than mean value pCR (Number of persons) | Not Higher than mean value Total (Number of persons) | Odds ratio | Odds ratio | 95% confidence interval |
|---|---|---|---|---|---|---|---|---|
| 2-1 | $ER^{+}$, $HER2^{-}$ | 22 | 148 | 16 | 227 | 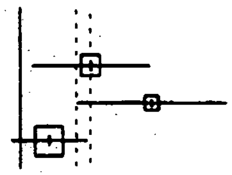 | 2.30 | [1.17;4.55] |
| 2-2 | $ER^{+-}$, $HER2^{+}$ | 26 | 56 | 9 | 58 | | 4.72 | [2.95;11.42] |
| 2-3 | $ER^{-}$, $HER2^{-}$ | 64 | 178 | 59 | 208 | | 1.42 | [0.92;2.18] |

# FIG. 22

| Subject group number | Classification | Higher than mean value: pCR (Number of persons) | Higher than mean value: Total (Number of persons) | Not Higher than mean value: pCR (Number of persons) | Not Higher than mean value: Total (Number of persons) | Odds ratio | Odds ratio | 95% confidence interval |
|---|---|---|---|---|---|---|---|---|
| 3-1 | Epirubicin | 10 | 58 | 6 | 56 | 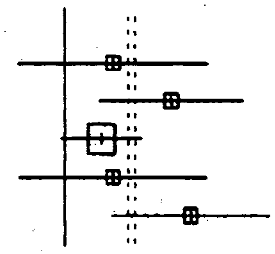 | 1.74 | [0.59;5.15] |
| 3-2 | FAC or FEC | 20 | 68 | 11 | 101 | | 3.41 | [1.51;7.70] |
| 3-3 | A. paclitaxel | 49 | 179 | 49 | 250 | | 1.55 | [0.98;2.43] |
| 3-4 | A. docetaxel | 10 | 25 | 10 | 36 | | 1.73 | [0.59;5.12] |
| 3-5 | A. Ixabepilone | 25 | 65 | 8 | 63 | | 4.30 | [1.76;10.51] |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011065533 A
- WO 2013014296 A1
- JP 2013179877 A


### Non-patent literature cited in the description


- **IWAMOTO T et al.** Gene pathways associated with prognosis and chemotherapy sensitivity in molecular subtypes of breast cancer. *Journal of the National Cancer Institute,* 2011, vol. 103, 264-272
- **SCHMIDT M et al.** The humoral immune system has a key prognostic impact in node-negative breast cancer. *Cancer research,* 2008, vol. 68, 5405-5413
- **SCHMIDT M et al.** A comprehensive analysis of human gene expression profiles identifies stromal immunoglobulin kappa C as a compatible prognostic marker in human solid tumors. *Clinical Cancer Research,* 2012, vol. 18, 2695-2703
- **TESCHENDORFF AE et al.** An immune response gene expression module identifies a good prognosis subtype in estrogen receptor negative breast cancer. *Genome Biology,* 2007, vol. 8, R157
- **SCHMIDT M et al.** *Cancer research,* 2008, vol. 68, 5405-5413
- **SCHMIDT M et al.** *Clinical Cancer Research,* 2012, vol. 18, 2695-2703
- **TESCHENDORFF AE et al.** *Genome Biology,* 2007, vol. 8, R157
- **SAMBROOK, J. et al.** Molecular Cloning: A Laboratory Manual. Cold Spring Harbor Laboratory Press, 1998
- **IWAMOTO T et al.** *Journal of the National Cancer Institute,* 2011, vol. 103, 264-272
- **SCHMIDT M et al.** *Clinical' Cancer Research,* 2012, vol. 18, 2695-2703
- **SCHMIDT M et al.** *Clinical Cancer. Research,* 2012, vol. 18, 2695-2703
- **THECHENDORFF AE et al.** *Genome Biology,* 2007, vol. 8, R157